# EUROPEAN PATENT APPLICATION

(11) **EP 2 626 352 A1**
(43) Date of publication of application: **14.08.2013**
(21) Application number: 11822202.5
(22) Date of filing: 30.08.2011
(51) Int. Cl.: C07D 401/12, C07D 401/14, C07D 405/14, A61K 31/4427, A61K 31/4523, A61P 25/28

(54) **HETEROCYCLIC LOW-MOLECULAR SAPP MIMETICS, A PHARMACEUTICAL COMPOSITION AND METHODS FOR PRODUCING AND USING SAME**

(30) Priority: 31.08.2010 RU 2010135900
(71) Applicant: Obschestvo S Ogranichennoy Otvetstvennostju "Biopharm-Memorien", Kaluzhskaya obl. 249031 (RU)
(72) Inventor: NENAYDENKO, Valentin Georgievich, Moscow 117303 (RU); TKACHENKO, Sergey Evgenievich, Chernogolovka Moskovskaya obl. 142432 (RU); BACHURIN, Sergey Olegovich, Chernogolovka Moskovskaya obl. 142432 (RU); KABAKOV, Vladimir Evgenievich, Moscow 121614 (RU); ANOKHIN, Konstantin Vladimirovich, Moscow 121170 (RU)
(74) Representative: Anohins, Vladimirs
(86) International application number: PCT/RU2011/000662
(87) International publication number: WO 2012/030258

(57) **Abstract**

The invention relates to a novel class of non-peptide heterocyclic low-molecular peptide mimetics of secretory amyloid precursor protein (sAPP mimetics) capable of acting effectively upon the processes of the formation, storage and regeneration of normal and pathological memory. The compounds can also be used for studying (*in vitro* and *in vivo*) biochemical signaling, neurotrophic and neuroprotective processes related to the secretory protein sAPP and for studying the mechanisms of the formation, storage and regeneration of memory. Specifically, the invention relates to (azaheterocyclyl)alkyl derivative amides of 2-(het)arylglycines of general formula (I) 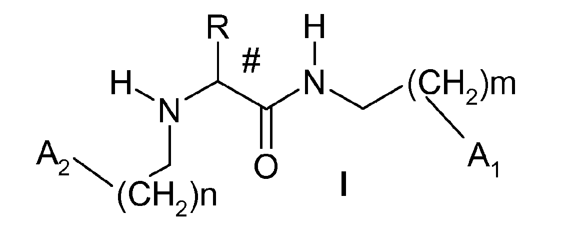 in which: n and m can have the values 0, 1, 2 and 3; the symbol (#) here and hereinafter signifies the possible presence of a chiral centre; R represents optionally substituted C₅-C₁₀aryl or a 5-7-membered hetaryl containing 1-3 heteroatoms selected from nitrogen, oxygen and sulphur and optionally condensed with a benzene ring; A1 and A2 independently represent an optionally substituted, 3-7-membered, saturated, partially saturated or aromatic azaheterocycle which contains from 1 to 3 atoms of nitrogen in the cycle and is optionally condensed with a benzene ring; or their pharmaceutically acceptable salts or alkyl esters in the form of individual optical isomers, or combinations thereof.

## Description

The invention refers to novel compounds of non-peptide nature which are peptidomimetic of a secretory precursor of amyloid sAPP peptide (sAPP-mimetic). The compounds can be used to restore memory lost in health and none.

Currently, dementia in older people represents a serious medical problem which is evident in 4% of the population aged 65 years and at least in 35% of the population aged 85 years. The number of patients suffering from dementia rise rapidly due to the increase in life expectancy and it11 is expected that it will reach 80 million in 2040. Alzheimer's disease is the most common form of dementia (AD, about 60% of all cases), which is a progressive, irreversible neurodegenerative disease with a poorly understood pathogenesis and with no effective treatment. The development of such medicine has been conducted for many years, but up to now the main focus is on pharmacological correction of cholinergic deficit, that led to creation a number of medecines on the base of acetylcholinesterase inhibitors (Takrin, Amiridin, Arisept etc.) that have a limited clinical applicability. An antagonist of NMDA receptors Mementin which leads to short-term improvement of cognitive function, but does not prevent the progression of AD is widly used nowadays.

It is known that one of the synaptic glycoprotein sAPP, plays a key role in the regulation of neuronal activity, plasticity and memory formation. An unusual feature of APP is that this protein is a precursor of both neurotoxic amyloid β-A which plays a key role in pathogenesis and secretory neuropeptide sAPP. Glycoprotein APP cleaves due to three enzymes: α-, β- and γ-secretase. Amyloid β-A consisting of 40-42 aminoacids forms by amyloidogenic processing of APP consecutive action of β- and γ-secretase. Soluble forms of β-A exhibit neurotoxic properties and cause memory impairment in experimental animals. Aggregated β-A peptides are the main component of senile plaques that are formed in the brains of AD patients. However normally the main focus of APP proteolysis is a nonamyloidogenic processing due to α- secretase. The main result of α- secretase action is a release of soluble secretable peptide sAPP, which has neuroprotective and neurotrophic properties. A special role sAPP peptide plays in learning, memory formation and consolidation.

Neuroprotective, neurotrophic and promnestic properties of secretable peptide sAPP allow to consider the activation of nonamyloidogenic processing of protein-precursor APP as a new and potential strategy for creating disease-modyfying agents to treat AD. This strategy has two directions: 1) creation of neuropharmacological drugs that activate α- secretase; 2) creation peptidomimetic neurotropic drugs that exhibit effects similar to neuroprotective, neurotrophic and promnestic properties of peptide sAPP. Within the first direction different α- secretase activators have been found. The search of sAPP-mimetics within the second direction was less successful. At present only small peptides, mainly fragments of APP protein (particularly, pentapeptides) with neuroprotective and promnestic properties have been discovered. Taking into account the low bioavailability and easy proteolytic degradation of peptide sAPP-mimetics, it is seems imposible the clinical use of founded small peptide sAPP-mimetics.

It should also be stated that up to nowadays none of sAPP-mimetic of nonpeptide nature was found.

Among nonpeptide structural analogues, which can be used in the treatment of neurodegenerative, neurological and psychotic disorders, the compounds described in the documents listed below may be mentioned.

In US 6943149B2 and US 6858577B1 indole and imidazole peptidomimetics which may be used for neurodegenerative diseases treatment are described. The applications US 2010/0048656A1 and US 2009/0325993A1 describes cyclic amide derivatives of aminoacetic acid for treating neurological and psychotic disorders such as psychosis, dementia or attention deficit disorder. It is known the derivatives of arylglycinamide described in patent RU 2167866 which show the properties of neurokinin antagonists and may be used to treat CNS diseases, such as dementia, AD, schizophrenia, psychosis, etc.

The object of the present invention is to find new high-performance medecines for tretment and early detection of AD with novel mechanism of action, particularly compounds that block and inhibit development of neurodegenerative processes.

The compounds of the present unvention relates to a novel class of non-peptide sAPP-mimetics which have a unique combination of two main approaches in treatment, for example, AD. In this case, the target of the compounds is a metabolic cascade of APP and, in addition, the compounds have the ability to stimulate long-term memory.

The compounds of the present invention represent novel (azaheterocyclyl) alkyl derivative amides of 2-(het)arylglycines of general formula (I) 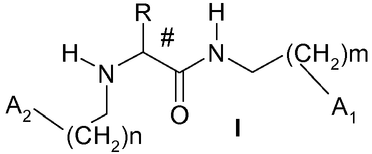 in which:
**n** and m can have the values 0, 1, 2 and 3;
the symbol (#) here and hereinafter signifies the possible presence of a chiral centre;
**R** represents optionally substituted C₅-C₁₀aryl or a 5-7-membered hetaryl containing 1-3 heteroatoms selected from nitrogen, oxygen and sulphur and optionally condensed with a benzene ring;
**A1** and **A2** independently represent an optionally substituted, 3-7-membered, saturated, partially saturated or aromatic azaheterocycle which contains from 1 to 3 atoms of nitrogen in the cycle and is optionally condensed with a benzene ring;
or their pharmaceutically acceptable salts or alkyl esters in the form of individual optical isomers, or combinations thereof.

The preffered compounds represent (azaheterocyclyl)alkyl derivatives of arylglycines amides of the general formula **I.1,** 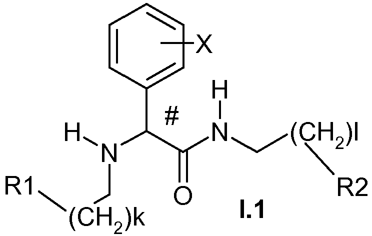 in which:
**k** and **I** can have the values 0 and 1;
**X** represents H, optionally substituted C₁-C₈alkyl, optionally substituted C₁-C₈alkylalkoxy, halogen, OH, CF₃, CN, CF₃O, optionally substituted amino, C₁-C₆acyl, optionally substituted C₆aryl or 5-6-membered hetaryl containing 1-3 heteroatoms, C₂-C₄alkenyl, C₂-C₄alkynyl, C₆arC₁-C₆alkyl, C₁-C₈alkoxycarbonyl, 5-6-membered hetaryl which contains 1-2 heteroatoms selected from nitrogen, oxygen and sulfur, C₆arylsulfonyl, or C₁-C₈alkylsulfonyl, optionally N-substituted aminosulfonyl;
or **X** represents benzene ring annelated with phenyl or 5-7-membered heterocyclyl containing 1-3 heteroatoms selected from nitrogen, oxygen, sulfur;
**R1** and **R2** independently represent optionally substituted 6-membered azaheterocycle such as pyridin, piperidin, pyrimidine, triazine, quinoline, isoquinoline, quinazoline, quinoxaline, or a pharmaceutically acceptable salt thereof.

Thus the compounds **I.1** individual isomers or as a mixture of R-(-) - and S-(+)-isomers, respectively, represented by the general formulae **I.1.1** and **I.1.2:** 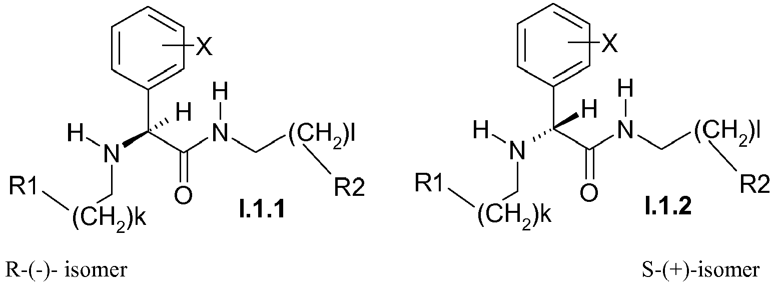 in which:
**k** and **I** can have the values 0 and 1;
**X** represents H, optionally substituted C₁-C₈alkyl, optionally substituted C₁-C₈alkylalkoxy, halogen, OH, CF₃, CN, CF₃O, optionally substituted amino, C₁-C₆acyl, optionally substituted C₆aryl or 5-6-membered hetaryl containing 1-3 heteroatoms, C₂-C₄alkenyl, C₂-C₄alkynyl, C₆arC₁-C₆alkyl, C₁-C₈alkoxycarbonyl, 5-6-membered hetaryl which contains 1-2 heteroatoms selected from nitrogen, oxygen and sulfur, C₆arylsulfonyl, or C₁-C₈alkylsulfonyl, optionally N-substituted aminosulfonyl;
or X represents benzene ring annelated with phenyl or 5-7-membered heterocyclyl containing 1-3 heteroatoms selected from nitrogen, oxygen, sulfur;
**R1** and **R2** independently represent optionally substituted 6- membered azaheterocycle such as pyridin, piperidin, pyrimidine, triazine, quinoline, isoquinoline, quinazoline, quinoxaline, or a pharmaceutically acceptable salt thereof.

The compounds of the formula **I** potentiate the current of amino methyl propanoic acid receptors so it can serve for cognitive function enhancement including training, memory formation, consolidation and retrieval.

In addition, it has been found that the compounds of formula 1 may influence on the other parts of memory that is to improve the processes of memory retrieval, so it makes them unique among the others well-known drugs that have an impact on memory.

The ability to restore memory with the compounds of formula **I** is not obvious and does not appear in the chemical structure of the compounds. The described properties allow using the compounds as a medicine for recovery lost memory of patients of all ages in health and none. The compounds may be used to restore memory losted, for example, as a result of a neurodegenerative disease or other diseases of CNS such as Alzheimer's, Parkinson's and Huntington's diseases, multiple sclerosis, cerebellar ataxia,; amyotrophic lateral sclerosis, dementia with Lewy bodies, spinal muscular atrophy, peripheral neuropathy, spongiform encephalitis, AIDS-related dementia; multi-infarct dementia, frontotemporal dementia, leukoencephalopathy, chronic neurodegenerative disease, cerebral accident, hypoxic, ischemic and reperfusion brain damage, epilepsy; cerebral ischemia, glaucoma, Down's syndrome, encephalomyelitis, meningitis, encephalitis, sympathicoblastoma; adolescent insanity, mental depression and neurodegenerative processes.

In the compounds of the present invention in A1, A2, R - groups, C₁-C₈alkyl is a linear or branched alkyl optionally cyclic, in which one or more SN2 group can be replaced by oxygen, nitrogen or sulfur. For example: methyl, ethyl, isopropile, n-propyl, isobutyl, s-butyl, tert-butyl, hexyl, nonyl cyclopropyl, cyclohexyl, cyclobutyl, cyclopentyl, cyclooctyl etc.

**Alkyl substituents** may be selected from halogen atoms, atoms of chlorine, bromine, iodine, C₁-C₈, preferable C₁-C₄ alkoxy, halogened C₁-C₄ alkoxy, hydrooxy, aryloxy, amino, mono- or C1-C₄ alkyl amine, cyano, nitro, alkylthio, etc.

**Amino-group substituents** are selected first of all from C₁-C₈ alkyl, preferably C₁-C₄ alkyl, which may be branched, or C₁-C₄ alkyl, substituted with hydroxy, C₁-C₄ alkoxy or amino, mono-, or C₁-C₄ alkylaminogroups.

**Aryl or hetaryl substituents** are selected from H, optionally substituted alkyl, where the substituents are as defined above, optionally substituted C₁-C₄ alkoxy, for example, halogenated alkoxy, such as CF₃O, halogen, OH, CN, optionally substituted, as defined above, amino-group, C₁-C₆acyl group, optionally substituted C₆aryl or 5-6 membered hetaryl, containing 1-3 heteroatoms, where where each aryl and hetaryl substituents can be selected from halogen, C₁-C₄ alkyl, C₂-C₄ alkenyl; C₂-C₄ alkenyl, C₂-C₄ alkenyl, C₁-C₆ alkyl, C₁-C₈ alkoxycarbonyl, carboxy group, 5-6 membered hetaryl containing 1-2 heteroatoms selected from nitrogen, oxygen and sulfur, C₆arylsulfonyl or C₁-C₈ alkylsulfonyl, optionally N-substituted, as defined above, aminosulfonyl.

Benzene ring, annulated with phenyl or 5-7 membered heterocyclyl, containing 1-3 heteroatoms selected from nitrogen, oxygen, sulfur, usually represents a bicyclic or tricyclic group selected from, for example, naphthalene, quinolyl, indolyl, benzopyrazolyl, benzothiophenyl, benzofuranyl, benzoisofuranyl, benzodioxalyl, benzodioxsin, benzazepin, benxodiazepin, benzimidazole, benzizoxazolyl, benzoxazolyl, benzthiazolyl, benzoisothiazolyl, pyridopyrimidine, pyridoimidazolyl, pyridotriazolyl etc. Annulated groups may be substituted for example with halogen, C₁-C₄ alkyl, C₁-C₄ alkoxy.

The salts of hydrohalic salts, especially hydrochlorides, the salts of sulfuric and sulfonic acids, such as mesylate, tosylate, phosphoric acid, maleic acid, fumaric acid, citric acid, tartaric acid, acetic acid, carboxylic acid, for example, bicarbonates, the salts of alkali or alkaline earth metals may be used as pharmaceutically acceptable salts.

Preferable compounds may be selected from the following compounds:
2-(2,4-dichlorophenyl)-N-(2-pyridin-4-ylethyl)-2-[(2-pyridin-4-ylethyl)amino]acetamide;
2-(2,4-dichlorophenyl)-N-(pyridin-4-ylmethyl)-2-[(pyridin-4-ylmethyl)-amino]acetamide;
2-(2,4-dichlorophenyl)-2-[(2-piperidin-4-ylethyl)amino]-N-(2-pyridin-4-ylethyl)acetamide;
2-(2,4-dichlorophenyl)-2-[(2-piperidin-4-ylethyl)-amino]-N-(pyridin-4-ylmethyl)acetamide;
2-(2,5-dimethylphenyl)-N-(2-pyridin-4-ylethyl)-2-[(2-pyridin-3-ylethyl)amino]acetamide;
2-(2,4-dichlorophenyl)-N-(pyridin-4-ylmethyl)-2-[(pyridin-3-ylmethyl)amino]acetamide;
2-(2,4-dichlorophenyl)-N-(pyridin-3-ylmethyl)-2-[(pyridin-4-ylmethyl)amino]acetamide;
2-(2,4-dichlorophenyl)-N-(2-pyridin-3-ylethyl)-2-[(2-pyridin-3-ylethyl)amino]acetamide;
2-(2,4-dichlorophenyl)-N-(2-pyridin-4-ylethyl)-2-[(2-pyridin-3-ylethyl)amino]acetamide;

2-(2,4-dichlorophenyl)-N-(2-pyridin-3-ylethyl)-2-[(2-pyridin-4-ylethyl)amino]acetamide;
2-(2,5-dichlorophenyl)-N-(2-pyridin-4-ylethyl)-2-[(2-pyridin-4-ylethyl)amino]acetamide;
2-(2,5-dichlorophenyl)-N-(pyridin-4-ylmethyl)-2-[(pyridin-4-ylmethyl)amino]acetamide;
2-(2,5-dichlorophenyl)-2-[(2-piperidin-4-ylethyl)-amino]-N-(2-pyridin-4-ylethyl)acetamide;
2-(2,5-dichlorophenyl)-2-[(2-piperidin-4-ylethyl)amino]-N-(pyridin-4-ylmethyl)acetamide;
2-(2,5-dichlorophenyl)-N-(pyridin-3-ylmethyl)-2-[(pyridin-3-ylmethyl)amino]acetamide;
2-(2,5-dichlorophenyl)-N-(pyridin-4-ylmethyl)-2-[(pyridin-3-ylmethyl)amino]acetamide;
2-(2,5-dichlorophenyl)-N-(pyridin-3-ylmethyl)-2-[(pyridin-4-ylmethyl)amino]acetamide;
2-(2,5-dichlorophenyl)-N-(2-pyridin-3-ylethyl)-2-[(2-pyridin-3-ylethyl)amino]acetamide;
2-(2,5-dichlorophenyl)-N-(2-pyridin-4-ylethyl)-2-[(2-pyridin-3-ylethyl)amino]acetamide;
2-(2,5-dichlorophenyl)-N-(2-pyridin-3-ylethyl)-2-[(2-pyridin-4-ylethyl)amino]acetamide;
2-(2,4-dimethylphenyl)-N-(2-pyridin-4-ylethyl)-2-[(2-pyridin-4-ylethyl)amino]acetamide;
2-(2,4-dimethylphenyl)-N-(pyridin-4-ylmethyl)-2-[(pyridin-4-ylmethyl)amino]acetamide;
2-(2,4-dimethylphenyl)-2-[(2-piperidin-4-ylethyl)amino]-N-(2-pyridin-4-ylethyl)acetamide;
2-(2,4-dimethylphenyl)-2-[(2-piperidin-4-ylethyl)amino]-N-(pyridin-4-ylmethyl)acetamide;
2-(2,4-dimethylphenyl)-N-(pyridin-3-ylmethyl)-2-[(pyridin-3-ylmethyl)amino]acetamide;
2-(2,4-dimethylphenyl)-N-(pyridin-4-ylmethyl)-2-[(pyridin-3-ylmethyl)amino]acetamide;
2-(2,4-dimethylphenyl)-N-(pyridin-3-ylmethyl)-2-[(pyridin-4-ylmethyl)amino]acetamide;
2-(2,4-dimethylphenyl)-N-(2-pyridin-3-ylethyl)-2-[(2-pyridin-3-ylethyl)amino]acetamide;
2-(2,4-dimethylphenyl)-N-(2-pyridin-4-ylethyl)-2-[(2-pyridin-3-ylethyl)amino]acetamide;
2-(2,4-dimethylphenyl)-N-(2-pyridin-3-ylethyl)-2-[(2-pyridin-4-ylethyl)amino]acetamide;
2-(2,5-dimethylphenyl)-N-(2-pyridin-4-ylethyl)-2-[(2-pyridin-4-ylethyl)amino]acetamide;
2-(2,5-dimethylphenyl)-N-(pyridin-4-ylmethyl)-2-[(pyridin-4-ylmethyl)amino]acetamide;
2-(2,5-dimethylphenyl)-2-[(2-piperidin-4-ylethyl)amino]-N-(2-pyridin-4-ylethyl)acetamide;
2-(2,5-dimethylphenyl)-2-[(2-piperidin-4-ylethyl)amino]-N-(pyridin-4-ylmethyl)acetamide;
2-(2,5-dimethylphenyl)-N-(pyridin-3-ylmethyl)-2-[(pyridin-3-ylmethyl)amino]acetamide;
2-(2,5-dimethylphenyl)-N-(pyridin-4-ylmethyl)-2-[(pyridin-3-ylmethyl)amino]acetamide;
2-(2,5-dimethylphenyl)-N-(pyridin-3-ylmethyl)-2-[(pyridin-4-ylmethyl)amino]acetamide;
2-(2,5-dimethylphenyl)-N-(2-pyridin-3-ylethyl)-2-[(2-pyridin-3-ylethyl) amino]acetamide;
2-(2,5-dimethylphenyl)-N-(2-pyridin-4-ylethyl)-2-[(2-pyridin-3-ylethyl) amino]acetamide;
2-(2,5-dimethylphenyl)-N-(2-pyridin-3-ylethyl)-2-[(2-pyridin-4-ylethyl) amino]acetamide;
2-(2-ethoxyphenyl)-N-(2-pyridin-4-ylethyl)-2-[(2-pyridin-4 ylethyl) amino]acetamide;
2-(2-ethoxyphenyl)-N-(pyridin-4-ylmethyl)-2-[(pyridin-4-ylmethyl)-amino]acetamide;
2-(2-ethoxyphenyl)-2-[(2-piperidin-4-ylethyl) amino]-N-(2-piridin-4-ylethyl)acetamide;

2-(2-ethoxyphenyl)-2-[(2-piperidin-4-ylethyl) amino]-N-(pyridin-4-ylmethyl)acetamide;
2-(2-ethoxyphenyl)-N-(pyridin-3-ylmethyl)-2-[(pyridin-3-ylmethyl)amino]acetamide;
2-(2-ethoxyphenyl)-N-(pyridin-4-ylmethyl)-2-[(pyridin-3-ylmethyl)amino]acetamide;
2-(2-ethoxyphenyl)-N-(pyridin-3-ylmethyl)-2-[(pyridin-4-ylmethyl)amino]acetamide;
2-(2-ethoxyphenyl)-N-(2-pyridin-3-ylethyl)-2-[(2-pyridin-3-ylethyl)amino]acetamide;
2-(2-ethoxyphenyl)-N-(2-pyridin-4-ylethyl)-2-[(2-pyridin-3-ylethyl)amino]acetamide;
2-(2-ethoxyphenyl)-N-(2-pyridin-3-ylethyl)-2-[(2-pyridin-4-ylethyl)amino]acetamide;
2-(4-ethoxyphenyl)-N-(2-pyridin-4-ylethyl)-2-[(2-pyridin-4-ylethyl)amino]acetamide;
2-(4-ethoxyphenyl)-N-(pyridin-4-ylmethyl)-2-[(pyridin-4-ylmethyl)amino]acetamide;
2-(4-ethoxyphenyl)-2-[(2-piperidin-4-ylethyl) amino]-N-(2-pyridin-4-ylethyl)acetamide;
2-(4-ethoxyphenyl)-2-[(2-piperidin-4- ylethyl) amino]-N-(pyridin-4-ylmethyl)acetamide;
2-(4-ethoxyphenyl)-N-(pyridin-3-ylmethyl)-2-[(pyridin-3-ylmethyl)amino]acetamide;
2-(4-ethoxyphenyl)-N-(pyridin-4-ylmethyl)-2-[(pyridin-3-ylmethyl)amino]acetamide;
2-(4-ethoxyphenyl)-N-(pyridin-3-ylmethyl)-2-[(pyridin-4-ylmethyl)amino]acetamide;
2-(4-ethoxyphenyl)-N-(2-pyridin-3-ylethyl)-2-[(2-pyridin-3-ylethyl)amino]acetamide;
2-(4-ethoxyphenyl)-N-(2-pyridin-4-ylethyl)-2-[(2-pyridin-3-ylethyl)amino]acetamide;

2-(4-ethoxyphenyl)-N-(2-pyridin-3-ylethyl)-2-[(2-pyridin-4-ylethyl)amino]acetamide;
2-(3-ethoxyphenyl)-N-(2-pyridin-4-ylethyl)-2-[(2-pyridin-4-ylethyl)amino]acetamide;
2-(3-ethoxyphenyl)-N-(pyridin-4-ylmethyl)-2-[(pyridin-4-ylmethyl)amino]acetamide;
2-(3-ethoxyphenyl)-2-[(2-piperidin-4-ylethyl)amino]-N-(2-pyridin-4-ylethyl)acetamide;
2-(3-ethoxyphenyl)-2-[(2-piperidin-4-ylethyl)amino]-N-(pyridin-4-ylmethyl)acetamide;
2-(3-ethoxyphenyl)-N-(pyridin-3-ylmethyl)-2-[(pyridin-3-ylmethyl)amino]acetamide;
2-(3-ethoxyphenyl)-N-(pyridin-4-ylmethyl)-2-[(pyridin-3-ylmethyl)-amino] acetamide;
2-(3-ethoxyphenyl)-N-(pyridin-3-ylmethyl)-2-[(pyridin-4-ylmethyl) amino]acetamide;
2-(3-ethoxyphenyl)-N-(2-pyridin-3-ylethyl)-2-[(2-pyridin-3-ylethyl) amino]acetamide;
2-(3-ethoxyphenyl)-N-(2-pyridin-4-ylethyl)-2-[(2-pyridin-3-ylethyl) amino]acetamide;
2-(3-ethoxyphenyl)-N-(2-pyridin-3-ylethyl)-2-[(2-pyridin-4-ylethyl) amino]acetamide;
N-(2-pyridin-4-ylethyl)-2-[(2-pyridin-4-ylethyl)amino]-2-[2-(trifluoromethyl)phenyl]acetamide;
N-(pyridin-4-ylmethyl)-2-[(pyridin-4-ylmethyl)amino]-2-[2-(trifluoromethyl)phenyl]acetamide;
2-[(2-piperidin-4-ylethyl)amino]-N-(2-pyridin-4-ylethyl)-2-[2-(trifluoromethyl)phenyl]acetamide;
2-[(2-piperidin-4-ylethyl)amino]-N-(pyriclin-4-ylmethyl)-2-[2-(trifluoromethyl)phenyl]acetamide;
N-(pyridin-3-ylmethyl)-2-[(pyridin-3-ylmethyl)amino]-2-[2-(trifluoromethyl)phenyl]acetamide;
N-(pyridin-4-ylmethyl)-2-[(pyridin-3-ylmethyl)amino]-2-[2-(trifluoromethyl)phenyl]acetamide;
N-(pyridin-3-ylmethyl)-2-[(pyridin-4-ylmethyl)amino]-2-[2-(trifluoromethyl)phenyl]acetamide;
N-(2-pyridin-3-ylethyl)-2-[(2-pyridin-3-ylethyl)amino]-2-[2-(trifluoromethyl)phenyl]acetamide;
N-(2-pyridin-4-ylethyl)-2-[(2-pyridin-3-ylethyl)amino]-2-[2-(trifluoromethyl)phenyl]acetamide;
N-(2-pyridin-3-ylethyl)-2-[(2-pyridin-4-ylethyl)amino]-2-[2-(trifluoromethyl)phenyl]acetamide;
N-(2-pyridin-4-ylethyl)-2-[(2-pyridin-4-ylethyl)amino]-2-[3-(trifluoromethyl)phenyl]acetamide;
N-(pyridin-4-ylmethyl)-2-[(pyridin-4-ylmethyl)amino]-2-[3-(trifluoromethyl)phenyl]acetamide;
2-[(2-piperidin-4-ylethyl)amino]-N-(2-pyridin-4-ylethyl)-2-[3-(trifluoromethyl)phenyl]acetamide;
2-[(2-piperidin-4-ylethyl)amino]-N-(pyridin-4-ylmethyl)-2-[3-(trifluoromethyl)phenyl]acetamide;
N-(pyridin-3-ylmethyl)-2-[(pyridin-3-ylmethyl)amino]-2-[3-(trifluoromethyl)phenyl]acetamide;
N-(pyridin-4-ylmethyl)-2-[(pyridin-3-ylmethyl)amino]-2-[3-(trifluoromethyl)phenyl]acetamide;
N-(pyridin-3-ylmethyl)-2-[(pyridin-4-ylmethyl)amino]-2-[3-(trifluoromethyl)phenyl]acetamide;
N-(2-pyridin-3-ylethyl)-2-[(2-pyridin-3-ylethyl)amino]-2-[3-(trifluoromethyl)phenyl]acetamide;
N-(2-pyridin-4-ylethyl)-2-[(2-pyridin-3-ylethyl)amino]-2-[3-(trifluoromethyl)phenyl]acetamide;
N-(2-pyridin-3-ylethyl)-2-[(2-pyridin-4-ylethyl)amino]-2-[3-(trifluoromethyl)phenyl]acetamide;
2-[4-(methylthio)phenyl]-N-(2-pyridin-4-ylethyl)-2-[(2-pyridin-4-ylethyl) amino]acetamide;
2-[4-(methylthio)phenyl]-N-(pyridin-4-ylmethyl)-2-[(pyridin-4-ylmethyl)amino]acetamide;
2-[4-(methylthio)phenyl]-2-[(2-piperidin-4-ylethyl)amino]-N-(2-pyridin-4-ylethyl)acetamide;
2-[4-(methylthio)-phenyl]-2-[(2-piperidin-4-ylethyl)amino]-N-(pyridin-4-ylethyl)acetamide;
2-[4-(methylthio)phenyl]-N-(pyridin-3-ylmethyl)-2-[(pyridin-3-ylmethyl)amino]acetamide;
2-[4-(methylthio)phenyl]-N-(pyridin-4-ylmethyl)-2-[(pyridin-3-ylmethyl)-amino]acetamide;
2-[4-(methylthio)phenyl]-N-(pyridin-3-ylmethyl)-2-[(pyridin-4-ylmethyl) amino]acetamide;
2-[4-(methylthio) phenyl]-N-(2-pyridin-3-ylethyl)-2-[(2-pyridin-3-ylethyl)amino]acetamide;
2-[4-(methylthio)phenyl]-N-(2-pyridin-4-ylethyl)-2-[(2-pyridin-3-ylethyl)amino]acetamide;
2-[4-(methylthio)phenyl]-N-(2-pyridin-3-ylethyl)-2-[(2-pyridin-4-ylethyl)amino]acetamide;
N-(2-pyridin-4-ylethyl)-2-[(2-pyridin-4-ylethyl)amino]-2-[4-(trifluoromethyl)phenyl]acetamide;
N-(pyridin-4-ylmethyl)-2-[(pyridin-4-ylmethyl)amino]-2-[4-(trifluoromethyl)phenyl]acetamide;
2-[(2-piperidin-4-ylethyl)amino]-N-(2-pyridin-4-ylethyl)-2-[4-(trifluoromethyl)phenyl]acetamide;
2-[(2-piperidin-4-ylethyl)amino]-N-(pyridin-4-ylmethyl)-2-[4-(trifluoromethyl)phenyl]acetamide;
N-(pyridin-3-ylmethyl)-2-[(pyridin-3-ylmethyl)amino]-2-[4-(trifluoromethyl)phenyl]acetamide;
N-(pyridin-4-ylmethyl)-2-[(pyridin-3-ylmethyl)amino]-2-[4-(trifluoromethyl)phenyl]acetamide;
N-(pyridin-3-ylmethyl)-2-[(pyridin-4-ylmethyl)amino]-2-[4-(trifluoromethyl)phenyl]acetamide;
N-(2-pyridin-3-ylethyl)-2-[(2-pyridin-3-ylethyl)amino]-2-[4-(trifluoromethyl)phenyl]acetamide;
N-(2-pyridin-4-ylethyl)-2-[(2-pyridin-3-ylethyl)amino]-2-[4-(trifluoromethyl)phenyl]acetamide;
N-(2-pyridin-3-ylethyl)-2-[(2-pyridin-4-ylethyl)amino]-2-[4-(trifluoromethyl)phenyl]acetamide;
2-(4- methoxyphenyl)-N-(2-pyridin-4-ylethyl)-2-[(2-pyridin-4-ylethyl)amino]acetamide;
2-(4- methoxyphenyl)-N-(pyridin-4-ylmethyl)-2-[(pyridin-4-ylmethyl)amino]acetamide;
2-(4-methoxyphenyl)-2-[(2-piperidin-4-ylethyl)amino]-N-(2-pyridin-4-ylethyl)acetamide;
2-(4-methoxyphenyl)-2-[(2-piperidin-4-ylethyl)amino]-N-(pyridin-4-ylmethyl)acetamide;
2-(4-methoxyphenyl)-N-(piperidin-3-ylmethyl)-2-[(pyridin-3-ylmethyl)amino]acetamide;
2-(4-methoxyphenyl)-N-(pyridin-4-ylmethyl)-2-[(pyridin-3-ylmethyl)amino]acetamide;
2-(4-methoxyphenyl)-N-(pyridin-3-ylmethyl)-2-[(pyridin-4-ylmethyl)amino]acetamide;
2-(4-methoxyphenyl)-N-(2-pyridin-3-ylethyl)-2-[(2-pyridin-3-ylethyl)amino]acetamide;
2-(4-methoxyphenyl)-N-(2-pyridin-4-ylethyl)-2-[(2-pyridin-3-ylethyl)amino]acetamide;
2-(4-methoxyphenyl)-N-(2-pyridin-3-ylethyl)-2-[(2-pyridin-4-ylethyl)amino]acetamide;
2-(3-methoxyphenyl)-N-(2-pyridin-4-ylethyl)-2-[(2-pyridin-4-ylethyl)amino]acetamide;
2-(3-methoxyphenyl)-N-(pyridin-4-ylmethyl)-2-[(pyridin-4-ylmethyl)amino]acetamide;
2-(3-methoxyphenyl)-2-[(2-piperidin-4-ylethyl)amino]-N-(2-pyridin-4-ylethyl)acetamide;
2-(3-methoxyphenyl)-2-[(2-piperidin-4-ylethyl)amino]-N-(pyridin-4-ylmethyl)acetamide;
2-(3-methoxyphenyl)-N-(pyridin-3-ylmethyl)-2-[(pyridin-3-ylmethyl)amino]acetamide;
2-(3-methoxyphenyl)-N-(pyridin-4-ylmethyl)-2-[(pyridin-3-ylmethyl)amino]acetamide;
2-(3-methoxyphenyl)-N-(pyridin-3-ylmethyl)-2-[(pyridin-4-ylmethyl)amino]acetamide;
2-(3-methoxyphenyl)-N-(2-pyridin-3-ylethyl)-2-[(2-pyridin-3-ylethyl)amino]acetamide;
2-(3-methoxyphenyl)-N-(2-pyridin-4-ylethyl)-2-[(2-pyridin-3-ylethyl)amino]acetamide;
2-(3-methoxyphenyl)-N-(2-pyridin-3-ylethyl)-2-[(2-pyridin-4-ylethyl)amino]acetamide;
2-(2-methoxyphenyl)-N-(2-pyridin-4-ylethyl)-2-[(2-pyridin-4-ylethyl)amino]acetamide;
2-(2--methoxyphenyl)-N-(pyridin-4-ylmethyl)-2-[(pyridin-4-ylmethyl)amino] acetamide;
2-(2-methoxyphenyl)-2-[(2-piperidin-4-ylethyl)amino]-N-(2-pyridin-4-ylethyl)acetamide;
2-(2-methoxyphenyl)-2-[(2-piperidin-4-ylethyl)amino]-N-(pyridin-4-ylmethyl)acetamide;
2-(2-methoxyphenyl)-N-(pyridin-3-ylmethyl)-2-[(pyridin-3-ylmethyl)amino]acetamide;
2-(2-methoxyphenyl)-N-(pyridin-4-ylmethyl)-2-[(pyridin-3-ylmethyl)amino]acetamide;
2-(2-methoxyphenyl)-N-(pyridin-3-ylmethyl)-2-[(pyridin-4-ylmethyl)amino]acetamide;
2-(2-methoxyphenyl)-N-(2-pyridin-3-ylethyl)-2-[(2-pyridin-3-ylethyl)amino]acetamide;
2-(2-methoxyphenyl)-N-(2-pyridin-4-ylethyl)-2-[(2-pyridin-3-ylethyl)amino]acetamide;
2-(2-methoxyphenyl)-N-(2-pyridin-3-ylethyl)-2-[(2-pyridin-4-ylethyl)amino]acetamide;
2-(3-chlorophenyl)-N-(2-pyridin-4-ylethyl)-2-[(2-pyridin-4-ylethyl)amino]acetamide;
2-(3-chlorophenyl)-N-(pyridin-4-ylmethyl)-2-[(pyridin-4-ylmethyl)amino] acetamide;
2-(3-chlorophenyl)-2-[(2-piperidin-4-ylethyl) amino]-N-(2-pyridin-4-ylethyl)acetamide;
2-(3-chlorophenyl)-2-[(2-piperidin-4-ylethyl) amino]-N-(pyridin-4-ylmethyl)acetamide;
2-(3-chlorophenyl)-N-(pyridin-3-ylmethyl)-2-[(pyridin-3-ylmethyl)amino]acetamide;
2-(3-chlorophenyl)-N-(pyridin-4-ylmethyl)-2-[(pyridin-3-ylmethyl)amino]acetamide;
2-(3-chlorophenyl)-N-(pyridin-3-ylmethyl)-2-[(pyridin-4-ylmethyl)amino]acetamide;
2-(3-chlorophenyl)-N-(2-pyridin-3-ylethyl)-2-[(2-pyridin-3-ylethyl)amino]acetamide;
2-(3-chlorophenyl)-N-(2-pyridin-4-ylethyl)-2-[(2-pyridin-3-ylethyl)amino]acetamide;
2-(3-chlorophenyl)-N-(2-pyridin-3-ylethyl)-2-[(2-pyridin-4-ylethyl)amino]acetamide;
2-(2-bromophenyl)-N-(2-pyridin-4-ylethyl)-2-[(2-pyridin-4-ylethyl)amino]acetamide;
2-(2-bromophenyl)-N-(pyridin-4-ylmethyl)-2-[(pyridin-4-ylmethyl)amino] acetamide;
2-(2-bromophenyl)-2-[(2-piperidin-4-ylethyl)amino]-N-(2-pyridin-4-ylethyl)acetamide;
2-(2-bromophenyl)-2-[(2-piperidin-4-ylethyl)amino]-N-(pyridin-4-ylmethyl)acetamide;
2-(2-bromophenyl)-N-(pyridin-3-ylmethyl)-2-[(pyridin-3-ylmethyl)amino]acetamide;
2-(2-bromophenyl)-N-(pyridin -4-ylmethyl)-2-[(pyridin-3-ylmethyl)amino]acetamide;
2-(2-bromophenyl)-N-(pyridin -3-ylmethyl)-2-[(pyridin-4-ylmethyl)amino]acetamide;
2-(2-bromophenyl)-N-(2-pyridin-3-ylethyl)-2-[(2-pyridin-3-ylethyl)amino]acetamide;
2-(2-bromophenyl)-N-(2-pyridin -4-ylethyl)-2-[(2-pyridin-3-ylethyl)amino]acetamide;
2-(2-bromophenyl)-N-(2-pyridin -3-ylethyl)-2-[(2-pyridin-4-ylethyl)amino]acetamide;
2-(3-bromophenyl)-N-(2-pyridin-4-ylethyl)-2-[(2-pyridin-4-ylethyl)amino]acetamide;
2-(3-bromophenyl)-N-(pyridin-4-ylmethyl)-2-[(pyridin-4-ylmethyl)amino]acetamide;
2-(3-bromophenyl)-2-[(2-piperidin-4-ylethyl) amino]-N-(2-pyridin-4-ylethyl)acetamide;
2-(3-bromophenyl)-2-[(2-piperidin-4-ylethyl) amino]-N-(pyridin-4-ylmethyl)acetamide;
2-(3-bromophenyl)-N-(pyridin-3-ylmethyl)-2-[(pyridin-3-ylmethyl)amino]acetamide;
2-(3-bromophenyl)-N-(pyridin-4-ylmethyl)-2-[(pyridin-3-ylmethyl)amino]acetamide;
2-(3-bromophenyl)-N-(pyridin-3-ylmethyl)-2-[(pyridin-4-ylmethyl)amino]acetamide;
2-(3-bromophenyl)-N-(2-pyridin-3-ylethyl)-2-[(2-pyridin-3-ylethyl)amino]acetamide;
2-(3-bromophenyl)-N-(2-pyridin-4-ylethyl)-2-[(2-pyridin-3-ylethyl)amino]acetamide;
2-(3-bromophenyl)-N-(2-pyridin-3-ylethyl)-2-[(2-pyridin-4-ylethyl)amino]acetamide;
2-(4-bromophenyl)-N-(2-pyridin-4-ylethyl)-2-[(2-pyridin-4-ylethyl)amino]acetamide;
2-(4-bromophenyl)-N-(pyridin-4-ylmethyl)-2-[(pyridin-4-ylmethyl)amino]acetamide;
2-(4-bromophenyl)-2-[(2-piperidin-4-ylethyl) amino]-N-(2-pyridin-4-ylethyl)acetamide;
2-(4-bromophenyl)-2-[(2-piperidin -4-ylethyl)amino]-N-(pyridin-4-ylmethy)acetamide;
2-(4-bromophenyl)-N-(pyridin-3-ylmethyl)-2-[(pyridin-3-ylmethyl)amino]acetamide;
2-(4-bromophenyl)-N-(pyridin-4-ylmethyl)-2-[(pyridin-3-ylmethyl)amino]acetamide;
2-(4-bromophenyl)-N-(pyridin-3-ylmethyl)-2-[(pyridin-4-ylmethyl)amino]acetamide;
2-(4-bromophenyl)-N-(2-pyridin-3-ylethyl)-2-[(2-pyridin-3-ylethyl)amino]acetamide;
2-(4-bromophenyl)-N-(2-pyridin-4-ylethyl)-2-[(2-pyridin-3-ylethyl)amino]acetamide;
2-(4-bromophenyl)-N-(2-pyridin-3-ylethyl)-2-[(2-pyridin-4-ylethyl)amino]acetamide;
2-(4-chlorophenyl)-N-(2-pyridin-4-ylethyl)-2-[(2-pyridin-4-ylethyl)amino]acetamide;
2-(4-chlorophenyl)-N-(pyridin-4-ylmethyl)-2-[(pyridin-4-ylmethyl)amino]acetamide;
2-(4-chlorophenyl)-2-[(2-piperidin-4-ylethyl)amino]-N-(2-pyridin-4-ylethyl)acetamide;
2-(4-chlorophenyl)-2-[(2-piperidin-4-ylethyl)amino]-N-(pyridin-4-ylethyl)acetamide;
2-(4-chlorophenyl)-N-(pyridin-3-ylmethyl)-2-[(pyridin-3-ylmethyl)amino]acetamide;
2-(4-chlorophenyl)-N-(pyridin-4-ylmethyl)-2-[(pyridin-3-ylmethyl)amino]acetamide;
2-(4-chlorophenyl)-N-(pyridin-3-ylmethyl)-2-[(pyridin-4-ylmethyl)amino]acetamide;
2-(4-chlorophenyl)-N-(2-pyridin-3-ylethyl)-2-[(2-pyridin-3-ylethyl)amino]acetamide;
2-(4-chlorophenyl)-N-(2-pyridin-4-ylethyl)-2-[(2-pyridin-3-ylethyl)amino]acetamide;
2-(4-chlorophenyl)-N-(2-pyridin-3-ylethyl)-2-[(2-pyridin-4-ylethyl)amino]acetamide;
2-(2-chlorophenyl)-N-(2-pyridin-4-ylethyl)-2-[(2-pyridin-4-ylethyl)amino]acetamide;
2-(2-chlorophenyl)-N-(pyridin-4-ylmethyl)-2-[(pyridin-4-ylmethyl)amino]acetamide;
2-(2-chlorophenyl)-2-[(2-piperidin-4-ylethyl)amino]-N-(2-pyridin-4-ylethyl)acetamide;
2-(2-chlorophenyl)-2-[(2-piperidin-4-ylethyl)amino]-N-(pyridin-4-ylmethyl)acetamide;
2-(2-chlorophenyl)-N-(pyridin-3-ylmethyl)-2-[(pyridin-3-ylmethyl)amino]acetamide;
2-(2-chlorophenyl)-N-(pyridin-4-ylmethyl)-2-[(pyridin-3-ylmethyl)amino]acetamide;

2-(2-chlorophenyl)-N-(pyridin-3-ylmethyl)-2-[(pyridin-4-ylmethyl)amino]acetamide;
2-(2-chlorophenyl)-N-(2-pyridin-3-ylethyl)-2-[(2-pyridin-3-ylethyl)amino]acetamide;
2-(2-chlorophenyl)-N-(2-pyridin-4-ylethyl)-2-[(2-pyridin-3-ylethyl)amino]acetamide;
2-(2-chlorophenyl)-N-(2-pyridin-3-ylethyl)-2-[(2-pyridin-3-ylethyl)amino]acetamide;
2-(2-fluorophenyl)-N-(2-pyridin-4-ylethyl)-2-[(2-pyridin-4-ylethyl)amino]acetamide;
2-(2-fluorophenyl)-N-(pyridin-4-ylmethyl)-2-[(pyridin-4-ylmethyl)amino]acetamide;
2-(2-fluorophenyl)-2-[(2-piperidin-4-ylethyl)amino]-N-(2-pyridin-4-ylethyl)acetamide;
2-(2-fluorophenyl)-2-[(2-piperidin-4-ylethyl)amino]-N-(pyridin-4-ylmethyl)acetamide;
2-(2-fluorophenyl)-N-(pyridin-3-ylmethyl)-2-[(pyridin-3-ylmethyl)amino]acetamide;
2-(2-fluorophenyl)-N-(pyridin-4-ylmethyl)-2-[(pyridin-3-ylmethyl)amino]acetamide;
2-(2-fluorophenyl)-N-(pyridin-3-ylmethyl)-2-[(pyridin-4-ylmethyl)amino]acetamide;
2-(2-fluorophenyl)-N-(2-pyridin-3-ylethyl)-2-[(2-pyridin-3-ylethyl)amino]acetamide;
2-(2-fluorophenyl)-N-(2-pyridin-4-ylethyl)-2-[(2-pyridin-3-ylethyl)amino]acetamide;
2-(2-fluorophenyl)-N-(2-pyridin-3-ylethyl)-2-[(2-pyridin-4-ylethyl)amino]acetamide;
2-(2,4-dimethoxyphenyl)-N-(2-pyridin-4-ylethyl)-2-[(2-pyridin-4-ylethyl)amino]acetamide;
2-(2,4-dimethoxyphenyl)-N-(pyridin-4-ylethyl)-2-[(pyridin-4-ylmethyl)amino]acetamide;
2-(2,4-dimethoxyphenyl)-2-[(2-piperidin-4-ylethyl)amino]-N-(2-pyridin-4-ylethyl)acetamide;
2-(2,4-dimethoxyphenyl)-2-[(2-piperidin-4-ylethyl)amino]-N-(pyridin-4-ylmethyl)acetamide;
2-(2,4-dimethoxyphenyl)-N-(pyridin-3-ylmethyl)-2-[(pyridin-3-ylmethyl)amino]acetamide;
2-(2,4-dimethoxyphenyl)-N-(pyridin-4-ylmethyl)-2-[(pyridin-3-ylmethyl)amino]acetamide;
2-(2,4-dimethoxyphenyl)-N-(pyridin-3-ylmethyl)-2-[(pyridin-4-ylmethyl)amino]acetamide;
2-(2,4-dimethoxyphenyl)-N-(2-pyridin-3-ylethyl)-2-[(2-pyridin-3-ylethyl)amino]acetamide;
2-(2,4-dimethoxyphenyl)-N-(2-pyridin-4-ylethyl)-2-[(2-pyridin-3-ylethyl)amino]acetamide;
2-(2,4-dimethoxyphenyl)-N-(2-pyridin-3-ylethyl)-2-[(2-pyridin-4-ylethyl)amino]acetamide;
2-(2,5-dimethoxyphenyl)-N-(2-pyridin-4-ylethyl)-2-[(2-pyridin-4-ylethyl)amino]acetamide;
2-(2,5-dimethoxyphenyl)-N-(pyridin-4-ylmethyl)-2-[(pyridin-4-ylmethy)amino]acetamide;
2-(2,5-dimethoxyphenyl)-2-[(2-piperidin-4-ylethyl)amino]-N-(2-pyridin-4-ylethyl)acetamide;
2-(2,5-dimethoxyphenyl)-2-[(2-piperidin-4-ylethyl)amino]-N-(pyridin-4-ylmethy)acetamide;
2-(2,5-dimethoxyphenyl)-N-(pyridin-3-ylmethyl)-2-[(pyridin-3-ylmethyl)amino]acetamide;
2-(2,5-dimethoxyphenyl)-N-(pyridin-4-ylmethyl)-2-[(pyridin-3-ylmethyl)amino]acetamide;
2-(2,5-dimethoxyphenyl)-N-(pyridin-3-ylmethyl)-2-[(pyridin-4-ylmethy)amino]acetamide;
2-(2,5-dimethoxyphenyl)-N-(2-pyridin-3-ylethyl)-2-[(2-pyridin-3-ylethyl)amino]acetamide;
2-(2,5-dimethoxyphenyl)-N-(2-pyridin-4-ylethyl)-2-[(2-pyridin-3-ylethyl)amino]acetamide;
2-(2,5-dimethoxyphenyl)-N-(2-pyridin-3-ylethyl)-2-[(2-pyridin-4-ylethyl)amino]acetamide;
2-(3,4-dimethoxyphenyl)-N-(2-pyridin-4-ylethyl)-2-[(2-pyridin-4-ylethyl)amino]acetamide;
2-(3,4-dimethoxyphenyl)-N-(pyridin-4-ylmethyl)-2-[(pyridin-4-ylmethyl)amino]acetamide;
2-(3,4-dimethoxyphenyl)-2-[(2-piperidin -4-ylethyl) amino]-N-(2-pyridin-4-ylethyl)acetamide;
2-(3,4-dimethoxyphenyl)-2-[(2-piperidin-4-ylethyl)amino]-N-(pyridin-4-ylmethyl)acetamide;
2-(3,4-dimethoxyphenyl)-N-(pyridin-3-ylmethyl)-2-[(pyridin-3-ylmethyl)amino]acetamide;
2-(3,4-dimethoxyphenyl)-N-(pyridin-4-ylmethyl)-2-[(pyridin-3-ylmethyl)amino]acetamide;
2-(3,4-dimethoxyphenyl)-N-(pyridin-3-ylmethyl)-2-[(pyridin-4-ylmethyl)amino]acetamide;
2-(3,4-dimethoxyphenyl)-N-(2-pyridin-3-ylethyl)-2-[(2-pyridin-3-ylethyl)amino]acetamide;
2-(3,4-dimethoxyphenyl)-N-(2-pyridin-4-ylethyl)-2-[(2-pyridin-3-ylethyl)amino]acetamide;
2-(3,4-dimethoxyphenyl)-N-(2-pyridin-3-ylethyl)-2-[(2-pyridin-4-ylethyl)amino]acetamide;
2-(3-fluorophenyl)-N-(2-pyridin-4-ylethyl)-2-[(2-pyridin-4-ylethyl)amino]acetamide;
2-(3-fluorophenyl)-N-(pyridin-4-ylmethyl)-2-[(pyridin-4-ylmethyl)amino]acetamide;
2-(3-fluorophenyl)-2-[(2-piperidin-4-ylethyl)ylethyl]-N-(2-pyridin-4-ylethyl)acetamide;
2-(3-fluorophenyl)-2-[(2-piperidin-4-ylethyl)amino]-N-(pyridin-4-ylmethyl)acetamide;
2-(3-fluorophenyl)-N-(pyridin-3-ylmethyl)-2-[(pyridin-3-ylmethyl)amino]acetamide;
2-(3-fluorophenyl)-N-(pyridin-4-ylmethyl)-2-[(pyridin-3-ylmethyl)amino]acetamide;
2-(3-fluorophenyl)-N-(pyridin-3-ylmethyl)-2-[(pyridin-4-ylmethyl)amino]acetamide;
2-(3-fluorophenyl)-N-(2-pyridin-3-ylethyl)-2-[(2-pyridin-3-ylethyl)amino]acetamide;
2-(3-fluorophenyl)-N-(2-pyridin-4-ylethyl)-2-[(2-pyridin-3-ylethyl)amino]acetamide;
2-(3-fluorophenyl)-N-(2-pyridin-3-ylethyl)-2-[(2-pyridin-4-ylethyl)amino]acetamide;
2-(4-fluorophenyl)-N-(2-pyridin-4-ylethyl)-2-[(2-pyridin-4-ylethyl)amino]acetamide;
2-(4-fluorophenyl)-N-(pyridin-4-ylmethyl)-2-[(pyridin-4-ylmethyl)amino]acetamide;
2-(4-fluorophenyl)-2-[(2-piperidin-4-ylethyl)amino]-N-(2-pyridin-4-ylethyl)acetamide;
2-(4-fluorophenyl)-2-[(2-piperidin-4-pyridin)amino]-N-(pyridin-4-ylmethyl)acetamide;
2-(4-fluorophenyl)-N-(pyridin-3-ylmethyl)-2-[(pyridin-3-ylmethyl)amino]acetamide;
2-(4-fluorophenyl)-N-(pyridin-4-ylmethyl)-2-[(pyridin-3-ylmethyl)amino]acetamide;
2-(4-fluorophenyl)-N-(pyridin-3-ylmethyl)-2-[(pyridin-4-ylmethyl)amino]acetamide;
2-(4-fluorophenyl)-N-(2-pyridin-3-ylethyl)-2-[(2-pyridin-3-ylethyl)amino]acetamide;
2-(4-fluorophenyl)-N-(2-pyridin-4-ylethyl)-2-[(2-pyridin-3-ylethyl)amino]acetamide;
2-(4-fluorophenyl)-N-(2-pyridin-3-ylethyl)-2-[(2-pyridin-4-ylethyl)amino]acetamide;
2-(1-naphthyl)-N-(2-pyridin-4-ylethyl)-2-[(2-pyridin-4-ylethyl)amino]acetamide;
2-(1-naphthyl)-N-(pyridin-4-ylmethyl)-2-[(pyridin-4-ylmethyl)amino]acetamide;
2-(1-naphthyl)-2-[(2-piperidin-4-ylethyl)amino]-N-(2-pyridin-4-ylethyl)acetamide;
2-(1-naphthyl)-2-[(2-piperidin-4-ylethyl)amino]-N-(pyridin-4-ylmethyl)acetamide;
2-(1-naphthyl)-N-(pyridin-3-ylmethyl)-2-[(pyridin-3-ylmethyl)amino]acetamide;
2-(1-naphthyl)-N-(pyridin-4-ylmethyl)-2-[(pyridin-3-ylmethyl)amino]acetamide;
2-(1-naphthyl)-N-(pyridin-3-ylmethyl)-2-[(pyridin-4-ylmethyl)amino]acetamide;
2-(1-naphthyl)-N-(2-pyridin-3-ylethyl)-2-[(2-pyridin-3-ylethyl)amino]acetamide;
2-(1-naphthyl)-N-(2-pyridin-4-ylethyl)-2-[(2-pyridin-3-ylethyl)amino]acetamide;
2-(1-naphthyl)-N-(2-pyridin-3-ylethyl)-2-[(2-pyridin-4-ylethyl)amino]acetamide;
2-(1,3-benzodioxole-5-yl)-N-(2-pyridin-4-ylethyl)-2-[(2-pyridin-4-ylethyl)amino]acetamide;
2-(1,3-benzodioxole-5-yl)-N-(pyridin-4-ylmethyl)-2-[(pyridin-4-ylmethyl)amino]acetamide;
2-(1,3-benzodioxole-5-yl)-2-[(2-piperidin-4-ylethyl)amino]-N-(2-piperidin-4-ylethyl)acetamide;
2-(1,3-benzodioxole-5-yl)-2-[(2-piperidin-4-ylethyl)amino]-N-(pyridin-4-ylmethyl)acetamide;
2-(1,3-benzodioxole-5-yl)-N-(pyridin-3-ylmethyl)-2-[(pyridin-3-ylmethyl)amino]acetamide;
2-(1,3-benzodioxole-5-yl)-N-(pyridin-4-ylmethyl)-2-[(pyridin-3-ylmethyl)amino]acetamide;
2-(1,3-benzodioxole-5-yl)-N-(pyridin-3-ylmethyl)-2-[(pyridin-4-ylmethyl)amino]acetamide;
2-(1,3-benzodioxole-5-yl)-N-(2-pyridin-3-ylethyl)-2-[(2-pyridin-3-ylethyl)-amino]acetamide;
2-(1,3-benzodioxole-5-yl)-N-(2-pyridin-4-ylethyl)-2-[(2-pyridin-3-ylethyl)-amino]acetamide;
2-(1,3-benzodioxole-5-yl)-N-(2-pyridin-3-ylethyl)-2-[(2-pyridin-4-ylethyl)amino]acetamide;
2-(2-methylphenyl)-N-(2-pyridin-4-ylethyl)-2-[(2-pyridin-4-ylethyl)amino]acetamide;
2-(2-methylphenyl)-N-(pyridin-4-ylmethyl)-2-[(pyridin-4-ylmethyl)amino]acetamide;
2-(2-methylphenyl)-2-[(2-piperidin-4-ylethyl) amino]-N-(2-pyridin-4-ylethyl)acetamide;
2-(2-methylphenyl)-2-[(2-piperidin-4-ylethyl) amino]-N-(pyridin-4-ylmethyl)acetamide;
2-(2-methylphenyl)-N-(pyridin-3-ylmethyl)-2-[(pyridin-3-ylmethyl)amino]acetamide;
2-(2-methylphenyl)-N-(pyridin-4-ylmethyl)-2-[(pyridin-3-ylmethyl)amino] acetamide;
2-(2-methylphenyl)-N-(pyridin-3-ylmethyl)-2-[(pyridin-4-ylmethyl)amino]acetamide;
2-(2-methylphenyl)-N-(2-pyridin-3-ylethyl)-2-[(2-pyridin-3-ylethyl)amino]acetamide;
2-(2-methylphenyl)-N-(2-pyridin-4-ylethyl)-2-[(2-pyridin-3-ylethyl)amino]acetamide;
2-(2-methylphenyl)-N-(2-pyridin-3-ylethyl)-2-[(2-pyridin-4-ylethyl)amino]acetamide;
2-(3-methylphenyl)-N-(2-pyridin-4-ylethyl)-2-[(2-pyridin-4-ylethyl)amino]acetamide;
2-(3-methylphenyl)-N-(pyridin-4-ylmethyl)-2-[(pyridin-4-ylmethyl)amino] acetamide;
2-(3-methylphenyl)-2-[(2-piperidin-4-ylethyl) amino]-N-(2-pyridin-4-ylethyl)acetamide;
2-(3-methylphenyl)-2-[(2-piperidin-4-ylethyl)-amino]-N-(pyridin-4-ylmethyl)acetamide;
2-(3-methylphenyl)-N-(pyridin-3-ylmethyl)-2-[(pyridin-3-ylmethyl)amino]acetamide;
2-(3-methylphenyl)-N-(pyridin-4-ylmethyl)-2-[(pyridin-3-ylmethyl)amino] acetamide;
2-(3-methylphenyl)-N-(pyridin-3-ylmethyl)-2-[(pyridin-4-ylmethyl)amino]acetamide;
2-(3-methylphenyl)-N-(2-pyridin-3-ylethyl)-2-[(2-pyridin-3-ylethyl)amino]acetamide;
2-(3-methylphenyl)-N-(2-pyridin-4-ylethyl)-2-[(2-pyridin-3-ylethyl)amino]acetamide;
2-(3-methylphenyl)-N-(2-pyridin-3-ylethyl)-2-[(2-pyridin-4-ylethyl)amino]acetamide;
2-(4-methylphenyl)-N-(2-pyridin-4-ylethyl)-2-[(2-pyridin-4-ylethyl)amino]acetamide;
2-(4-methylphenyl)-N-(pyridin-4-ylmethyl)-2-[(pyridin-4-ylmethyl)amino]acetamide;
2-(4-methylphenyl)-2-[(2-piperidin-4-ylethyl)amino]-N-(2-pyridin-4-ylethyl)acetamide;
2-(4-methylphenyl)-2-[(2-piperidin-4-ylethyl)amino]-N-(pyridin-4-ylmethyl)acetamide;
2-(4-methylphenyl)-N-(pyridin-3-ylmethyl)-2-[(pyridin-3-ylmethyl)amino]acetamide;
2-(4-methylphenyl)-N-(pyridin-4-ylmethyl)-2-[(pyridin-3-ylmethyl)amino] acetamide;
2-(4-methylphenyl)-N-(pyridin-3-ylmethyl)-2-[(pyridin-4-ylmethyl)amino]acetamide;
2-(4-methylphenyl)-N-(2-pyridin-3-ylethyl)-2-[(2-pyridin-3-ylethyl)amino]acetamide;
2-(4-methylphenyl)-N-(2-pyridin-4-ylethyl)-2-[(2-pyridin-3-ylethyl)amino]acetamide;
2-(4-methylphenyl)-N-(2-pyridin-3-ylethyl)-2-[(2-pyridin-4-ylethyl)amino]acetamide;
2-(4-ethylphenyl)-N-(2-pyridin-4-ylethyl)-2-[(2-pyridin-4-ylethyl)amino]acetamide;
2-(4-ethylphenyl)-N-(pyridin-4-ylmethyl)-2-[(pyridin-4-ylmethyl)amino]acetamide;
2-(4-ethylphenyl)-2-[(2-piperidin-4-ylethyl) amino]-N-(2-pyridin-4-ylethyl)acetamide;
2-(4-ethylphenyl)-2-[(2-piperidin-4-ylethyl) amino]-N-(pyridin-4-ylmethyl)acetamide;
2-(4-ethylphenyl)-N-(pyridin-3-ylmethyl)-2-[(pyridin-3-ylmethyl)amino]acetamide;
2-(4-ethylphenyl)-N-(pyridin-4-ylmethyl)-2-[(pyridin-3-ylmethyl)amino]acetamide;
2-(4-ethylphenyl)-N-(pyridin-3-ylmethyl)-2-[(pyridin-4-ylmethyl)amino]acetamide;
2-(4-ethylphenyl)-N-(2-pyridin-3-ylethyl)-2-[(2-pyridin-3-ylethyl)amino]acetamide;
2-(4-ethylphenyl)-N-(2-pyridin-4-ylethyl)-2-[(2-pyridin-3-ylethyl)amino]acetamide;
2-(4-ethylphenyl)-N-(2-pyridin-3-ylethyl)-2-[(2-pyridin-4-ylethyl)amino]acetamide;
2-(4-isopropylphenyl)-N-(2-pyridin-4-ylethyl)-2-[(2-pyridin-4-ylethyl)amino]acetamide;
2-(4-isopropylphenyl)-N-(pyridin-4-ylmethyl)-2-[(pyridin-4-ylmethyl)amino]acetamide;
2-(4-isopropylphenyl)-2-[(2-piperidin-4-ylethyl)amino]-N-(2-piperidin-4-ylethyl)-acetamide;
2-(4-isopropylphenyl)-2-[(2-piperidin-4-ylethyl)amino]-N-(pyridin-4-ylmethyl)acetamide;
2-(4-isopropylphenyl)-N-(pyridin-3-ylmethyl)-2-[(pyridin-3-ylmethyl)amino]acetamide;
2-(4-isopropylphenyl)-N-(pyridin-4-ylmethyl)-2-[(pyridin-3-ylmethyl)amino]acetamide;
2-(4-isopropylphenyl)-N-(pyridin-3-ylmethyl)-2-[(pyridin-4-ylmethyl)amino]acetamide;
2-(4-isopropylphenyl)-N-(2-pyridin-3-ylethyl)-2-[(2-pyridin-3-ylethyl)amino]acetamide;
2-(4-isopropylphenyl)-N-(2-pyridin-4-ylethyl)-2-[(2-pyridin-3-ylethyl)amino]acetamide;
2-(4-isopropylphenyl)-N-(2-pyridin-3-ylethyl)-2-[(2-pyridin-4-ylethyl)amino]acetamide;
2-(3,5-dimethylphenyl)-N-(2-pyridin-4-ylethyl)-2-[(2-pyridin-4-ylethyl)amino]acetamide;
2-(3,5-dimethylphenyl)-N-(pyridin-4-ylmethyl)-2-[(pyridin-4-ylmethyl)amino]acetamide;
2-(3,5-dimethylphenyl)-2-[(2-piperidin-4-ylethyl)amino]-N-(2-pyridin-4-ylethyl)acetamide;
2-(3,5-dimethylphenyl)-2-[(2-piperidin-4-ylethyl)amino]-N-(pyridin-4-ylmethyl) acetamide;
2-(3,5-dimethylphenyl)-N-(pyridin-3-ylmethyl)-2-[(pyridin-3-ylmethyl)amino]acetamide;
2-(3,5-dimethylphenyl)-N-(pyridin-4-ylmethyl)-2-[(pyridin-3-ylmethyl)amino]acetamide;
2-(3,5-dimethylphenyl)-N-(pyridin-3-ylmethyl)-2-[(pyridin-4-ylmethyl)amino]acetamide;
2-(3,5-dimethylphenyl)-N-(2-pyridin-3-ylethyl)-2-[(2-pyridin-3-ylethyl)amino]- acetamide;
2-(3,5-dimethylphenyl)-N-(2-pyridin-4-ylethyl)-2-[(2-pyridin-3-ylethyl)-amino]acetamide;
2-(3,5-dimethylphenyl)-N-(2-pyridin-3-ylethyl)-2-[(2-pyridin-4-ylethyl)amino]acetamide;
2-(2-naphthyl)-N-(2-pyridin-4-ylethyl)-2-[(2-pyridin-4-ylethyl)amino]acetamide;
2-(2-naphthyl)-N-(pyridin-4-ylmethyl)-2-[(pyridin-4-ylmethyl)amino]acetamide;
2-(2-naphthyl)-2-[(2-piperidin-4-ylethyl)amino]-N-(2-pyridin-4-ylethyl)acetamide;
2-(2-naphthyl)-2-[(2-piperidin-4-ylethyl) amino]-N-(pyridin-4-ylmethyl)acetamide;
2-(2-naphthyl)-N-(pyridin-3-ylmethyl)-2-[(pyridin-3-ylmethyl) amino]acetamide;
2-(2-naphthyl)-N-(pyridin-4-ylmethyl)-2-[(pyridin-3-ylmethyl)amino]acetamide;
2-(2-naphthyl)-N-(pyridin-3-ylmethyl)-2-[(pyridin-4-ylmethyl)amino]acetamide;
2-(2-naphthyl)-N-(2-pyridin-3-ylethyl)-2-[(2-pyridin-3-ylethyl)amino]acetamide;
2-(2-naphthyl)-N-(2-pyridin-4-ylethyl)-2-[(2-pyridin-3-ylethyl)amino]acetamide;
2-(2-naphthyl)-N-(2-pyridin-3-ylethyl)-2-[(2-pyridin-4-ylethyl)amino]acetamide;
N-(2-pyridin-4-ylethyl)-2-[(2-pyridin-4-ylethyl)amino]-2-(2,4,5-trimethylphenyl)acetamide;
N-(pyridin-4-ylmethyl)-2-[(pyridin-4-ylmethyl)amino]-2-(2,4,5-trimethylphenyl)acetamide;
2-[(2-piperidin-4-ylethyl)-amino]-N-(2-pyridin-4-ylethyl)-2-(2,4,5-trimethylphenyl)acetamide;
2-[(2-piperidin-4-ylethyl)amino]-N-(pyridin-4-ylmethyl)-2-(2,4,5-trimethylphenyl)acetamide;
N-(pyridin-3-ylmethyl)-2-[(pyridin-3-ylmethyl)amino]-2-(2,4,5-trimethylphenyl)acetamide;
N-(pyridin-4-ylmethyl)-2-[(pyridin-3-ylmethyl)amino]-2-(2,4,5-trimethylphenyl)acetamide;
N-(pyridin-3-ylmethyl)-2-[(pyridin-4-ylmethyl)amino]-2-(2,4,5-trimethylphenyl)acetamide;
N-(2-pyridin-3-ylethyl)-2-[(2-pyridin-3-ylethyl)amino]-2-(2,4,5-trimethylphenyl)acetamide;
N-(2-pyridin-4-ylethyl)-2-[(2-pyridin-3-ylethyl)amino]-2-(2,4,5-trimethylphenyl)acetamide;
N-(2-pyridin-3-ylethyl)-2-[(2-pyridin-4-ylethyl)amino]-2-(2,4,5-trimethylphenyl)acetamide;
2-(4-tert-butylphenyl)-N-(2-pyridin-4-ylethyl)-2-[(2-pyridin-4-ylethyl)amino]acetamide;
2-(4-tert-butylphenyl)-N-(pyridin-4-ylmethyl)-2-[(pyridin-4-ylmethyl)amino]acetamide;
2-(4-tert-butylphenyl)-2-[(2-piperidin-4-ylethyl)amino]-N-(2-pyridin-4-ylethyl)acetamide;
2-(4-tert-butylphenyl)-2-[(2-piperidin-4-ylethyl)amino]-N-(pyridin-4-ylmethyl)acetamide;
2-(4-tert-butylphenyl)-N-(pyridin-3-ylmethyl)-2-[(pyridin-3-ylmethyl)amino]acetamide;
2-(4-tert-butylphenyl)-N-(pyridin-4-ylmethyl)-2-[(pyridin-3-ylmethyl)amino]acetamide;
2-(4-tert-butylphenyl)-N-(pyridin-3-ylmethyl)-2-[(-(pyridin-4-ylmethyl)amino]acetamide;
2-(4-tert-butylphenyl)-N-(2-pyridin-3-ylethyl)-2-[(2-pyridin-3-ylethyl)amino]acetamide;
2-(4-tert-butylphenyl)-N-(2-pyridin-4-ylethyl)-2-[(2-pyridin-3-ylethyl)amino]acetamide;
2-(4-tert-butylphenyl)-N-(2-pyridin-3-ylethyl)-2-[(2-pyridin-4-ylethyl)amino]acetamide;
2-[4-(dimethylamino)phenyl]-N-(2-pyridin-4-ylethyl)-2-[(2-pyridin-4-ylethyl)mino]acetamide;
2-[4-(dimethylamino)phenyl]-N-(pyridin-4-ylmethyl)-2-[(pyridin-4-ylmethyl)amino]acetamide;
2-[4-(dimethylamino)phenyl]-2-[(2-piperidin-4-ylethyl)amino]-N-(2-pyridin-4-ylethyl)acetamide;
2-[4-(dimethylamino)phenyl]-2-[(2-piperidin-4-ylethyl)amino]-N-(pyridin-4-ylmethyl)acetamide;
2-[4-(dimethylamino)phenyl]-N-(pyridin-3-ylmethyl)-2-[(pyridin-3-ylmethyl)amino]acetamide;
2-[4-(dimethylamino)phenyl]-N-(pyridin-4-ylmethyl)-2-[(pyridin-3-ylmethyl)amino]acetamide;
2-[4-(dimethylamino)phenyl]-N-(pyridin-3-ylmethyl)-2-[(pyridin-4-ylmethyl)amino]acetamide;
2-[4-(dimethylamino)phenyl]-N-(2-pyridin-3-ylethyl)-2-[(2-pyridin-3-ylethyl)amino]acetamide;
2-[4-(dimethylamino)phenyl]-N-(2-pyridin-4-ylethyl)-2-[(2-pyridin-3-ylethyl)amino]acetamide;
2-[4-(dimethylamino)phenyl]-N-(2-pyridin-3-ylethyl)-2-[(2-pyridin-4-ylethyl)amino]acetamide;
2-(4-propoxyphenyl)-N-(2-pyridin-4-ylethyl)-2-[(2-pyridin-4-ylethyl)amino]acetamide;
2-(4-propoxyphenyl)-N-(pyridin-4-ylmethyl)-2-[(pyridin-4-ylmethyl)amino]acetamide;
2-[(2-piperidin-4-ylethyl)amino]-2-(4-propoxyphenyl)-N-(2-pyridin-4-ylethyl)acetamide;
2-[(2-piperidin-4-ylethyl)amino]-2-(4-propoxyphenyl)-N-(pyridin-4-ylmethyl)-acetamide;
2-(4-propoxyphenyl)-N-(pyridin-3-ylmethyl)-2-[(pyridin-3-ylmethyl)amino]acetamide;
2-(4-propoxyphenyl)-N-(pyridin-4-ylmethyl)-2-[(pyridin-3-ylmethyl)amino]acetamide;
2-(4-propoxyphenyl)-N-(pyridin-3-ylmethyl)-2-[(pyridin-4-ylmethyl)amino]acetamide;
2-(4-propoxyphenyl)-N-(2-pyridin-3-ylethyl)-2-[(2-pyridin-3-ylethyl)amino]acetamide;
2-(4-propoxyphenyl)-N-(2-pyridin-4-ylethyl)-2-[(2-pyridin-3-ylethyl)amino]acetamide;
2-(4-propoxyphenyl)-N-(2-pyridin-3-ylethyl)-2-[(2-pyridin-4-ylethyl)amino]acetamide;
2-(4-butoxyphenyl)-N-(2-pyridin-4-ylethyl)-2-[(2-pyridin-4-ylethyl)amino]acetamide;
2-(4-butoxyphenyl)-N-(pyridin-4-ylmethyl)-2-[(pyridin-4-ylmethyl)amino]acetamide;
2-(4-butoxyphenyl)-2-[(2-piperidin-4-ylethyl)amino]-N-(2-pyridin-4-ylethyl)acetamide;
2-(4-butoxyphenyl)-2-[(2-piperidin-4-ylethyl)amino]-N-(pyridin-4-ylmethyl)acetamide;
2-(4-butoxyphenyl)-N-(pyridin-3-ylmethyl)-2-[(pyridin-3-ylmethyl)amino]acetamide;
2-(4-butoxyphenyl)-N-(pyridin-4-ylmethyl)-2-[(pyridin-3-ylmethyl)amino]acetamide;
2-(4-butoxyphenyl)-N-(pyridin-3-ylmethyl)-2-[(pyridin-4-ylmethyl)amino]acetamide;
2-(4-butoxyphenyl)-N-(2-pyridin-3-ylethyl)-2-[(2-pyridin-3-ylethyl)amino]acetamide;
2-(4-butoxyphenyl)-N-(2-pyridin-4-ylethyl)-2-[(2-piperidin-3- ylethyl)amino]acetamide;
2-(4-butoxyphenyl)-N-(2-pyridin-3-ylethyl)-2-[(2-pyridin-4-ylethyl)amino]acetamide;
2-(2,6-dichlorophenyl)-N-(2-pyridin-4-ylethyl)-2-[(2-pyridin-4-ylethyl)amino]-acetamide;
2-(2,6-dichlorophenyl)-N-(pyridin-4-ylmethyl)-2-[(pyridin-4-ylmethyl)amino]acetamide;
2-(2,6-dichlorophenyl)-2-[(2-piperidin-4-ylethyl)amino]-N-(2-pyridin-4-ylethyl)acetamide;
2-(2,6-dichlorophenyl)-2-[(2-piperidin-4-ylethyl)amino]-N-(pyridin-4-ylmethyl)acetamide;
2-(2,6-dichlorophenyl)-N-(pyridin-3-ylmethyl)-2-[(pyridin-3-ylmethyl)amino]acetamide;
2-(2,6-dichlorophenyl)-N-(pyridin-4-ylmethyl)-2-[(pyridin-3-ylmethyl)amino]acetamide;
2-(2,6-dichlorophenyl)-N-(pyridin-3-ylmethyl)-2-[(pyridin-4-ylmethyl)amino]acetamide;
2-(2,6-dichlorophenyl)-N-(2-pyridin-3-ylethyl)-2-[(2-pyridin-3-ylethyl)amino]acetamide;
2-(2,6-dichlorophenyl)-N-(2-pyridin-4-ylethyl)-2-[(2-pyridin-3-ylethyl)amino]acetamide;
2-(2,6-dichlorophenyl)-N-(2-pyridin-3-ylethyl)-2-[(2-pyridin-4-ylethyl)amino]acetamide;
2-(2-chloro-6-fluorophenyl)-N-(2-pyridin-4-ylethyl)-2-[(2-pyridin-4-ylethyl)amino]acetamide;
2-(2-chloro-6-fluorophenyl)-N-(pyridin-4-ylmethyl)-2-[(pyridin-4-ylmethyl)amino]acetamide;
2-(2-chloro-6-fluorophenyl)-2-[(2-piperidin-4-ylethyl)amino]-N-(2-pyridin-4-ylethyl)acetamide;
2-(2-chloro-6-fluorophenyl)-2-[(2-piperidin-4-ylethyl)amino]-N-(pyridin-4-ylmethyl)acetamide;
2-(2-chloro-6-fluorophenyl)-N-(pyridin-3-ylmethyl)-2-[(pyridin-3-ylmethyl)amino]acetamide;
2-(2-chloro-6-fluorophenyl)-N-(pyridin-4-ylmethyl)-2-[(pyridin-3-ylmethyl)amino]acetamide;
2-(2-chloro-6-fluorophenyl)-N-(pyridin-3-ylmethyl)-2-[(pyridin-4-ylmethyl)amino]acetamide;
2-(2-chloro-6-fluorophenyl)-N-(2-pyridin-3-ylethyl)-2-[(2-pyridin-3-ylethyl)amino]acetamide;
2-(2-chloro-6-fluorophenyl)-N-(2-pyridin-4-ylethyl)-2-[(2-pyridin-3-ylethyl)amino]acetamide;
2-(2-chloro-6-fluorophenyl)-N-(2-pyridin-3-ylethyl)-2-[(2-pyridin-4-ylethyl)amino]acetamide;
2-(2,3-dimethylphenyl)-N-(2-pyridin-4-ylethyl)-2-[(2-pyridin-4-ylethyl)amino]acetamide;
2-(2,3-dimethylphenyl)-N-(pyridin-4-ylmethyl)-2-[(pyridin-4-ylmethyl)amino]acetamide;
2-(2,3-dimethylphenyl)-2-[(2-piperidin-4-ylethyl)amino]-N-(2-pyridin-4-ylethyl)acetamide;
2-(2,3-dimethylphenyl)-2-[(2-piperidin-4-ylethyl)amino]-N-(pyridin-4-ylmethyl)acetamide;
2-(2,3-dimethylphenyl)-N-(pyridin-3-ylmethyl)-2-[(pyridin-3-ylmethyl)amino] acetamide;
2-(2,3-dimethylphenyl)-N-(pyridin-4-ylmethyl)-2-[(pyridin-3-ylmethyl)amino]acetamide;
2-(2,3-dimethylphenyl)-N-(pyridin-3-ylmethyl)-2-[(pyridin-4-ylmethyl)amino]acetamide;
2-(2,3-dimethylphenyl)-N-(2-pyridin-3-ylethyl)-2-[(2-pyridin-3-ylethyl)amino]acetamide;
2-(2,3-dimethylphenyl)-N-(2-pyridin-4-ylethyl)-2-[(2-pyridin-3-ylethyl)- amino]acetamide;
2-(2,3-dimethylphenyl)-N-(2-pyridin-3-ylethyl)-2-[(2-pyridin-4-ylethyl)amino]acetamide;
2-(4-hydroxy-3-methoxyphenyl)-N-(2-pyridin-4-ylethyl)-2-[(2-pyridin-4-ylethyl)amino]acetamide;
2-(4-hydroxy-3-methoxyphenyl)-N-(pyridin-4-ylmethyl)-2-[(pyridin-4-ylmethyl)amino]acetamide;
2-(4-hydroxy-3-methoxyphenyl)-2-[(2-piperidin-4-ylethyl)amino]-N-(2-pyridin-4-ylethyl)-acetamide;
2-(4-hydroxy-3-methoxyphenyl)-2-[(2-piperidin-4-ylethyl)amino]-N-(pyridin-4-ylethyl)acetamide;
2-(4-hydroxy-3-methoxyphenyl)-N-(pyridin-3-ylmethyl)-2-[(pyridin-3-ylmethyl)amino]acetamide;
2-(4-hydroxy-3-methoxyphenyl)-N-(pyridin-4-ylmethyl)-2-[(pyridin-3-ylmethyl)amino]acetamide;
2-(4-hydroxy-3-methoxyphenyl)-N-(pyridin-3-ylmethyl)-2-[(pyridin-4-ylmethyl)amino]acetamide;
2-(4-hydroxy-3-methoxyphenyl)-N-(2-pyridin-3-ylethyl)-2-[(2-pyridin-3-ylethyl)-amino] acetamide;
2-(4-hydroxy-3-methoxyphenyl)-N-(2-pyridin-4-ylethyl)-2-[(2-pyridin-3-ylethyl)amino]acetamide;
2-(4-hydroxy-3-methoxyphenyl)-N-(2-pyridin-3-ylethyl)-2-[(2-pyridin-4-ylethyl)amino]acetamide;
N-(2-pyridin-4-ylethyl)-2-[(2-pyridin-4-ylethyl)amino]-2-(2-thienyl)acetamide;
N-(pyridin-4-ylmethyl)-2-[(pyridin-4-ylmethyl)amino]-2-(2-thienyl)acetamide;
2-[(2-piperidin-4-ylethyl)amino]-N-(2-piperidin-4-ylethyl)-2-(2-thienyl)acetamide;
2-[(2-piperidin-4-ylethyl)amino]-N-(pyridin-4-ylmethyl)-2-(2-thienyl)acetamide;
N-(pyridin-3-ylmethyl)-2-[(pyridin-3-ylmethyl)amino]-2-(2-thienyl)acetamide;
N-(pyridin-4-ylmethyl)-2-[(pyridin-3-ylmethyl)amino]-2-(2-thienyl)acetamide;
N-(pyridin-3-ylmethyl)-2-[(-(pyridin-4-ylmethyl)amino]-2-(2-thienyl)acetamide;
N-(2-pyridin-3-ylethyl)-2-[(2-pyridin-3-ylethyl)amino]-2-(2-thienyl)acetamide;
N-(2-pyridin-4-ylethyl)-2-[(2-pyridin-3-ylethyl)amino]-2-(2-thienyl)acetamide;
N-(2-pyridin-3-ylethyl)-2-[(2-pyridin-4-ylethyl)amino]-2-(2-thienyl)acetamide;
2-(5-methyl-2-thienyl)-N-(2-pyridin-4-ylethyl)-2-[(2-pyridin-4-ylethyl)amino]acetamide;
2-(5-methyl-2-thienyl)-N-(pyridin-4-ylmethyl)-2-[(pyridin-4-ylmethyl)amino]acetamide;
2-(5-methyl-2-thienyl)-2-[(2-piperidin-4-ylethyl)amino]-N-(2-pyridin-4-ylethyl)acetamide;
2-(5-methyl-2-thienyl)-2-[(2-piperidin-4-ylethyl)amino]-N-(pyridin-4-ylmethyl)acetamide;
2-(5-methyl-2-thienyl)-N-(pyridin-3-ylmethyl)-2-[(pyridin-3-ylmethyl)amino]acetamide;
2-(5-methyl-2-thienyl)-N-(pyridin-4-ylmethyl)-2-[(pyridin-3-ylmethyl)amino]acetamide;
2-(5-methyl-2-thienyl)-N-(pyridin-3-ylmethyl)-2-[(pyridin-4-ylmethyl)amino]acetamide;
2-(5-methyl-2-thienyl)-N-(2-pyridin-3-ylethyl)-2-[(2-pyridin-3-ylethyl)amino]acetamide;
2-(5-methyl-2-thienyl)-N-(2-pyridin-4-ylethyl)-2-[(2-pyridin-3-ylethyl)amino]acetamide;
2-(5-methyl-2-thienyl)-N-(2-pyridin-3-ylethyl)-2-[(2-pyridin-4-ylethyl)amino]acetamide;
2-(3-methyl-2-thienyl)-N-(2-pyridin-4-ylethyl)-2-[(2-pyridin-4-ylethyl)amino]acetamide;
2-(3-methyl-2-thienyl)-N-(pyridin-4-ylmethyl)-2-[(pyridin-4-ylmethyl)amino]acetamide;
2-(3-methyl-2-thienyl)-2-[(2-piperidin-4-ylethyl)amino]-N-(2-pyridin-4-ylethyl)acetamide;
2-(3-methyl-2-thienyl)-2-[(2-piperidin-4-ylethyl)amino]-N-(pyridin-4-ylmethyl)acetamide;
2-(3-methyl-2-thienyl)-N-(pyridin-3-ylmethyl)-2-[(pyridin-3-ylmethyl)amino]acetamide;
2-(3-methyl-2-thienyl)-N-(pyridin-4-ylmethyl)-2-[(pyridin-3-ylmethyl)amino]acetamide;
2-(3-methyl-2-thienyl)-N-(pyridin-3-ylmethyl)-2-[(pyridin-4-ylmethyl)amino]acetamide;
2-(3-methyl-2-thienyl)-N-(2-pyridin-3-ylethyl)-2-[(2-pyridin-3-ylethyl)amino]acetamide;
2-(3-methyl-2-thienyl)-N-(2-pyridin-4-ylethyl)-2-[(2-pyridin-3-ylethyl)amino]-acetamide;
2-(3-methyl-2-thienyl)-N-(2-pyridin-3-ylethyl)-2-[(2-pyridin-4-ylmethyl)amino]acetamide;
N-(2-pyridin-4-ylethyl)-2-[(2-pyridin-4-ylethyl)amino]-2-(3-thienyl)acetamide;
N-(pyridin-4-ylmethyl)-2-[(pyridin-4-ylmethyl)amino]-2-(3-thienyl)acetamide;
2-[(2-piperidin-4-ylethyl)amino]-N-(2-pyridin-4-ylethyl)-2-(3-thienyl) acetamide;
2-[(2-piperidin-4-ylethyl)amino]-N-(pyridin-4-ylmethyl)-2-(3-thienyl)acetamide;
N-(pyridin-3-ylmethyl)-2-[(pyridin-3-ylmethyl)amino]-2-(3-thienyl)acetamide;
N-(pyridin-4-ylmethyl)-2-[(pyridin-3-ylmethyl)amino]-2-(3-thienyl)acetamide;
N-(pyridin-3-ylmethyl)-2-[(pyridin-4-ylmethyl)amino]-2-(3-thienyl)acetamide;
N-(2-pyridin-3-ylethyl)-2-[(2-pyridin-3-ylethyl)amino]-2-(3-thienyl)acetamide;
N-(2-pyridin-4-ylethyl)-2-[(2-pyridin-3-ylethyl)amino]-2-(3-thienyl)acetamide;
N-(2-pyridin-3-ylethyl)-2-[(2-pyridin-4-ylethyl)amino]-2-(3-thienyl)acetamide;
2-pyridin-4-yl-N-(2-pyridin-4-ylethyl)-2-[(2-pyridin-4-ylethyl)amino]acetamide;
2-pyridin-4-yl-N-(pyridin-4-ylmethyl)-2-[(pyridin-4-ylmethyl)amino]acetamide;
2-[(2-piperidin-4-ylethyl)amino]-2-pyridin-4-yl-N-(2-pyridin-4-ylethyl)acetamide;
2-[(2-piperidin-4-ylethyl)amino]-2-pyridin-4-yl-N-(pyridin-4-ylmethyl)acetamide;
2-pyridin-4-yl-N-(pyridin-3-ylmethyl)-2-[(pyridin-3-ylmethyl)amino]acetamide;
2-pyridin-4-yl-N-(pyridin-4-ylmethyl)-2-[(pyridin-3-ylmethyl)amino]acetamide;
2-pyridin-4-yl-N-(pyridin-3-ylmethyl)-2-[(pyridin-4-ylmethyl)amino]acetamide;
2-pyridin-4-yl-N-(2-pyridin-3-ylethyl)-2-[(2-pyridin-3-ylethyl)amino]acetamide;
2-pyridin-4-yl-N-(2-pyridin-4-ylethyl)-2-[(2-pyridin-3-ylethyl)amino]acetamide;
2-pyridin-4-yl-N-(2-pyridin-3-ylethyl)-2-[(2-pyridin-4-ylethyl)amino]acetamide;
2-pyridin-2-yl-N-(2-pyridin-4-ylethyl)-2-[(2-pyridin-4-ylethyl)amino]acetamide;
2-pyridin-2-yl-N-(pyridin-4-ylmethyl)-2-[(pyridin-4-ylmethyl)amino]acetamide;
2-[(2-piperidin-4-ylethyl)amino]-2-pyridin-2-yl-N-(2-pyridin-4-ylethyl)acetamide;
2-[(2-piperidin-4-ylethyl)amino]-2-pyridin-2-yl-N-(pyridin-4-ylmethyl)acetamide;
2-pyridin-2-yl-N-(pyridin-3-ylmethyl)-2-[(pyridin-3-ylmethyl)amino]acetamide;
2-pyridin-2-yl-N-(pyridin-4-ylmethyl)-2-[(pyridin-3-ylmethyl)amino]acetamide;
2-pyridin-2-yl-N-(pyridin-3-ylmethyl)-2-[(pyridin-4-ylmethylamino]acetamide;
2-pyridin-2-yl-N-(2-pyridin-3-ylethyl)-2-[(2-pyridin-3-ylethyl)amino]acetamide;
2-pyridin-2-yl-N-(2-pyridin-4-ylethyl)-2-[(2-pyridin-3-ylethyl)amino]acetamide;
2-pyridin-2-yl-N-(2-pyridin-3-ylethyl)-2-[(2-pyridin-4-ylethyl)amino]acetamide;
N-(2-pyridin-4-ylethyl)-2-[(2-pyridin-4-ylethyl)amino]-2-quinoline-2-ylacetamide;
N-(pyridin-4-ylmethyl)-2-[(pyridin-4-ylmethyl)amino]-2-quinoline-2-ylacetamide;
2-[(2-piperidin-4-ylethyl)amino]-N-(2-pyridin-4-ylethyl)-2-quinoline-2-ylacetamide;
2-[(2-piperidin-4-ylethyl)amino]-N-(pyridin-4-ylmethyl)-2-quinoline-2-ylacetamide;
N-(pyridin-3-ylmethyl)-2-[(pyridin-3-ylmethyl)amino]-2-quinoline-2-ylacetamide;
N-(pyridin-4-ylmethyl)-2-[(pyridin-3-ylmethyl)amino]-2-quinoline-2-ylacetamide;
N-(pyridin-3-ylmethyl)-2-[(pyridin-4-ylmethyl)amino]-2-quinoline-2-ylacetamide;
N-(2-pyridin-3-ylethyl)-2-[(2-pyridin-3-ylethyl)amino]-2-quinoline-2-ylacetamide;
N-(2-pyridin-4-ylethyl)-2-[(2-pyridin-3-ylethyl)amino]-2-quinoline-2-ylacetamide;
N-(2-pyridin-3-ylethyl)-2-[(2-pyridin-4-ylethyl)amino]-2-quinoline-2-ylacetamide;
2-(2,3-dimethoxyphenyl)-N-(2-pyridin-4-ylethyl)-2-[(2-pyridin-4-ylethyl)amino]acetamide;
2-(2,3-dimethoxyphenyl)-N-(pyridin-4-ylmethyl)-2-[(pyridin-4-ylmethyl)amino]acetamide;
2-(2,3-dimethoxyphenyl)-2-[(2-piperidin-4-ylethyl)amino]-N-(2-pyridin-4-ylethyl)acetamide;
2-(2,3-dimethoxyphenyl)-2-[(2-piperidin-4-ylethyl)amino]-N-(pyridin-4-ylmethyl)acetamide;
2-(2,3-dimethoxyphenyl)-N-(pyridin-3-ylmethyl)-2-[(pyridin-3-ylmethyl)amino]acetamide;
2-(2,3-dimethoxyphenyl)-N-(pyridin-4-ylethyl)-2-[(pyridin-3-ylmethyl)amino]acetamide;
2-(2,3-dimethoxyphenyl)-N-(pyridin-3-ylmethyl)-2-[(pyridin-4-ylmethyl)amino]acetamide;
2-(2,3-dimethoxyphenyl)-N-(2-pyridin-3-ylethyl)-2-[(2-pyridin-3-ylethyl)amino]acetamide;

2-(2,3-dimethoxyphenyl)-N-(2-pyridin-4-ylethyl)-2-[(2-pyridin-3ylethyl)amino]acetamide;
2-(2,3-dimethoxyphenyl)-N-(2-pyridin-3-ylethyl)-2-[(2-pyridin-4-ylethyl)amino]acetamide;
2-(4-nitrophenyl)-N-(2-pyridin-4-ylethyl)-2-[(2-pyridin-4-ylethyl)amino]acetamide;
2-(4-nitrophenyl)-N-(pyridin-4-ylmethyl)-2-[(pyridin-4-ylmethyl)amino]acetamide;
2-(4-nitrophenyl)-2-[(2-piperidin-4-ylethyl)amino]-N-(2-pyridin-4-ylethyl)acetamide;
2-(4-nitrophenyl)-2-[(2-piperidin-4-ylethyl) amino]-N-(pyridin-4-ylmethyl)acetamide;
2-(4-nitrophenyl)-N-(pyridin-3-ylmethyl)-2-[(pyridin-3-ylmethyl)amino]acetamide;
2-(4-nitrophenyl)-N-(pyridin-4-ylmethyl)-2-[(pyridin-3-ylmethyl)amino]acetamide;
2-(4-nitrophenyl)-N-(pyridin-3-ylmethyl)-2-[(pyridin-4-ylmethyl)amino]acetamide;
2-(4-nitrophenyl)-N-(2-pyridin-3-ylethyl)-2-[(2-pyridin-3-ylethyl)amino]acetamide;
2-(4-nitrophenyl)-N-(2-pyridin-4-ylethyl)-2-[(2-pyridin-3-ylethyl)amino]acetamide;
2-(4-nitrophenyl)-N-(2-pyridin-3-ylethyl)-2-[(2-pyridin-4-ylethyl)amino]acetamide;
2-(2-nitrophenyl)-N-(2-pyridin-4-ylethyl)-2-[(2-pyridin-4-ylethyl)amino]acetamide;
2-(2-nitrophenyl)-N-(pyridin-4-ylmethyl)-2-[(pyridin-4-ylmethyl)amino]acetamide;
2-(2-nitrophenyl)-2-[(2-piperidin-4-ylethyl)amino]-N-(2-pyridin-4-ylethyl)acetamide;
2-(2-nitrophenyl)-2-[(2-piperidin-4-ylethyl)amino]-N-(pyridin-4-ylmethyl)acetamide;
2-(2-nitrophenyl)-N-(pyridin-3-ylethyl)-2-[(pyridin-3-ylethyl)amino]acetamide;
2-(2-nitrophenyl)-N-(pyridin-4-ylmethyl)-2-[(pyridin-3-ylmethyl)amino]acetamide;
2-(2-nitrophenyl)-N-(pyridin-3-ylmethyl)-2-[(pyridin-4-ylmethyl)amino]acetamide;
2-(2-nitrophenyl)-N-(2-pyridin-3-ylethyl)-2-[(2-pyridin-3-ylethyl)amino]acetamide;
2-(2-nitrophenyl)-N-(2-pyridin-4-ylethyl)-2-[(2-pyridin-3-ylethyl)amino]acetamide;
2-(2-nitrophenyl)-N-(2-pyridin-3-ylethyl)-2-[(2-pyridin-4-ylethyl)amino]acetamide;
2-(3-nitrophenyl)-N-(2-pyridin-4-ylethyl)-2-[(2-pyridin-4-ylethyl)amino] acetamide;
2-(3-nitrophenyl)-N-(pyridin-4-ylmethyl)-2-[(pyridin-4-ylmethyl)amino]acetamide;
2-(3-nitrophenyl)-2-[(2-piperidin-4-ylethyl)amino]-N-(2-pyridin-4-ylethyl)acetamide;
2-(3-nitrophenyl)-2-[(2-piperidin-4-ylethyl)amino]-N-(pyridin-4-ylmethyl)acetamide;
2-(3-nitrophenyl)-N-(pyridin-3-ylmethyl)-2-[(pyridin-3-ylmethyl)amino]acetamide;
2-(3-nitrophenyl)-N-(pyridin-4-ylmethyl)-2-[(pyridin-3-ylmethyl)amino]acetamide;
2-(3-nitrophenyl)-N-(pyridin-3-ylmethyl)-2-[(pyridin-4-ylmethyl)amino]acetamide;
2-(3-nitrophenyl)-N-(2-pyridin-3-ylethyl)-2-[(2-pyridin-3-ylethyl)amino]acetamide;
2-(3-nitrophenyl)-N-(2-pyridin-4-ylethyl)-2-[(2-pyridin-3-ylethyl)amino]acetamide;
2-(3-nitrophenyl)-N-(2-pyridin-3-ylethyl)-2-[(2-pyridin-4-ylethyl)amino]acetamide;
2-pyridin-3-yl-N-(2-pyridin-4-ylethyl)-2-[(2-pyridin-4-ylethyl)amino]acetamide;
2-pyridin-3-yl-N-(pyridin-4-ylmethyl)-2-[(pyridin-4-ylmethyl)amino]acetamide;
2-[(2-piperidin-4-ylethyl)-amino]-2-pyridin-3-yl-N-(2-pyridin-4-ylethyl)acetamide;
2-[(2-piperidin-4-ylethyl)amino]-2-pyridin-3-yl-N-(pyridin-4-ylmethyl)acetamide;
2-pyridin-3-yl-N-(pyridin-3-ylmethyl)-2-[(pyridin-3-ylmethyl)amino]acetamide;
2-pyridin-3-yl-N-(pyridin-4-ylmethyl)-2-[(pyridin-3-ylmethyl)amino]acetamide;
2-pyridin-3-yl-N-(pyridin-3-ylmethyl)-2-[(pyridin-4-ylmethyl)amino]acetamide;
2-pyridin-3-yl-N-(2-pyridin-3-ylethyl)-2-[(2-pyridin-3-ylethyl)amino]acetamide;
2-pyridin-3-yl-N-(2-pyridin-4-ylethyl)-2-[(2-pyridin-3-ylethyl)amino]acetamide;
2-pyridin-3-yl-N-(2-pyridin-3-ylethyl)-2-[(2-pyridin-4-ylethyl)amino]acetamide;
2-(2,3-dichlorophenyl)-N-(2-pyridin-4-ylethyl)-2-[(2-pyridin-4-ylethyl)amino]acetamide;
2-(2,3-dichlorophenyl)-N-(pyridin-4-ylmethyl)-2-[(pyridin-4-ylmethyl)amino]acetamide;
2-(2,3-dichlorophenyl)-2-[(2-piperidin-4-ylethyl)amino]-N-(2-pyridin-4-ylethyl)acetamide;
2-(2,3-dichlorophenyl)-2-[(2-piperidin-4-ylethyl)amino]-N-(pyridin-4-ylmethyl)acetamide;
2-(2,3-dichlorophenyl)-N-(pyridin-3-ylmethyl)-2-[(pyridin-3-ylmethyl)amino]acetamide;
2-(2,3-dichlorophenyl)-N-(pyridin-4-ylmethyl)-2-[(pyridin-3-ylmethyl)amino]acetamide;
2-(2,3-dichlorophenyl)-N-(pyridin-3-ylmethyl)-2-[(pyridin-4-ylmethyl)amino]acetamide;
2-(2,3-dichlorophenyl)-N-(2-pyridin-3-ylethyl)-2-[(2-pyridin-3-ylethyl)amino]acetamide;
2-(2,3-dichlorophenyl)-N-(2-pyridin-4-ylethyl)-2-[(2-pyridin-3-ylethyl)amino]acetamide;
2-(2,3-dichlorophenyl)-N-(2-pyridin-3-ylethyl)-2-[(2-pyridin-4-ylethyl)amino]acetamide;
2-phenyl-N-(2-pyridin-4-ylethyl)-2-[(2-pyridin-4-ylethyl)amino]acetamide;
2-phenyl-N-(pyridin-4-ylmethyl)-2-[(pyridin-4-ylmethyl)amino]acetamide;
2-phenyl-2-[(2-piperidin-4-ylethyl)amino]-N-(2-pyridin-4-ylethyl)acetamide;
2-phenyl-2-[(2-piperidin-4-ylethyl)amino]-N-(pyridin-4-ylmethyl)acetamide;
2-phenyl-N-(pyridin-3-ylmethyl)-2-[(pyridin-3-ylmethyl)amino]acetamide;
2-phenyl-N-(pyridin-4-ylmethyl)-2-[(pyridin-3-ylmethyl)amino]acetamide;
2-phenyl-N-(pyridin-3-ylmethyl)-2-[(pyridin-4-ylmethyl)amino]acetamide;
2-phenyl-N-(2-pyridin-3-ylethyl)-2-[(2-pyridin-3-ylethyl)amino]acetamide;
2-phenyl-N-(2-pyridin-4-ylethyl)-2-[(2-pyridin-3-ylethyl)amino]acetamide;
2-phenyl-N-(2-pyridin-3-ylethyl)-2-[(2-pyridin-4-ylethyl)amino]acetamide;
2-(4-hydroxyphenyl)-N-(2-pyridin-4-ylethyl)-2-[(2-pyridin-4-ylethyl)amino]acetamide;
2-(4-hydroxyphenyl)-N-(pyridin-4-ylmethyl)-2-[(pyridin-4-ylmethyl)amino]acetamide;
2-(4-hydroxyphenyl)-2-[(2-piperidin-4-ylethyl) amino]-N-(2-pyridin-4-ylethyl)acetamide;
2-(4-hydroxyphenyl)-2-[(2-piperidin-4-ylethyl)amino]-N-(pyridin-4-ylmethyl)acetamide;
2-(4-hydroxyphenyl)-2-[(2-piperidin-4-ylethyl)amino]-N-(pyridin-4-ylmethyl)acetamide;
2-(4-hydroxyphenyl)-N-(pyridin-4-ylmethyl)-2-[(pyridin-3-ylmethyl)amino]acetamide;
2-(4-hydroxyphenyl)-N-(pyridin-3-ylmethyl)-2-[(pyridin-4-ylmethyl)amino]acetamide;
2-(4-hydroxyphenyl)-N-(2-pyridin-3-ylethyl)-2-[(2-pyriclin-3-ylethyl)amino]acetamide;
2-(4-hydroxyphenyl)-N-(2-pyridin-4-ylethyl)-2-[(2-pyridin-3-ylethyl)amino]acetamide;
2-(4-hydroxyphenyl)-N-(2-pyridin-3-ylethyl)-2-[(2-pyridin-4-ylethyl)amino]acetamide;
2-(3-hydroxyphenyl)-N-(2-pyridin-4-ylethyl)-2-[(2-pyridin-4-ylethyl)amino]acetamide;
2-(3-hydroxyphenyl)-N-(pyridin-4-ylmethyl)-2-[(pyridin -4-ylmethyl)amino]acetamide;
2-(3-hydroxyphenyl)-2-[(2-piperidin-4-ylethyl)amino]-N-(2-pyridin-4-ylethyl)acetamide;
2-(3-hydroxyphenyl)-2-[(2-piperidin-4-ylethyl)amino]-N-(pyridin-4-ylmethyl)acetamide;
2-(3-hydroxyphenyl)-N-(pyridin-3-ylmethyl)-2-[(pyridin-3-ylmethyl)amino]acetamide;
2-(3-hydroxyphenyl)-N-(pyridin-4-ylmethyl)-2-[(pyridin-3-ylmethyl)amino]acetamide;
2-(3-hydroxyphenyl)-N-(pyridin-3-ylmethyl)-2-[(pyridin-4-ylmethyl)amino]acetamide;
2-(3-hydroxyphenyl)-N-(2-pyridin-3-ylethyl)-2-[(2-pyridin-3-ylethyl)amino]acetamide;
2-(3-hydroxyphenyl)-N-(2-pyridin-4-ylethyl)-2-[(2-pyridin-3-ylethyl)amino]acetamide;
2-(3-hydroxyphenyl)-N-(2-pyridin-3-ylethyl)-2-[(2-pyridin-4-ylethyl)amino]acetamide;
2-(2-hydroxyphenyl)-N-(2-pyridin-4-ylethyl)-2-[(2-pyriclin-4-ylethyl)amino]acetamide;
2-(2-hydroxyphenyl)-N-(pyridin-4-ylmethyl)-2-[(pyridin-4-ylmethyl)amino]acetamide;
2-(2-hydroxyphenyl)-2-[(2-piperidin-4-ylethyl)amino]-N-(2-pyridin-4-ylethyl)acetamide;
2-(2-hydroxyphenyl)-2-[(2-piperidin-4-ylethyl) amino]-N-(pyridin-4-ylmethyl)acetamide;
2-(2-hydroxyphenyl)-N-(pyridin-3-ylmethyl)-2-[(pyridin-3-ylmethyl)amino]acetamide;
2-(2-hydroxyphenyl)-N-(pyridin-4-ylmethyl)-2-[(pyridin-3-ylmethyl)amino]acetamide;
2-(2-hydroxyphenyl)-N-(pyridin-3-ylmethyl)-2-[(pyridi-4-ylmethyl)amino]-acetamide;
2-(2-hydroxyphenyl)-N-(2-pyridin-3-ylethyl)-2-[(2-pyridin-3-ylethyl)amino]acetamide;
2-(2-hydroxyphenyl)-N-(2-pyridin-4-ylethyl)-2-[(2-pyridin-3-ylethyl)amino]acetamide;
2-(2-hydroxyphenyl)-N-(2-pyridm-3-ylethyl)-2-[(2-pyridin-4-ylethyl)amino]acetamide;
2-(2,3-dihydro-1,4-benzodioxin-6-yl)-N-(2-pyridin-4-ylethyl)-2-[(2-pyridin-4-ylethyl)amino] acetamide;
2-(2,3-dihydro-1,4-benzodioxin-6-yl)-N-(pyridin-4-ylmethyl)-2-[(pyridin-4-ylmethyl)amino] acetamide;
2-(2,3-dihydro-1,4-benzodioxin-6-yl)-2-[(2-piperidin-4-ylethyl)amino]-N-(2-pyridin-4-ylethyl) acetamide;
2-(2,3-dihydro-1,4-benzodioxin-6-yl)-2-[(2-piperidin-4-ylethyl)amino]-N-(pyridin-4-ylmethyl) acetamide;
2-(2,3-dihydro-1,4-benzodioxin-6-yl)-N-(pyridin-3-ylmethyl)-2-[(pyridin-3-ylmethyl)amino] acetamide;
2-(2,3-dihydro-1,4-benzodioxin-6-yl)-N-(pyridin-4-ylmethyl)-2-[(pyridin-3-ylmethyl)amino] acetamide;
2-(2,3-dihydro-1,4-benzodioxin-6-yl)-N-(pyridin-3-ylmethyl)-2-[(pyridin-4-ylmethyl)amino] acetamide;
2-(2,3-dihydro-1,4-benzodioxin-6-yl)-N-(2-pyridin-3-ylethyl)-2-[(2-pyridin-3-ylethyl)amino] acetamide;
2-(2,3-dihydro-1,4-benzodioxin-6-yl)-N-(2-pyridin-4-ylethyl)-2-[(2-pyridin-3-ylethyl) amino]acetamide;
2-(2,3-dihydro-1,4-benzodioxin-6-yl)-)-N-(2-pyridin-3-ylethyl)-2-[(2-pyridin-4-ylethyl)amino] acetamide;
N-(2-pyridin-4-ylethyl)-2-[(2-pyridin-4-ylethyl)amino]-2-(3,4,5-trimethoxyphenyl)acetamide;
N-(pyridin-4-ylmethyl)-2-[(pyridin-4-ylmethyl)amino]-2-(3,4,5-trimethoxyphenyl)-acetamide;
2-[(2-piperidin-4-ylethyl)amino]-N-(2-pyridm-4-ylethyl)-2-(3,4,5-trimethoxyphenyl)acetamide;
2-[(2-piperidin-4-ylethyl)amino]-N-(pyridin-4-ylmethyl)-2-(3,4,5-trimethoxyphenyl)acetamide;
N-(pyridin-3-ylmethyl)-2-[(pyridin-3-ylmethyl)amino]-2-(3,4,5-trimethoxyphenyl)acetamide;
N-(pyridin-4-ylmethyl)-2-[(pyridin-3-ylmethyl)amino]-2-(3,4,5-trimethoxyphenyl)acetamide;
N-(pyridin-3-ylmethyl)-2-[(pyridin-4-ylmethyl)amino]-2-(3,4,5-trimethoxyphenyl)acetamide;
N-(2-pyriclin-3-ylethyl)-2-[(2-pyridin-3-ylethyl)amino]-2-(3,4,5-trimethoxyphenyl)acetamide;
N-(2-pyridin-4-ylethyl)-2-[(2-pyridin-3-ylethyl)amino]-2-(3,4,5-trimethoxyphenyl)acetamide;
N-(2-pyridin-3-ylethyl)-2-[(2-pyridin-4-ylethyl)amino]-2-(3,4,5-trimethoxyphenyl)acetamide;
3-{2-oxo-1,2-bis[(2-pyridin-4-ylethyl)amino]ethyl}benzoic acid;
3-{2-oxo-1,2-bis[(pyridin-4-ylmethyl)amino]ethyl}benzoic acid;
3-{2-oxo-1,2-bis[(pyridin-4-ylmethyl)amino]ethyl}benzoic acid;
3-{2-oxo-1-[(2-piperidin-4-ylethyl)amino]-2-[(pyridin-4-ylmethyl)amino]ethyl}benzoic acid;
3-{2-oxo-1,2-bis[(pyridin-3-ylmethyl)amino]ethyl}benzoic acid;
3-{2-oxo-1-[(pyridin-3-ylmethyl)amino]-2-[(pyridin-4-ylmethyl)amino]ethyl}benzoic acid;
3-{2-oxo-2-[(pyridin-3-ylmethyl)amino]-1-[(pyridin-4-ylmethyl)amino]ethyl}benzoic acid;
3-{2-oxo-1,2-bis[(2-pyridin-3-ylethyl)amino]ethyl}benzoic acid;
3-{2-oxo-1-[(2-pyridin-3-ylethyl)amino]-2-[(2-pyridin-4-ylethyl)amino]ethyl}benzoic acid;
3-{2-oxo-2-[(2-pyridin-3-ylethyl)amino]-1-[(2-pyridin-4-ylethyl)amino]ethyl}benzoic acid;
4-{2-oxo-1,2-bis[(2-pyridin-4-ylethyl)amino]ethyl}benzoic acid;
4-{2-oxo-1,2-bis[(pyridin-4-ylmethyl)amino]ethyl}benzoic acid;
4-{2-oxo-1-[(2-piperidin-4-ylethyl)amino]-2-[(2-pyridin-4-ylethyl)amino]ethyl}benzoic acid;
4-{2-oxo-1-[(2-piperidin-4-ylethyl)amino]-2-[(pyridin-4-ylmethyl)amino]ethyl}benzoic acid;
4-{2-oxo-1,2-bis[(pyridin-3-ylmethyl)amino]ethyl}benzoic acid;
4-{2-oxo-1-[(pyridin-3-ylmethyl)amino]-2-[(piperidin-4-ylethyl)amino]ethyl}benzoic acid;
4-{2-oxo-2-[(pyridin-3-ylmethyl)amino]-1-[(pyridin-4-ylethyl)amino]ethyl}benzoic acid;
4-{2-oxo-1,2-bis[(2-pyridin-3-ylethyl)amino]ethyl}benzoic acid;
4-{2-oxo-1-[(2-pyridin-3-ylethyl)amino]-2-[(2-pyridin-4-ylethyl)amino]ethyl}benzoic acid;
4-{2-oxo-2-[(2-pyridin-3-ylethyl)amino]-1-[(2-pyridin-4-ylethyl)amino]ethyl}benzoic acid;
methyl 3-{2-oxo-1,2-bis[(2-pyridin-4-ylethyl)amino]ethyl}benzoate;
methyl 3-{2-oxo-1,2-bis[(pyridin-4-ylmethyl)amino]ethyl}benzoate;
methyl 3-{2-oxo-1-[(2-piperidin-4-ylethyl)amino]-2-[(2-pyridin-4-ylethyl)amino]ethyl}benzoate;
methyl 3-{2-oxo-1-[(2-piperidin-4-ylethyl)amino]-2-[(pyridin-4-ylmethyl)amino]ethyl}benzoate;
methyl 3-{2-oxo-1,2-bis[(pyridin-3-ylmethyl)amino]ethyl}benzoate;
methyl 3- {2-oxo-1,2-bis[(pyridin-3-ylmethyl)amino]ethyl}benzoate;
methyl 3-{2-oxo-2-[(pyridin-3-ylmethyl)amino]-1-[(pyridin-4-ylmethyl)amino]ethyl}benzoate;
methyl 3-{2-oxo-1,2-bis[(2-pyridin-3-ylethyl)amino]ethyl}benzoate;
methyl 3-{2-oxo-1-[(2-pyridin-3-ylethyl)amino]-2-[(2-pyridin-4-ylethyl)amino]ethyl}benzoate;
methyl 3-{2-oxo-2-[(2-pyridin-3-ylethyl)amino]-1-[(2-pyridin-4-ylethyl)amino]ethyl}benzoate;
methyl 4-{2-oxo-1,2-bis[(2-pyridin-4-ylethyl)amino]ethyl}benzoate;
methyl 4-{2-oxo-1,2-bis[(pyridin-4-ylmethyl)amino]ethyl}benzoate;
methyl 4-{2-oxo-1-[(2-piperidin-4-ylethyl)amino]-2-[(2-pyridin-4-ylethyl)amino]ethyl}benzoate;
methyl 4-{2-oxo-1-[(2-piperidin-4-ylethyl)amino]-2-[(pyridin-4-ylmethyl)amino]ethyl}benzoate;
methyl 4-{2-oxo-1,2-bis[(pyridin-3-ylmethyl)amino]ethyl}benzoate;
methyl 4-{2-oxo-1-[(pyridin-3-ylmethyl)amino]-2-[(pyridin-4-ylmethyl)amino]ethyl}benzoate;
methyl 4-{2-oxo-2-[(pyridin-3-ylmethyl)amino]-1-[(pyridin-4-ylmethyl)amino]ethyl}benzoate;
methyl 4-{2-oxo-1,2-bis[(2-pyridin-3-ylethyl)amino]ethyl}benzoate;
methyl 4-{2-oxo-1-[(2-pyridin-3-ylethyl)amino]-2-[(2-pyridin-4-ylethyl)amino]ethyl}benzoate;
methyl 4-{2-oxo-2-[(2-pyridin-3-ylethyl)amino]-1-[(2-pyridin-4-ylethyl)amino]ethyl}benzoate;
ethyl 4-{2-oxo-1,2-bis[(2-pyridin-4-ylethyl)amino]ethyl}benzoate;
ethyl 4-{2-oxo-1,2-bis[(pyridin-4-ylethyl)amino]ethyl} benzoate;
ethyl 4-{2-oxo-1-[(2-piperidin-4-ylethyl)amino]-2-[(2-pyridin-4-ylethyl)amino]ethyl}benzoate;
ethyl 4-{2-oxo-1-[(2-piperidin-4-ylethyl)amino]-2-[(pyridin-4-ylmethyl)amino]ethyl}benzoate;
ethyl 4-{2-oxo-1,2-bis[(pyridin-3-ylmethyl)amino]ethyl}benzoate;
ethyl 4-{2-oxo-1-[(pyridin-3-ylmethyl)amino]-2-[(pyridin-4-ylmethyl)amino]ethyl}benzoate;
ethyl 4-{2-oxo-2-[(pyridin-3-ylmethyl)amino]-1-[(pyridin-4-ylmethyl)amino]ethyl}benzoate;
ethyl4-{2-oxo-1,2-bis[(2-pyridin-3-ylethyl)amino]ethyl}benzoate;
ethyl 4-{2-oxo-1-[(2-pyridin-3-ylethyl)amino]-2-[(2-pyridin-4-ylethyl)amino]ethyl}benzoate;
ethyl 4-{2-oxo-2-[(2-pyridin-3-ylethyl)amino]-1-[(2-pyridin-4-ylethyl)amino]ethyl}benzoate;
ethyl 3-{2-oxo-1,2-bis[(2-pyridin-4-ylethyl)amino]ethyl}benzoate;
ethyl 3-{2-oxo-1,2-bis[(pyridin-4-ylmethyl)amino]ethyl}benzoate;
ethyl 3-{2-oxo-1-[(2-piperidin-4-ylethyl)amino]-2-[(2-pyridin-4-ylethyl)amino]ethyl}benzoate;
ethyl 3-{2-oxo-1-[(2-piperidin-4-ylethyl)amino]-2-[(pyridin-4-ylmethyl)amino]ethyl}benzoate;
ethyl 3-{2-oxo-1,2-bis[(pyridin-3-ylmethyl)amino]ethyl}benzoate;
ethyl 3-{2-oxo-1-[(pyridin-3-ylmethyl)amino]-2-[(pyridin-4-ylmethyl)amino]ethyl}benzoate;
ethyl 3-{2-oxo-2-[(pyridin-3-ylmethyl)amino]-1-[(pyridin-4-ylmethyl)amino]ethyl}benzoate;
ethyl 3-{2-oxo-1,2-bis[(2-pyridin-3-ylethyl)amino]ethyl}benzoate;
ethyl 3-{2-oxo-1-[(2-pyridin-3-ylethyl)amino]-2-[(2-pyridin-4-ylethyl)amino]ethyl}benzoate;
ethyl 3-{2-oxo-2-[(2-pyridin-3-ylethyl)amino]-1-[(2-pyridin-4-ylethyl)amino]ethyl}benzoate;
R-(-)-2-(4-methoxyphenyl)-2-[(2-piperidin-4-ylethyl)amino]-N-(2-pyridin-4-ylethyl)acetamide;
S-(+)-2-(4-methoxyphenyl)-2-[(2-piperidin-4-ylethyl)amino]-N-(2-pyridin-4-ylethyl)acetamide;
R-(-)-2-(3-chlorophenyl)-2-[(2-piperidin-4-ylethyl)amino]-N-(2-pyridin-4-ylethyl)acetamide;
S-(+)-2-(3-chlorophenyl)-2-[(2-piperidin-4-ylethyl)amino]-N-(2-pyridin-4-ylethyl)acetamide;
R-(-)-2-(2-fluorophenyl)-2-[(2-piperidin-4-ylethyl)amino]-N-(2-pyridin-4-ylethyl)acetamide;
S-(+)-2-(2-fluorophenyl)-2-[(2-piperidin-4-ylethyl)amino]-N-(2-pyridin-4-ylethyl)acetamide;
R-(-)-2-(1,3-benzodioxole-5-yl)-N-(pyridin-4-ylmethyl)-2-[(pyridin-4-ylmethyl)amino]acetamide;
S-(+)-2-(1,3-benzodioxole-5-yl)-N-(pyridin-4-ylmethyl)-2-[(pyridin-4-ylmethyl)amino]acetamide;
R-(-)-methyl 4-{2-oxo-1,2-bis[(2-pyridin-4-ylethyl)amino]ethyl}benzoate;
S-(+)-methyl4-{2-oxo-1,2-bis[(2-pyridin-4-ylethyl)amino]ethyl}benzoate;
R-(-)-methyl 3-{2-oxo-1,2-bis[(2-pyridin-4-ylethyl)amino]ethyl}benzoate;
S-(+)- methyl 3-{2-oxo-1,2-bis[(2-pyridin-4-ylethyl)amino]ethyl}benzoate;
R-(-)-2-(3-fluorophenyl)-N-(2-pyridin-4-ylethyl)-2-[(2-pyridin-3-ylethyl)amino]acetamide;
S-(+)-2-(3-fluorophenyl)-N-(2-pyridin-4-ylethyl)-2-[(2-pyridin-3-ylethyl)amino]acetamide;
R-(-)-2-(2-ethoxyphenyl)-N-(pyridin-4-ylmethyl)-2-[(pyridin-4-ylmethyl)amino]acetamide;
S-(+)-2-(2-ethoxyphenyl)-N-(pyridin-4-ylmethyl)-2-[(pyridin-4-ylmethyl)amino]acetamide;
R-(-)-2-(2- bromophenyl)-N-(2-pyridin-3-ylethyl)-2-[(2-pyridin-4-ylethyl)amino]acetamide;
S-(+)-2-(2- bromophenyl)-N-(2-pyridin-3-ylethyl)-2-[(2-pyridin-4-ylethyl)amino]acetamide;
R-(-)-2-(2,4-clichlorophenyl)-N-(2-pyridin-4-ylethyl)-2-[(2-pyriclin-4-ylethyl)amino]acetamide;
S-(+)-2-(2,4-dichlorophenyl)-N-(2-pyridin-4-ylethyl)-2-[(2-pyridin -4-ylethyl)amino]acetamide;
R-(-)-2-(2,4-dimethylphenyl)-N-(2-pyridin-4-ylethyl)-2-[(2-pyridin-4-ylethyl)amino]acetamide;
S-(+)-2-(2,4-dimethylphenyl)-N-(2-pyridin-4-ylethyl)-2-[(2-pyridin-4-ylethyl)amino]acetamide;
R-(-)-2-[(2-piperidin-4-ylethyl]-N-(2-pyridin-4-ylethyl)-2-[3-(trifluoromethyl)phenyl]acetamide;
S-(+)-2-[(2-piperidin-4-ylethyl)amino]-N-(2-pyridin-4-ylethyl)-2-[3-(trifluoromethyl)phenyl] acetamide;
R-(-)-N-(2-pyridin-4-ylethyl)-2-[(2-pyridin-4-ylethyl)amino]-2-(2-thienyl)acetamide;
S-(+)-N-(2-pyridin-4-ylethyl)-2-[(2-pyridin-4-ylethyl)amino]-2-(2-thienyl)acetamide;
R-(-)-2-phenyl-N-(pyridin-4-ylmethyl)-2-[(pyridin-4-ylmethyl)amino]acetamide;
S-(+)-2-phenyl-N-(pyridin-4-ylmethyl)-2-[(pyridin-4-ylmethyl)amino]acetamide;
R-(-)-N-(pyridin-4-ylmethyl)-2-[(pyridin-3-ylmethyl)amino]-2-(2,4,5-trimethylphenyl)acetamide;
S-(+)-N-(pyridin-4-ylmethyl)-2-[(pyridin-3-ylmethyl)amino]-2-(2,4,5-trimethylphenyl)acetamide;
or pharmaceutically acceptable salts thereof, unless it is specifically indicated.

The invention relates to the use of the compounds of the general formulae **I, 1.1, 1.1.1** and **I.1.2** as physiologically active agent that influence formation, storage and recall of memory and to the use of physiologically active agents for potentiation of early stages of memory formation to form more stable long-term memory, especially for potentiation of late stages of memory formation to enhance memory about previous experience.

The compounds of the formula **I** according to the present invention should be applied as a drug, which is also an object of the invention.

The compounds of the general formulae **I, 1.1, 1.1.1** and **I.1.2,** or pharmaceutically acceptable salts thereof or alkyl esters, in the form of the individual optical isomers, or mixtures thereof, with the ability to effectively influence on memory formation, storage and playback, may be used as an active component for the manufacture of pharmaceutical compositions and drugs.

The invention refers to the pharmaceutical composition that influences on formation, storage and recall of memory, containing the above mentioned component in effective amount.

The invention relates to a drug in the form of tablets, capsules and injections, placed in a pharmaceutically acceptable packing, for prophylaxis and treatment of impairment of formation, storage and recall of memory containing the above mentioned active component or the pharmaceutical composition thereof.

The pharmaceutical composition and the drug may be manufactured by the traditional methods for the pharmaceutical industry.

The pharmaceutical composition may be received by mixing the pharmaceutically acceptable carriers, solvents or diluents.

It is possible to add the following additeves to the composition: supplements, distributing and receptive agents, delivery agent; preservatives, stabilisers, fillers, choppers, wetting agents, processing agents, suspending agents, gelifires, sweeteners, flavoring agents, aromatizers, antibacterial agents, antifungals, lubricants, regulators of prolonged delivery. The selection and the ratio of the indicated components depend on the nature and mode of administration and dosage.

The term "effective amount" as used herein, means the use of the compound of the formula **I** in the amount that according to the indexes of activity and toxicity and on the base of specialict's knowladge should be effective in the present pharmaceutical composition and drug.

The content of the active component is equal to 1 - 20 % in combination with one or more pharmaceutically acceptable additives, such as diluents, adsorbents, fragrances, flavoring agents.

The drug may be in a liquid or solid form. The examles of the solid forms are tablets, pills, capsules, etc. The examples of the liquid form are for injection or parenteral administrations include solutions, emulsions, suspensions, etc.

The invention relates to the use of the compound of the general formulae **I, I.1, I.1.1** and **I.1.2** as molecular tools for pharmacological studies (in vitro and in vivo) of biochemical signaling, neurotrophic and neuroprotective processes related to the secretory protein sAPP and for studying the mechanisms of the formation, storage and regeneration of memory.

The compounds of the present invention may be obtaind by multi-component reaction of aldehyde, amine, izonitriles and carboxylic acid followed by removal of N-acyl group according with the scheme 1:

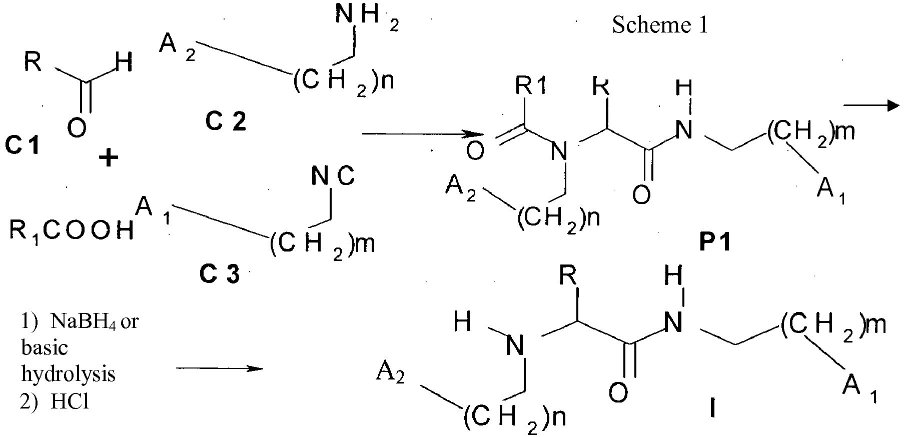

More detailed method is as follows.

According to the scheme I in multi-component reaction an equivalent quantity of aldehyde C1, amine C2, isonitrile and carboxylic acid are added, the process is carried out at a temperature of 10-25°C in an inert protic solvent (methanol, ethanol, trifluoroethanol etc.) and in an aprotic solvent (hexane, cyclohexane, ethylene carbonate, nitromethane etc.) with or without a catalyst. With that, the compounds of formula I are prepared by removing a carboxyl group from the preproduct P I using a reducing agent, such as alkali metal borohydride (NaBH₄, LiBH₄, KBH₄) or using a base, such as alkali metal hydroxide or alkali metal carbonate etc.

The following examples demonstrate the preparation of given compounds in accordance with the invention.

All the following compounds as well as the compounds listed above, but not mentioned in the examples, are derived in accordance with the scheme 1.

For synthesis the following initial reagents and the physicochemical methods for proving the structure of the synthesized compounds and their purity:

All the solvents and reagents were received from the suppliers such as Sigma-Aldrich (USA), Fluka (Germany), Acros (Belgium) and Lancaster (UK). Melting points (m.p.) were obtained on a Buchi B-520 apparatus. 1H and 13C NMR spectra were obtained on Varian (300 MHz) spectrometer in CDCl₃, D₂O 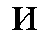 DMSO-d6, chemical shifts (∂) are expressed in ppm. Internal standard is tetramethylsilane.

Purity was monitored by LC-MS on Applied Biosystems apparatus (Shimadzu 10-AV LC, Gilson-215 automatic feed of samples, mass spectrometer API 150EX, detectors UV (215 and 254 nm) and ELS, column Luna-C18, Phenomenex, 5 cm x 2 mm). According to the LC/MS data all the synthesized compounds in the spectra MS had a parent peak M+1, the content of the main substance in all cases was more than 95%.

### Example1. 2-(1,3-benzodioxole-5-yl)-N-(2-pyridin-4ylethyl)-2-[(2-pyridin-4-ylethyl)-amino]acetamide (substance 271)

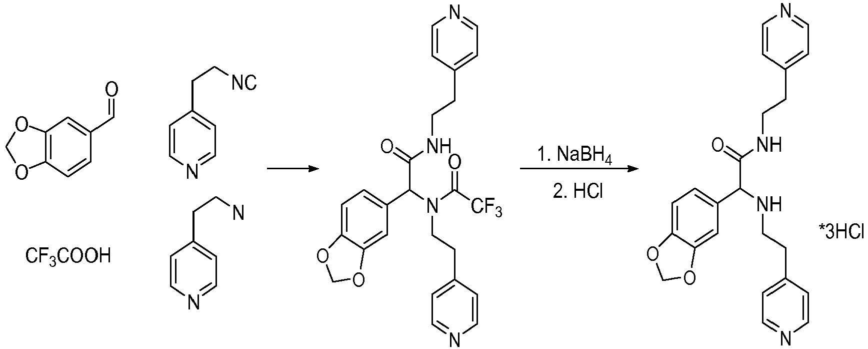

To a solvent of 1 mmol of aldehyde and 1 mmol of amine in 10 ml of methanol were added CF₃COOH (1 mmol) and 1 mmol of isonitrile. The mixture was stirred for 12 hours at room temperature, the solvent was evaporated under vacuum, the residue was purified by column chromatography CH₂Cl₂:MeOH 20:1. The obtained trifluoroacetamide was dissolved in methanol (10 ml), cooled to 0 ° C and 0,2g of NaBH₄ were added. The mixture was stirred for 12 hours at room temperature, the solvent was evaporated under vacuum and the residue was purified by column chromatography CH2Cl2: MeOH 20:1. The pure product was dissolved in methanol, saturated with HCl (gas) and evaporated under vacuum. 48% yield, 250 mg. Hereinafter, unless otherwise specified, the spectra of claimed compounds bases are given. 1H-NMR: 2,64-2,75 (m, 5H), 2,85-2,93 (m, 1H), 3,27-3,39 (m, 1H), 3,43-3, 54 (m, 1H), 3,99 (c, 1H), 5,87 (c, 2H), 6,52-6,55 (m, 4H), 6,97 (d, J = 5,8 Hz, 2H), 7,10 (d, J = 5,8 Hz, 2H), 8,43-8,50 (m, 4H).

### Example 2. Methyl 4-{2-oxo-1,2-bis[(2-pyridin-4-ylethyl)amino]ethyl}benzoate (substance 661)

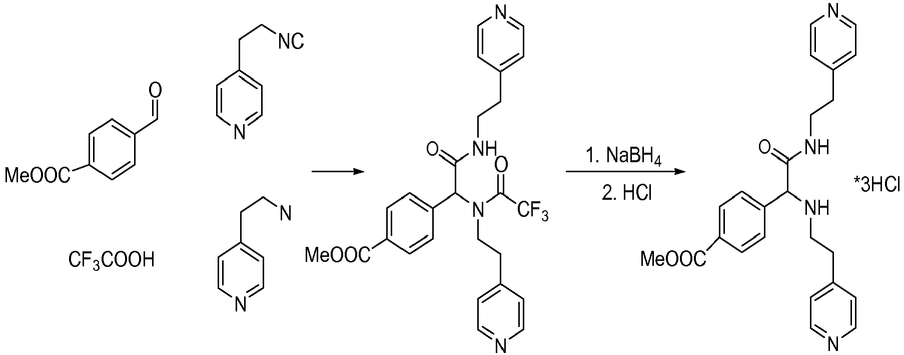

To a solvent of 1 mmol of aldehyde and 1 mmol of amine in 10 ml of methanol were added CF₃COOH (1 mmol) and 1 mmol of isonitrile. The mixture was stirred for 12 hours at room temperature, the solvent was evaporated under vacuum, the residue was purified by column chromatography CH₂Cl₂:MeOH 20:1. The obtained trifluoroacetamide was dissolved in methanol (10 ml), cooled to 0 ° C and 0,2g of NaBH₄ were added. The mixture was stirred for 12 hours at room temperature, the solvent was evaporated under vacuum and the residue was purified by column chromatography CH2Cl2: MeOH 20:1. The pure product was dissolved in methanol, saturated with HCl (gas) and evaporated under vacuum. 19% yield, 100 mg. 1H-NMR: 2,65-2,75 (m, 5H), 2,85-2,93 (m, 1H), 3,28-3,40 (m, 1H), 3,44-3,54 (m, 1H), 3,88 (c, 3H), 4,15 (c, 1H), 6,85 (t, J = 6,1 Hz, 1H), 6,95 (d, J = 5, 8 Hz, 2H), 7,06 (d, J = 5,8 Hz, 2H), 7,27 (d, J = 8,1 Hz, 2H), 7,95 (d, J = 8,1 Hz , 2H), 8,41 (d, J = 6,41 Hz, 2H), 8,46 (d, J = 8,46 Hz, 2H).

### Example 3. 2-(4-hydroxyphenyl)-N-(2-pyridin-4-ylethyl)-2-[(2-pyridin-4-ylethyl)amino]acetamide (substance 581)

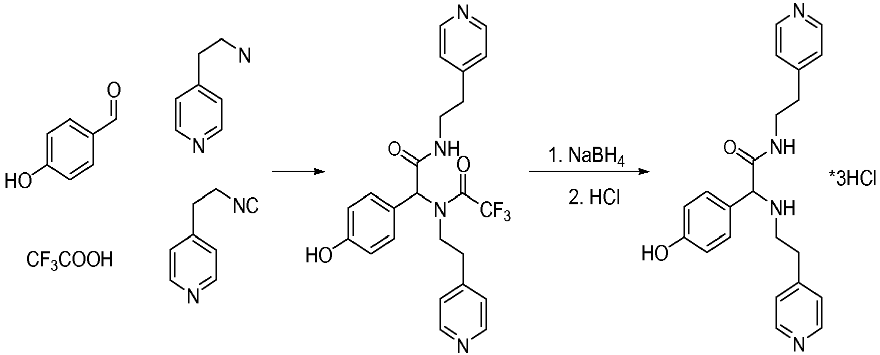

To a solvent of 1 mmol of aldehyde and 1 mmol of amine in 10 ml of methanol were added CF₃COOH (1 mmol) and 1 mmol of isonitrile. The mixture was stirred for 12 hours at room temperature, the solvent was evaporated under vacuum, the residue was purified by column chromatography CH₂Cl₂:MeOH 20:1. The obtained trifluoroacetamide was dissolved in methanol (10 ml), cooled to 0 ° C and 0,2g of NaBH₄ were added. The mixture was stirred for 12 hours at room temperature, the solvent was evaporated under vacuum and the residue was purified by column chromatography CH2Cl2: MeOH 20:1. The pure product was dissolved in methanol, saturated with HCl (gas) and evaporated under vacuum. 25% yield, 120 mg. 1H-NMR: 2,60-2,93 (m, 6H), 3,27-3,37 (m, 1H), 3,45-3,57 (m, 1H), 4,01 (c, 1H), 6.67 (d, J = 8,6 Hz, 2H), 6,92 (t, J = 6,1 Hz, 1H), 6,90-6,97 (m, 4H), 7,07 (d, J = 5,6 Hz, 2H), 8,37 (d, J 5,8 Hz, 2H), 9,45 (d, J = 5,8 Hz, 2H).

### Example 4. 2-(1,3-benzodioxole-5-yl)-N-(pyridin-4-ylmethyl)-2-[(pyridin-4-ylmethyl)-amino]acetamide (substance 272)

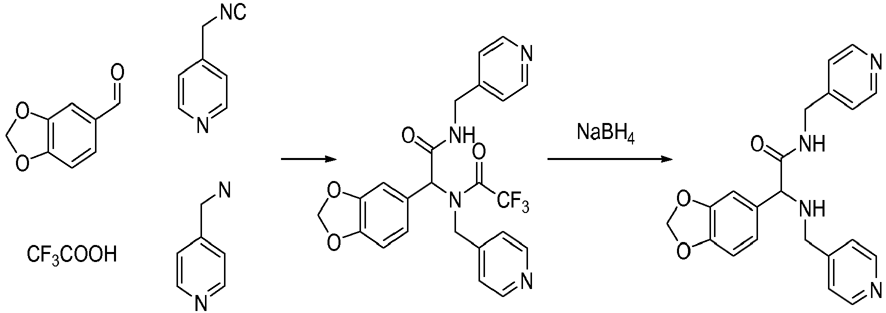

To a solvent of 1 mmol of aldehyde and 1 mmol of amine in 10 ml of methanol were added CF₃COOH (1 mmol) and 1 mmol of isonitrile. The mixture was stirred for 12 hours at room temperature, the solvent was evaporated under vacuum, the residue was purified by column chromatography CH₂Cl₂:MeOH 20:1. The obtained trifluoroacetamide was dissolved in methanol

(10 ml), cooled to 0 ° C and 0,2g of NaBH₄ were added. The mixture was stirred for 12 hours at room temperature, the solvent was evaporated under vacuum and the residue was purified by column chromatography CH₂Cl₂:MeOH 20:1. The pure product was dissolved in methanol, saturated with HCl (gas) and evaporated under vacuum 29% yield, 140 mg. 1H-NMR: 3.11 (d, J = 14,2 Hz, 2H), 3,16 (d, J = 6,8 Hz), 3.38 (d, J = 6,8 Hz, 2H), 3, 77 (d, J = 14,2 Hz, 2H), 5,98 (c, 2H), 6,69 (AB-system, J = 7,8 Hz, 2H), 6,98 (c, 1H), 7.13 (d, J = 4,8 Hz), 7.23 (d, J = 4,8 Hz), 8.49 (d, J = 4,1 Hz, 2H), 8,51 (d, J = 4,1 Hz, 2H).

### Example 5. 2-(1,3-benzodioxole-5-yl)-2-[(2-piperidin-4-ylethyl)amino]-N-(2-pyridin-4-ylethyl) acetamide (substance 273)

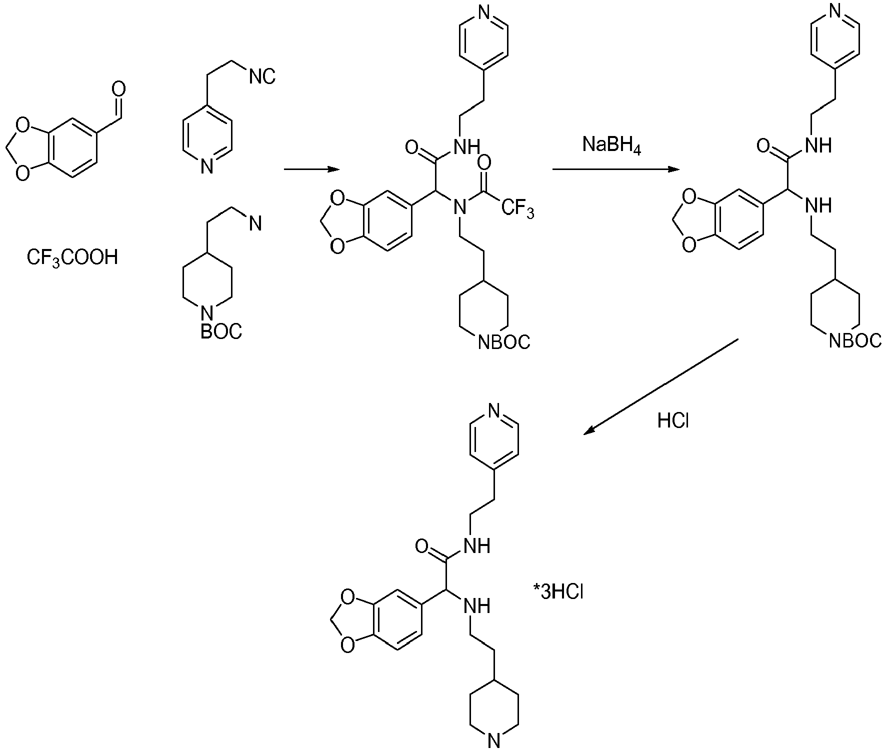

To a solvent of 1 mmol of aldehyde and 1 mmol of amine in 10 ml of methanol were added CF₃COOH (1 mmol) and 1 mmol of isonitrile. The mixture was stirred for 12 hours at room temperature, the solvent was evaporated under vacuum, the residue was purified by column chromatography CH₂Cl₂:MeOH 20:1. The obtained trifluoroacetamide was dissolved in methanol (10 ml), cooled to 0 ° C and 0,2g of NaBH₄ were added. The mixture was stirred for 12 hours at room temperature, the solvent was evaporated under vacuum and the residue was purified by column chromatography CH₂Cl₂:MeOH 20:1. The pure product was dissolved in methanol, saturated with HCl (gas) and evaporated under vacuum 29% yield, 150 mg. 1H-NMR: 1,25-1,40 (m, 2H), 1,55-1,70 (m, 3H), 1,78-1,86 (m, 2H), 2,75-2,95 (m, 4H), 2,95-3,13 (m, 2H), 3,25-3,33 (m, 2H), 3,44-3,52 (m, 1H), 3,69-3 , 80 (m, 1H), 5,98 (d, J = 21,5 Hz, 2H), 6,76-6,85 (m, 2H), 6,91-6,97 (m, 1H), 7.78 (d, J = 6,3 Hz, 2H), 8,58 (d, J = 6,3 Hz, 2H).

### Example 6. 2-(1,3-benzodioxole-5-yl)-2-[(2-piperidin-4-ylethyl)amino]-N-(pyridin-4-ylmethyl) acetamide (substance 274)

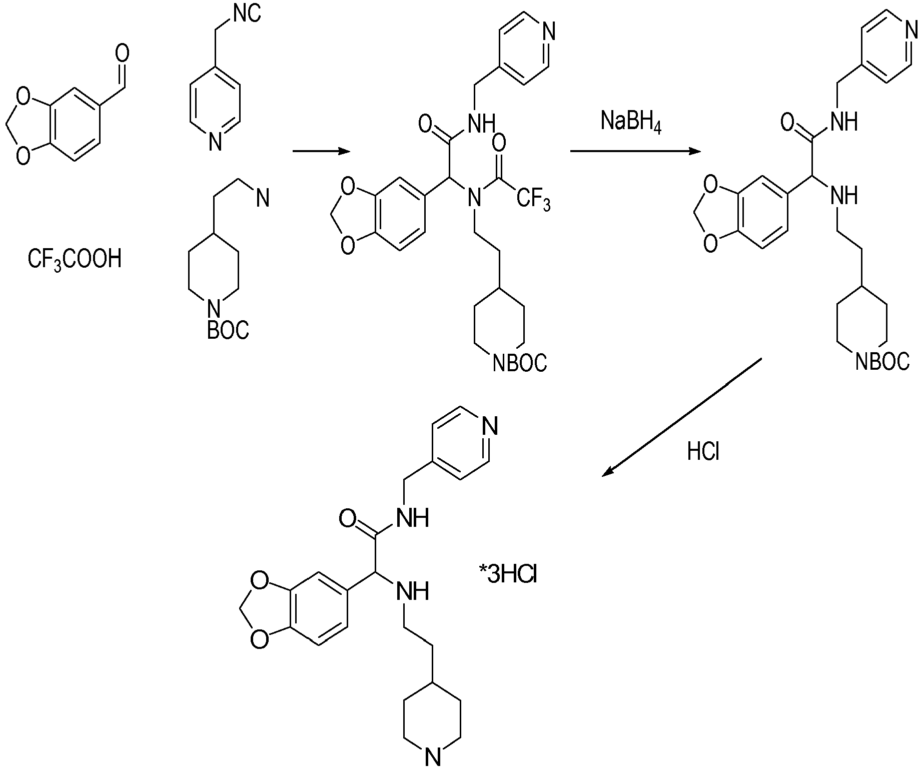

To a solvent of 1 mmol of aldehyde and 1 mmol of amine in 10 ml of methanol were added CF₃COOH (1 mmol) and 1 mmol of isonitrile. The mixture was stirred for 12 hours at room temperature, the solvent was evaporated under vacuum, the residue was purified by column chromatography CH₂Cl₂:MeOH 20:1. The obtained trifluoroacetamide was dissolved in methanol (10 ml), cooled to 0 °C and 0,2g of NaBH₄ were added. The mixture was stirred for 12 hours at room temperature, the solvent was evaporated under vacuum and the residue was purified by column chromatography CH₂Cl₂:MeOH 20:1. The pure product was dissolved in methanol, saturated with HCl (gas) and evaporated under vacuum. 20% yield, 100 mg. 1H-NMR: 1,07-1,24 (m, 2H), 1,50-1,70 (m, 5H), 2,57-2,76 (m, 3H), 2,80-2,90 (m, 1H), 3,00-3,17 (m, 3H), 5,14 (d, J = 8,6 Hz, 1H), 5,52 (d, J = 8,6 Hz, 1H), 5,68 (c, 2H), 6,50 (d, J 7,6 Hz, 1H), 6,75-6,79 (m, 1H), 6,93 (c, 1H), 8,04 (d, J = 5,6 Hz, 2H), 8,63 (d, J = 5,6 Hz, 2H).

### Example 7. 2-(4-hydroxyphenyl)-2-[(2-piperidin-4-ylethyl)amino]-N-(2-pyridin-4-ylethyl) acetamide (substance 583)

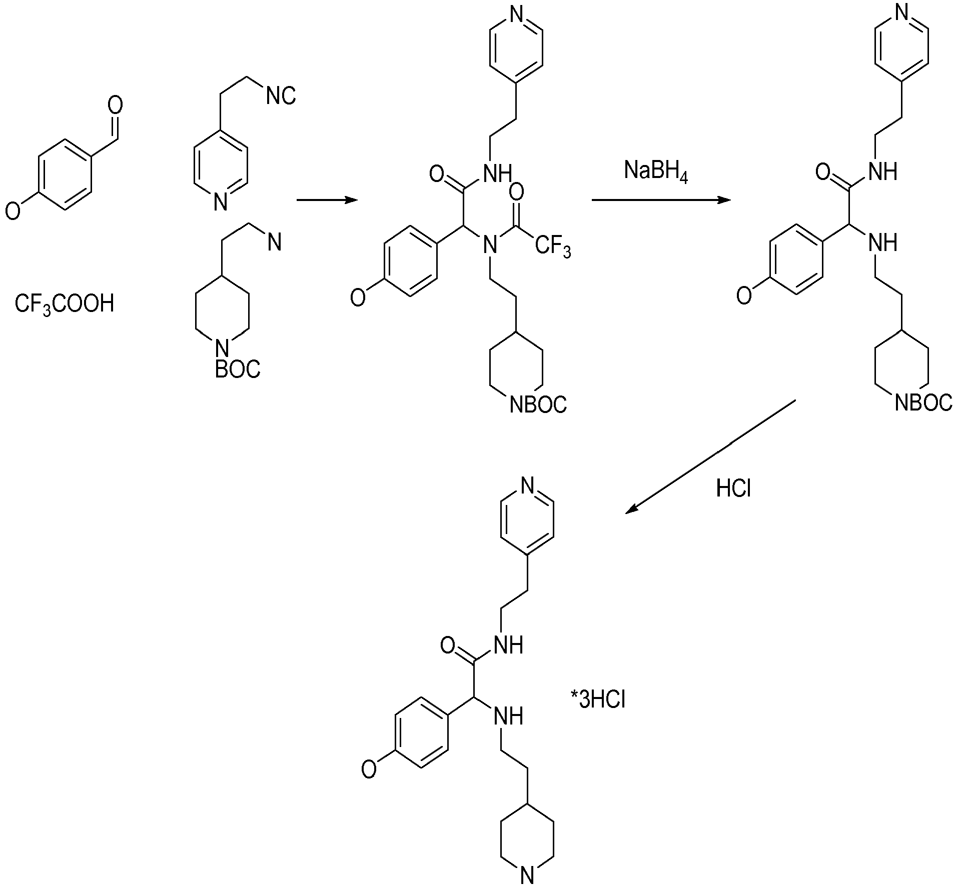

To a solvent of 1 mmol of aldehyde and 1 mmol of amine in 10 ml of methanol were added CF₃COOH (1 mmol) and 1 mmol of isonitrile. The mixture was stirred for 12 hours at room temperature, the solvent was evaporated under vacuum, the residue was purified by column chromatography CH₂Cl₂:MeOH 20:1. The obtained trifluoroacetamide was dissolved in methanol (10 ml), cooled to 0 ° C and 0,2g of NaBH₄ were added. The mixture was stirred for 12 hours at room temperature, the solvent was evaporated under vacuum and the residue was purified by column chromatography CH₂Cl₂:MeOH 20:1. The pure product was dissolved in methanol, saturated with HCl (gas) and evaporated under vacuum. 21% yield, 100 mg. 1H-NMR: 1,29-1,60 (m, 6H), 1,75-2,00 (m, 2H), 2,85-3,10 (m, 4H), 3,33-3,40 (m, 2H), 3,45-3,60 (m, 1H), 3,90-4,10 (m, 2H), 5,47 (c, 2H), 6,84 (d, J = 8,1 Hz, 2H), 7,01 (d, J = 8,1 Hz, 2H), 8,04 (d, J = 6,1 Hz, 2H), 8,74 (d, J = 6,1 Hz, 2H).

### Example 8. Methyl 4-{2-oxo-1-[(2-pyridin-3-ylethyl)amino]-2-[(2-pyridin-4-ylethyl)amino]ethyl} benzoate (substance 669)

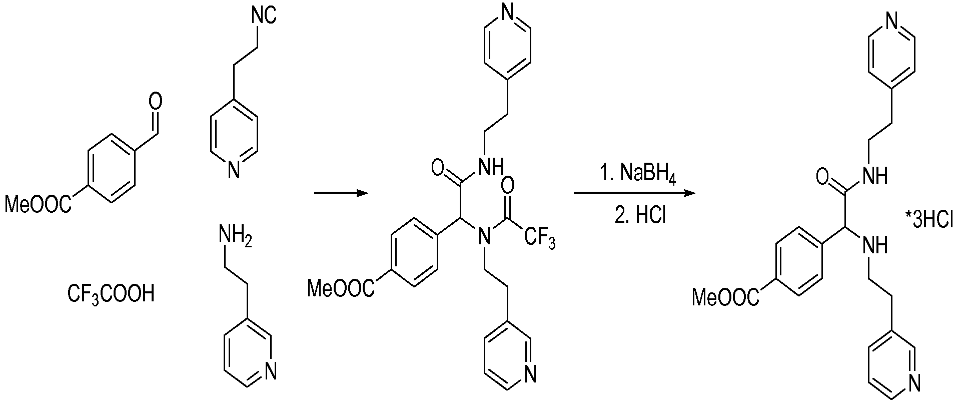

To a solvent of 1 mmol of aldehyde and 1 mmol of amine in 10 ml of methanol were added CF₃COOH (1 mmol) and 1 mmol of isonitrile. The mixture was stirred for 12 hours at room temperature, the solvent was evaporated under vacuum, the residue was purified by column chromatography CH₂Cl₂:MeOH 20:1. The obtained trifluoroacetamide was dissolved in methanol (10 ml), cooled to 0 ° C and 0,2g of NaBH₄ were added. The mixture was stirred for 12 hours at room temperature, the solvent was evaporated under vacuum and the residue was purified by column chromatography CH₂Cl₂:MeOH 20:1. The pure product was dissolved in methanol, saturated with HCl (gas) and evaporated under vacuum. 38% yield, 200 mg. 1H-NMR: 2,65-2,75 (m, 5H), 2,81-2.90 (m, 1H), 3,31-3.40 (m, 1H), 3,44-3,55 (m, 1H), 3, 87 (c, 3H), 4,15 (c, 1H), 6,85-7,05 (m, 3H), 7,2 (ush.c, 1H), 7,26 (d, J = 8, 1 Hz, 2H), 7,43 (d, J = 7,6 Hz, 1H), 7,93 (d, J = 8,1 Hz, 2H), 8,4 (ush.c, 4H).

### Example 9. Methyl 4- {2-oxo-1,2-bis[(2-pyridin-3-ylethyl)amino]ethyl} benzoate (substance 668)

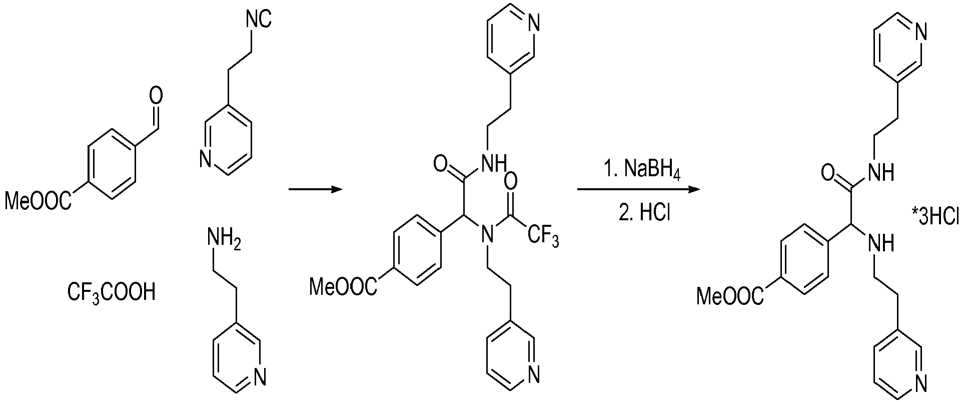

To a solvent of 1 mmol of aldehyde and 1 mmol of amine in 10 ml of methanol were added CF₃COOH (1 mmol) and 1 mmol of isonitrile. The mixture was stirred for 12 hours at room temperature, the solvent was evaporated under vacuum, the residue was purified by column chromatography CH₂Cl₂:MeOH 20:1. The obtained trifluoroacetamide was dissolved in methanol (10 ml), cooled to 0 ° C and 0,2g of NaBH₄ were added. The mixture was stirred for 12 hours at room temperature, the solvent was evaporated under vacuum and the residue was purified by column chromatography CH₂Cl₂:MeOH 20:1. The pure product was dissolved in methanol, saturated with HCl (gas) and evaporated under vacuum. 38% yield, 200 mg. 1H-NMR: 2,65-2,75 (m, 5H), 2,80-2,90 (m, 1H), 3,30-3,40 (m, 1H), 3,44-3,50 (m, 1H), 3,87 (c, 3H), 4,15 (c, 1H), 6,03 (t, J = 5,8 Hz), 7.13 (dd, J = 4,8, 2,8 Hz, 1H), 7,18 (dd, J = 4,8, 2,8 Hz, 1H), 7,27 (d, J = 8,3 Hz, 2H), 7,35 (d, J = 7, 8 Hz, 1H), 7,44 (d, J = 7,8 Hz, 1H), 7,93 (d, J = 8,3 Hz, 2H), 8,34 (c, 1H), 8,39 -8.47 (m, 3H).

### Example 10. Methyl 4-{2-oxo-2-[(2-pyridin-3-ylethyl)amino]-1-[(2-pyridin-4-ylethyl)amino]ethyl}benzoate (substance 670)

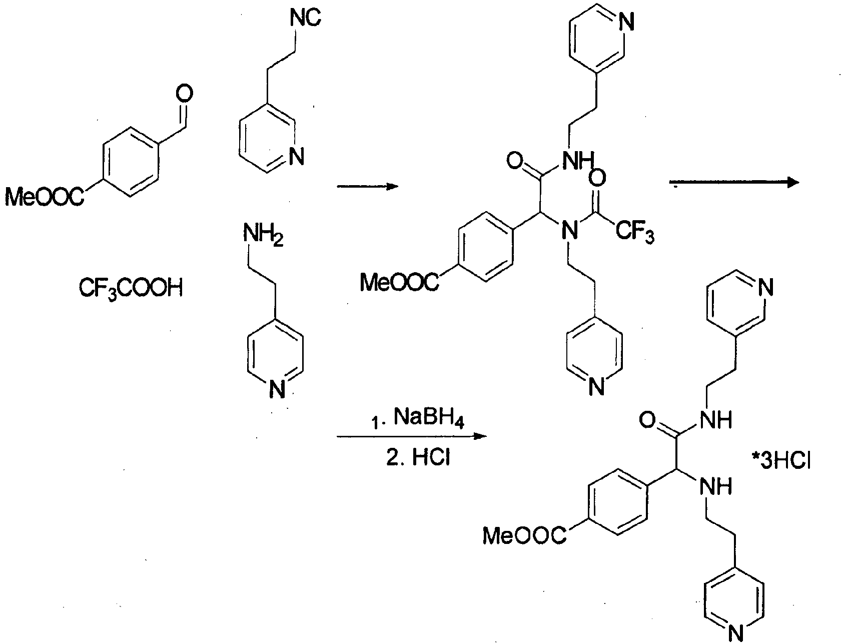

To a solvent of 1 mmol of aldehyde and 1 mmol of amine in 10 ml of methanol were added CF₃COOH (1 mmol) and 1 mmol of isonitrile. The mixture was stirred for 12 hours at room temperature, the solvent was evaporated under vacuum, the residue was purified by column chromatography CH₂Cl₂:MeOH 20:1. The obtained trifluoroacetamide was dissolved in methanol (10 ml), cooled to 0 ° C and 0,2g of NaBH₄ were added. The mixture was stirred for 12 hours at room temperature, the solvent was evaporated under vacuum and the residue was purified by column chromatography CH₂Cl₂:MeOH 20:1. The pure product was dissolved in methanol, saturated with HCl (gas) and evaporated under vacuum. 38% yield, 200 mg. 1H-NMR: 2,65-2,75 (m, 5H), 2,80-2,90 (m, 1H), 3,30-3,40 (m, 1H), 3,44-3,50 (m, 1H), 3,88 (c, 3H), 4,15 (c, 1H), 6,03 (t, J = 5,8 Hz), 7.06 (d, J = 5,6 Hz, 2H), 7,10-7,20 (m, 1H), 7,27 (d, J = 8,3 Hz, 2H), 7,35 (d, J = 7,8 Hz, 1H), 7, 94 (d, J = 8,3 Hz, 2H), 8,34 (c, 1H), 8,42 (d, J = 3,8 Hz, 1H), 8,47 (d, J = 5, 6 Hz, 2H).

### Example 11. Methyl 3-{2-oxo-1,2-bis[(2-pyridin-4-ylethyl)amino]ethyl} benzoate (substance 651)

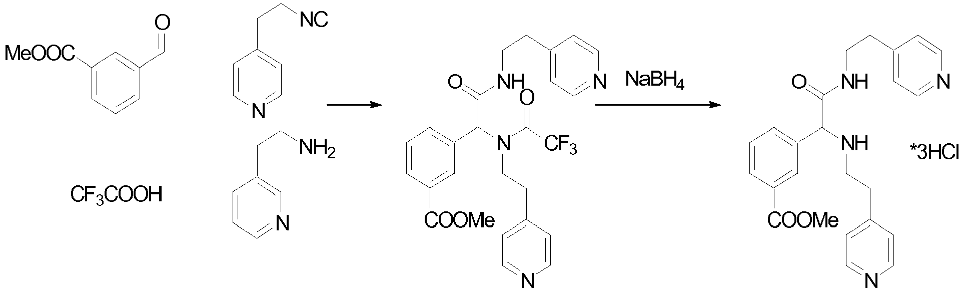

To a solvent of 1 mmol of aldehyde and 1 mmol of amine in 10 ml of methanol were added CF₃COOH (1 mmol) and 1 mmol of isonitrile. The mixture was stirred for 12 hours at room temperature, the solvent was evaporated under vacuum, the residue was purified by column chromatography CH₂Cl₂:MeOH 20:1. The obtained trifluoroacetamide was dissolved in methanol (10 ml), cooled to 0 ° C and 0,2g of NaBH₄ were added. The mixture was stirred for 12 hours at room temperature, the solvent was evaporated under vacuum and the residue was purified by column chromatography CH₂Cl₂:MeOH 20:1. The pure product was dissolved in methanol, saturated with HCl (gas) and evaporated under vacuum. 30% yield, 160 mg. 1H-NMR: 2,60-2,77 (m, 5H), 2,81-2,93 (m, 1H), 3,32-3,55 (m, 2H), 3,88 (c, 3H), 4,15 (c, 1H), 7,00 (c, 1H), 7,10-7,20 (m, 2H), 7,30-7,55 (m, 4H), 7,86 - 8.00 (m, 2H), 8,30-8,57 (m, 4H).

### Example 12. Methyl 3-{2-oxo-1-[(2-pyridin-3-ylethyl)amino]-2-[(2-pyridin-4-ylethyl)-amino]ethyl} benzoate (substance 659)

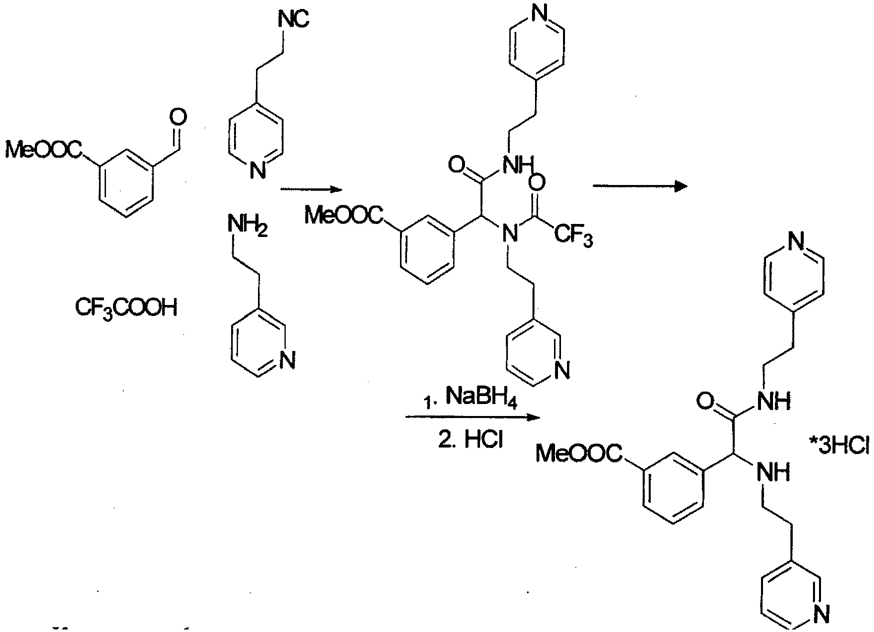

To a solvent of 1 mmol of aldehyde and 1 mmol of amine in 10 ml of methanol were added CF₃COOH (1 mmol) and 1 mmol of isonitrile. The mixture was stirred for 12 hours at room temperature, the solvent was evaporated under vacuum, the residue was purified by column chromatography CH₂Cl₂:MeOH 20:1. The obtained trifluoroacetamide was dissolved in methanol (10 ml), cooled to 0 ° C and 0,2g of NaBH₄ were added. The mixture was stirred for 12 hours at room temperature, the solvent was evaporated under vacuum and the residue was purified by column chromatography CH₂Cl₂:MeOH 20:1. The pure product was dissolved in methanol, saturated with HCl (gas) and evaporated under vacuum. 30% yield, 160 mg. 1H-NMR: 2,64-2,77 (m, 5H), 2,81-2,93 (m, 1H), 3,32-3,40 (m, 1H), 3,44-3,53 (m, 1H), 3,88 (c, 3H), 4,15 (c, 1H), 6,91-7,00 (m, 3H), 7,17-7,20 (m, 1H), 7,32-7,44 (m, 3H), 7,90-7,95 (m, 2H), 8,35-8,50 (m, 4H).

### Example 13. Methyl 3-{2-oxo-1,2-bis[(2-pyridin-3-ylethyl)amino]ethyl} benzoate (substance 658)

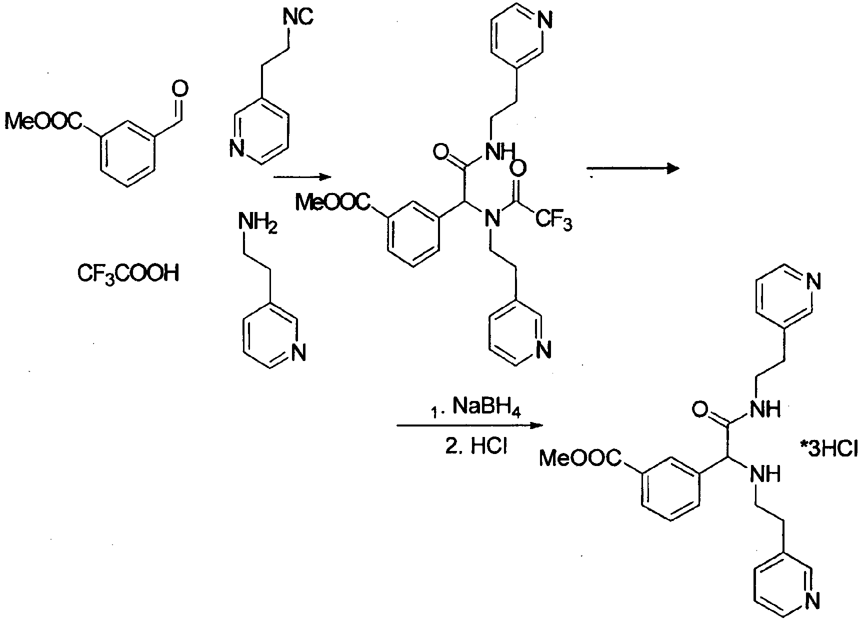

To a solvent of 1 mmol of aldehyde and 1 mmol of amine in 10 ml of methanol were added CF₃COOH (1 mmol) and 1 mmol of isonitrile. The mixture was stirred for 12 hours at room temperature, the solvent was evaporated under vacuum, the residue was purified by column chromatography CH₂Cl₂:MeOH 20:1. The obtained trifluoroacetamide was dissolved in methanol (10 ml), cooled to 0 ° C and 0,2g of NaBH₄ were added. The mixture was stirred for 12 hours at room temperature, the solvent was evaporated under vacuum and the residue was purified by column chromatography CH₂Cl₂:MeOH 20:1. The pure product was dissolved in methanol, saturated with HCl (gas) and evaporated under vacuum 23% yield, 120 mg. 1H-NMR: 2,60-2,77 (m, 5H), 2,81-2,93 (m, 1H), 3,32-3,55 (m, 2H), 3,88 (c, 3H), 4,15 (c, 1H), 7,00 (c, 1H), 7,10-7,20 (m, 2H), 7,30-7,55 (m, 4H), 7,86 - 8.00 (m, 2H), 8,30-8,57 (m, 4H).

### Example 14. 2-(1,3-benzoclioxole-5-yl)-N-(pyridin-3-ylmethyl)-2-[(pyridin-4-ylmethyl)-amino]acetamide (substance 277)

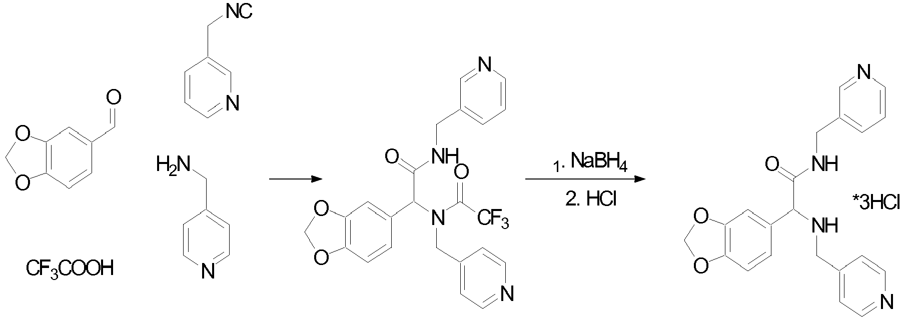

To a solvent of 1 mmol of aldehyde and 1 mmol of amine in 10 ml of methanol were added CF₃COOH (1 mmol) and 1 mmol of isonitrile. The mixture was stirred for 12 hours at room temperature, the solvent was evaporated under vacuum, the residue was purified by column chromatography CH₂Cl₂:MeOH 20:1. The obtained trifluoroacetamide was dissolved in methanol (10 ml), cooled to 0 ° C and 0,2g of NaBH₄ were added. The mixture was stirred for 12 hours at room temperature, the solvent was evaporated under vacuum and the residue was purified by column chromatography CH₂Cl₂:MeOH 20:1. The pure product was dissolved in methanol, saturated with HCl (gas) and evaporated under vacuum. 23% yield, 110 mg. 1H-NMR: 3,66-3,75 (m, 2H), 4,13 (c, 1H), 4,35-4,45 (m, 2H), 5,92 (c, 2H), 6, 72-6,78 (m, 2H), 6,92 (c, 1H), 7,15 (d, J = 5,3 Hz, 2H), 7,15-7,20 (m, 1H), 7 ,25-7, 30 (m, 1H), 7,48 (d, J = 8,1 Hz, 1H), 8,41 (c, 1H), 8,45 (d, J = 5,3 Hz).

### Example 15. 2-(1,3-benzoclioxole-5-yl)-N-(pyridin-3-ylmethyl)-2-[(pyridin-3-ylmethyl)-amino]acetamide (substance 275)

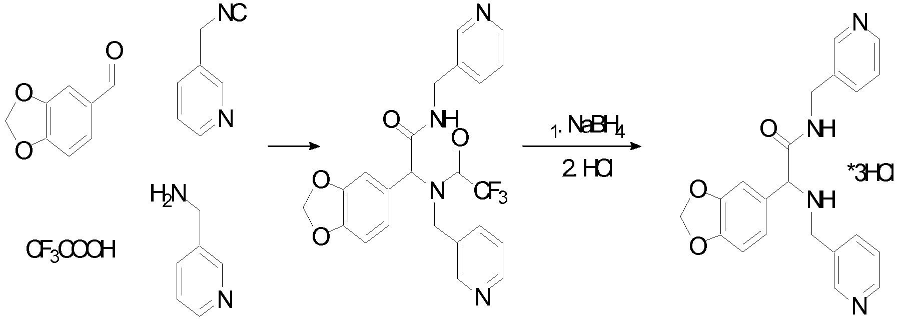

To a solvent of 1 mmol of aldehyde and 1 mmol of amine in 10 ml of methanol were added CF₃COOH (1 mmol) and 1 mmol of isonitrile. The mixture was stirred for 12 hours at room temperature, the solvent was evaporated under vacuum, the residue was purified by column chromatography CH₂Cl₂:MeOH 20:1. The obtained trifluoroacetamide was dissolved in methanol (10 ml), cooled to 0 ° C and 0,2g of NaBH₄ were added. The mixture was stirred for 12 hours at room temperature, the solvent was evaporated under vacuum and the residue was purified by column chromatography CH₂Cl₂:MeOH 20:1. The pure product was dissolved in methanol, saturated with HCl (gas) and evaporated under vacuum. 23% yield, 110 mg. 1H-NMR: 3,66 (AB-system, J = 8,6 Hz, 2H), 4,16 (c, 1H), 4,41 (d, J = 6,1 Hz, 2H), 5,93 (c, 2H), 6,73-6,79 (m, 2H), 6,83 (c, 1H), 7,18-7,23) m, 2H), 7,51 (d, J = 7, 8 Hz, 1H), 7,56 (d, J = 7,8 Hz, 1H), 8,41-8,49 (m, 4H).

### Example 16. 2-(2,3-dihydro-1,4-benzodioxin-6-yl)-N-(pyridin-4-ylmethyl)-2-[(pyridin-4-ylmethyl)amino]acetamide (substance 612)

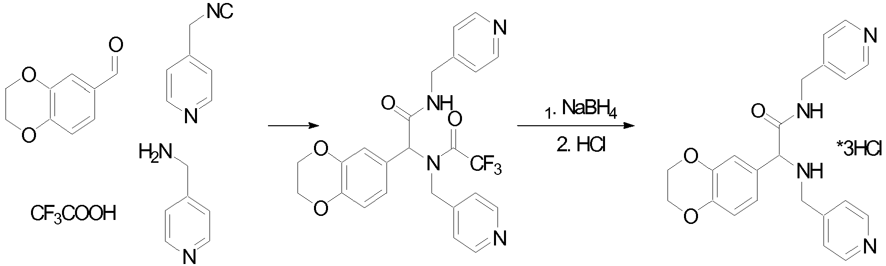

To a solvent of 1 mmol of aldehyde and 1 mmol of amine in 10 ml of methanol were added CF₃COOH (1 mmol) and 1 mmol of isonitrile. The mixture was stirred for 12 hours at room temperature, the solvent was evaporated under vacuum, the residue was purified by column chromatography CH₂Cl₂:MeOH 20:1. The obtained trifluoroacetamide was dissolved in methanol (10 ml), cooled to 0 ° C and 0,2g of NaBH₄ were added. The mixture was stirred for 12 hours at room temperature, the solvent was evaporated under vacuum and the residue was purified by column chromatography CH₂Cl₂:MeOH 20:1. The pure product was dissolved in methanol, saturated with HCl (gas) and evaporated under vacuum. 20% yield, 100 mg. 1H-NMR: 3,10-3,16 (m, 1H), 3,37 (d, J = 7,1 Hz), 3.73 (d, J = 14,2 Hz), 4.17 (d , J = 7,1 Hz), 4,20-4,30 (m, 5H), 6,70-6,85 (m, 2H), 6,93 (d, J = 1,8 Hz, 1H), 7.14 (d, J = 5,8 Hz, 2H), 7,23 (d, J = 5,8 Hz, 2H), 8,47 (d, J = 5,8 Hz, 2H), 8, 50 (d, J = 5,8 Hz, 2H).

### Example 17. 2-(2,3-dihydro-1,4-benzodioxin-6-yl)-N-(pyridin-3-ylmethyl)-2-[(pyridin-4-ylmethyl)amino]acetamide (substance 617)

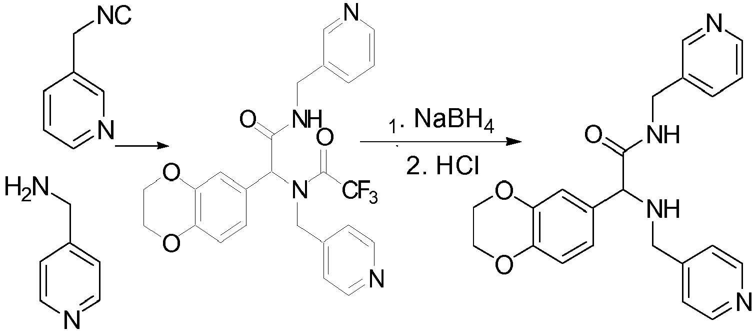

To a solvent of 1 mmol of aldehyde and 1 mmol of amine in 10 ml of methanol were added CF₃COOH (1 mmol) and 1 mmol of isonitrile. The mixture was stirred for 12 hours at room temperature, the solvent was evaporated under vacuum, the residue was purified by column chromatography CH₂Cl₂:MeOH 20:1. The obtained trifluoroacetamide was dissolved in methanol (10 ml), cooled to 0 °C and 0,2g of NaBH₄ were added. The mixture was stirred for 12 hours at room temperature, the solvent was evaporated under vacuum and the residue was purified by column chromatography CH₂Cl₂:MeOH 20:1. The pure product was dissolved in methanol, saturated with HCl (gas) and evaporated under vacuum. 32% yield, 160 mg. 1H-NMR: 3,65-3,74 (m, 2H), 4,10 (c, 1H), 4,19 (c, 4H), 4,33-4,43 (m, 2H), 6, 78 (c, 2H), 6,84 (c, 1H), 7,10-7,20 (m, 3H), 7,33 (t, J = 5,6 Hz, 1H), 7,47 (d, J = 7,8 Hz), 8,39 (c, 1H), 8,43 (d, J = 5,1 Hz, 3H).

### Example 18. 2-(2,3-dihydro-1,4-benzodioxin-6-yl)-N-(pyridin-3-ylmethyl)-2-[(pyridin-3-ylmethyl)amino]acetamide (substance 615)

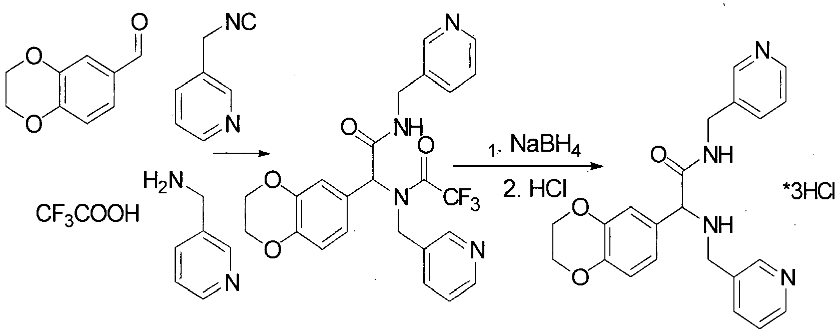

To a solvent of 1 mmol of aldehyde and 1 mmol of amine in 10 ml of methanol were added CF₃COOH (1 mmol) and 1 mmol of isonitrile. The mixture was stirred for 12 hours at room temperature, the solvent was evaporated under vacuum, the residue was purified by column chromatography CH₂Cl₂:MeOH 20:1. The obtained trifluoroacetamide was dissolved in methanol (10 ml), cooled to 0 °C and 0,2g of NaBH₄ were added. The mixture was stirred for 12 hours at room temperature, the solvent was evaporated under vacuum and the residue was purified by column chromatography CH₂Cl₂:MeOH 20:1. The pure product was dissolved in methanol, saturated with HCl (gas) and evaporated under vacuum. 25% yield, 100 mg. 1H-NMR: 3,65-3,74 (AB-system, J = 8,4 Hz, 2H), 4,14 (c, 1H), 4,21 (c, 4H), 4,42 (d, J = 6,1 Hz, 2H), 6,80 (c, 2H), 6,86 (c, 1H), 7,17-7,25 (m, 2H), 7,51 (d, J = 7 , 8 Hz, 1H), 7,56 (d, J = 7,8 Hz, 1H), 8,46 (ush. c, 4H).

### Example 19. Methyl 4-{2-oxo-1,2-bis[(pyridin-4-ylmethyl)amino]ethyl}benzoate (substance 662)

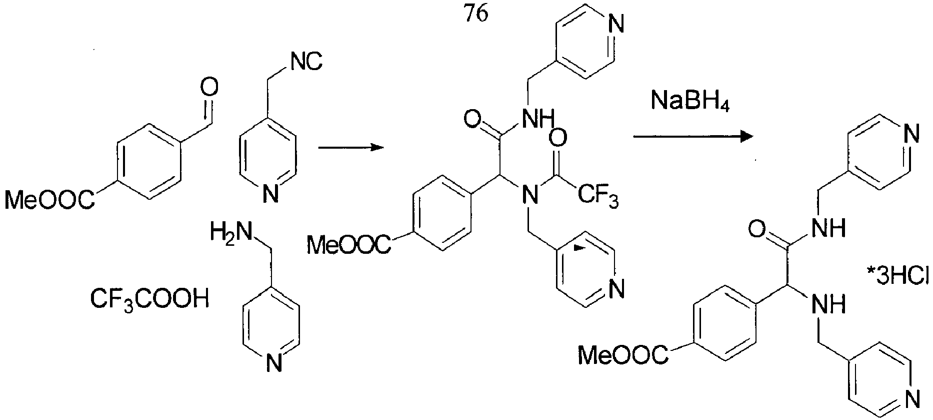

To a solvent of 1 mmol of aldehyde and 1 mmol of amine in 10 ml of methanol were added CF₃COOH (1 mmol) and 1 mmol of isonitrile. The mixture was stirred for 12 hours at room temperature, the solvent was evaporated under vacuum, the residue was purified by column chromatography CH₂Cl₂:MeOH 20:1. The obtained trifluoroacetamide was dissolved in methanol (10 ml), cooled to 0 ° C and 0,2g of NaBH₄ were added. The mixture was stirred for 12 hours at room temperature, the solvent was evaporated under vacuum and the residue was purified by column chromatography CH₂Cl₂:MeOH 20:1. The pure product was dissolved in methanol, saturated with HCl (gas) and evaporated under vacuum. 25% yield, 100 mg. 1H-NMR: 3,70-3,78 (m, 2H), 3,86 (c, 3H), 4,31 (c, 1H), 4,33-4,43 (m, 2H), 7, 08 (d, J = 5,6 Hz, 2H), 7,17 (d, J = 5,6 Hz), 7.42 (d, J = 8,3 Hz, 2H), 7,64 (t, J = 6,1 Hz, 1H), 7,07 (d, J = 8,3 Hz, 2H), 8,39 (d, J = 5,8 Hz, 2H), 8,43 (J = 5, 8 Hz, 2H).

### Example 20. N-(pyridin-4-ylmethyl)-2-[(pyridin-4-ylmethyl)amino]-2-(3,4,5-trimethoxyphenyl) acetamide (substance 622)

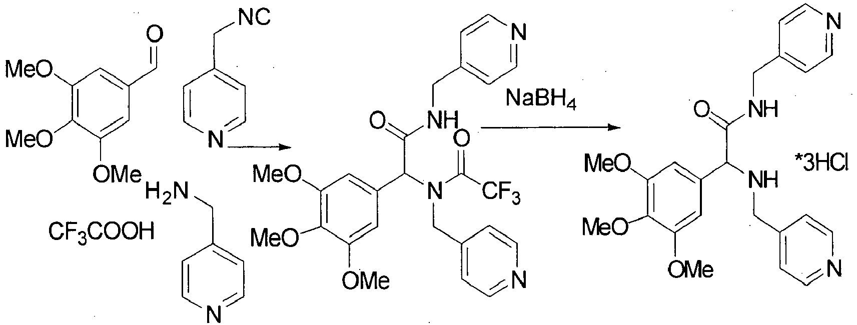

To a solvent of 1 mmol of aldehyde and 1 mmol of amine in 10 ml of methanol were added CF₃COOH (1 mmol) and 1 mmol of isonitrile. The mixture was stirred for 12 hours at room temperature, the solvent was evaporated under vacuum, the residue was purified by column chromatography CH₂Cl₂:MeOH 20:1. The obtained trifluoroacetamide was dissolved in methanol (10 ml), cooled to 0 ° C and 0,2g of NaBH₄ were added. The mixture was stirred for 12 hours at room temperature, the solvent was evaporated under vacuum and the residue was purified by column chromatography CH₂Cl₂:MeOH 20:1. The pure product was dissolved in methanol, saturated with HCl (gas) and evaporated under vacuum. 23% yield, 120 mg. 1H-NMR: 3,10-3,20 (m, 2H), 3,43 (d, J = 7,1 Hz), 3,80 (c, 6H), 3,84 (c, 3H), 4 , 21 (d, 6.8 Hz, 1H), 4,26 (d, 6.8 Hz, 1H), 6,54 (c, 1H), 7,14 (d, J = 5,8 Hz, 1H), 7,23 (c, 1H), 8,49 (d, J = 5,8 Hz, 2H), 8,52 (d, J = 5,8 Hz).

### Example 21. 2-(1,3-benzoclioxole-5-yl)-N-(pyridin-4-ylmethyl)-2-[(pyridin-3-ylmethyl)-amino]acetamide (substance 276)

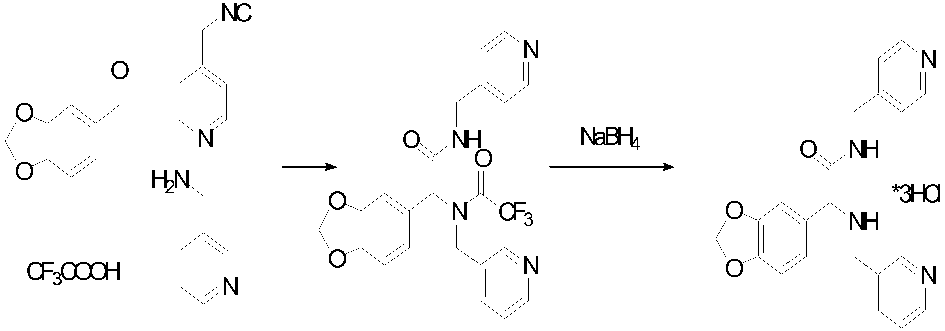

To a solvent of 1 mmol of aldehyde and 1 mmol of amine in 10 ml of methanol were added CF₃COOH (1 mmol) and 1 mmol of isonitrile. The mixture was stirred for 12 hours at room temperature, the solvent was evaporated under vacuum, the residue was purified by column chromatography CH₂Cl₂:MeOH 20:1. The obtained trifluoroacetamide was dissolved in methanol (10 ml), cooled to 0 ° C and 0,2g of NaBH₄ were added. The mixture was stirred for 12 hours at room temperature, the solvent was evaporated under vacuum and the residue was purified by column chromatography CH₂Cl₂:MeOH 20:1. The pure product was dissolved in methanol, saturated with HCl (gas) and evaporated under vacuum. 27% yield, 130 mg. 1H-NMR: 3,11-3,20 (m, 2H), 3,40 (d, J = 7,1 Hz, 1H), 3,76 (d, J = 13,7 Hz, 1H), 4 , 13 (d, J = 7,1 Hz, 1H), 4,24 (d, J = 7,1 Hz, 1H), 5,96 (c, 2H), 6,66 (d, J = 8, 1 Hz, 1H), 6,73 (d, J = 8,1 Hz), 6,97 (c, 1H), 7,11 (d, J = 5,8 Hz, 2H), 7,18-7 , 23 (m, 1H), 7,58 (d, J = 7,8 Hz, 1H), 8,47 (d, J = 5,8 Hz, 3H), 8,50 (c, 1H).

### Example 22. Methyl -4-[2-oxo-1,2-bis[[2-(4-pyridinyl)ethyl]amino]ethyl]-benzoate

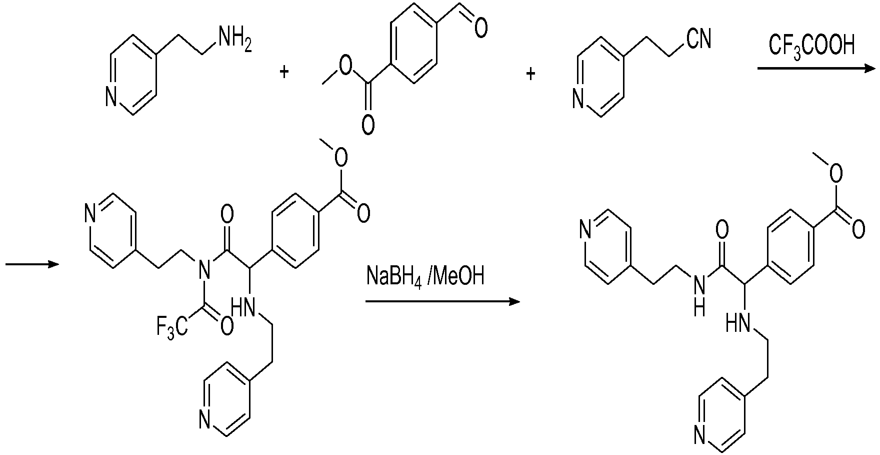

To a stirred and cooled in an ice bath solution of 10 g (0,08 mmol) of 4-(2-aminoethyl)pyridin in 200 ml of dried dry methylene chloride, which contains 25 g of hardened molecular sieves 4A, 13,4 g (0,08 mmol, 1 eq) of 4-carboxymethybenzaldehydel were added. The mixter was stirred for 1 hour; the solvent was decanted from the sieves and evaporated under vacuum The residue was dissolved in 150 ml of methanol, 6 ml of trifluoroacetic acid were added and at an ice bath 10,9 g of pyridinthylisonitril (0,08 mmol 1 eq) in 20 ml of methanol were added. The obtained solvent was stirred for 24 hours, evaporated, and the residue was purified by column chromatography using a mixture of dichloromethane/methanol 20/1 as an eluent. The obtained trifluoroacetamide was dissolved in methanol (150 ml) and sodium borohydride (15g) was added. After stirring for 24 hours, the reaction feed was concentrated on a rotary evaporator, treated with a small amount of dichloromethane, filtered and purified by column chromatography using a mixture of dichloromethane/methanol 20/1 as an eluent. 1,4 g of the amide as a yellow crystalline solid were received.

The examples given above demonstrate, but not limit, the invention. All the compounds were obtained and can be obtained in accordance with the given method of synthesis.

After the screening of several thousand molecules, about ten thousand of preferred structures for subsequent tests on models of memory consolidation in animals were selected.

Two compounds ACB-0002 (Methyl 4-{2-oxo-1,2-bis [(2-pyridin-4-ylethyl)amino]ethyl} benzoate) and ACB-0005 (2-(1,3- benzodioxole-5-yl)-N-(2-pyridin-4-ylethyl)-2-[(2-pyridin-4-ylethyl)- amino]acetamide) were selected for futher clinical studies.

### Compounds-leaders

The main characteristics of the selected compounds are given below:
- the compounds are the small molecules with proved structure and with known method of syntesis
- soluble in water
- correspond to Lipinski's rule of five
- active in behavioral tests in animals
- nontoxic
- noninfringing

All the methods of synthesis developed by the authors of the present application are the subject of the patent.

It is worthy of note that a computational analysis of chemical similarity of the compound ACB-0002 using the most extensive database of the known chemical structures SciFinder revealed only 70% of correspondence with the described molecules.

In fact, the matches of 99% can indicate the difference in the substituents in the ring (dissimilar structures). The matches of 70% indicate the absence of any coincidence that suggests a uniqueness of the patented structures.

### IN VIVO studies

### Behavioral tests in animals: weak memory improvement

The method of one-trial passive avoidance learning task has been used in order to investigate the compounds action on 1-3 days old chicken memory. This is the basic method using for investigation the mechanisms of memory formation. Cross chickens (males) "Ptichnoe" were used in experiments. The animals were delivered at the day of hatching and were kept in pairs in the cages which measured 20x25x20 cm, with constant access to water and feed, at a temperature 28°C and a light cycle of 12:12 hours. The minimal adaptation time was two hours.

### Injections

The compounds were dissolved in sterile saline. Intraperitoneal injections of 0,1 ml were used for systemic administration. Bilateral intracranial injections were administrated to the lateral ventricles with 10 µm microsyringe («Hamilton») using plastic headholder. The volume of injections was 5 µm for hemisphere. To control the localizations of injections the monitoring of the surface of skull and brain were carried out after the experiment and decapitation. Control groups received saline of the same volume.

The experiments were conducted according the following scheme: 20 minutes before training the animals were pretrained by being offered two neutral (wet with water) beaks fixed to a rod. For further training chicks that pecked each bead within first 10 sec were used. Chicks were offered an aversive bead wet with pungent solution (10% solution of methylanthranilate in ethanol). Chicks that pecked the bead showed species-specific defense reaction. Testing was conducted 24 hours after training by offering within 10 seconds a similar but dry aversive bead, and then - a neutral bead. The avoidance of aversive bead was estimated as a long-term memory. To asses the level of memory the percentage of animals demonstrated the avoidance reaction were compared in different experimental groups, statistical confidence of differences was assessed using a nonparametric χ².

**Fig.2****.** The effect of intraventricular administration of the compound ACB-0002 (10 mg) and ACB-0005 (5 mg) 30 min and 5 min before training to play skill passive avoidance in chicks 24 hours after weak training. Control - saline. * -p <0,05. Number of animals in the group with the introduction of ACB-0002: 20, 19, 20, 20, 15, 19, 19, 16; with the introduction ACB-0005: 19, 19, 19, 20, 18, 18, 19, 19.

**Fig.3** Dependence of the effect of the dose of ACB-0005 on weak training 24 hours after training * -p<0,05 control- saline/ H₂O; ** - p<0,01 control - water.

**A.** Intracranial injection (5 ml / hemisphere) 10 minutes before training. Number of animals in the group: 21, 19, 19, 20.18.

**B.** Intraperitoneal injection (0.1 ml) for 10 minutes before training. Number of animals per group: 18, 19, 21, 19, 20.

C. Oral administration (0.1 mL) for 30 minutes before training. Number of animals per group: 19, 20, 20, 19, 19.

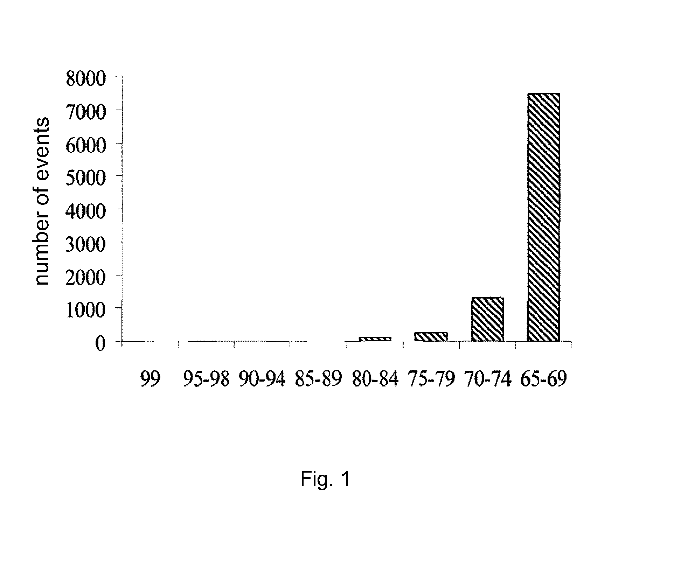

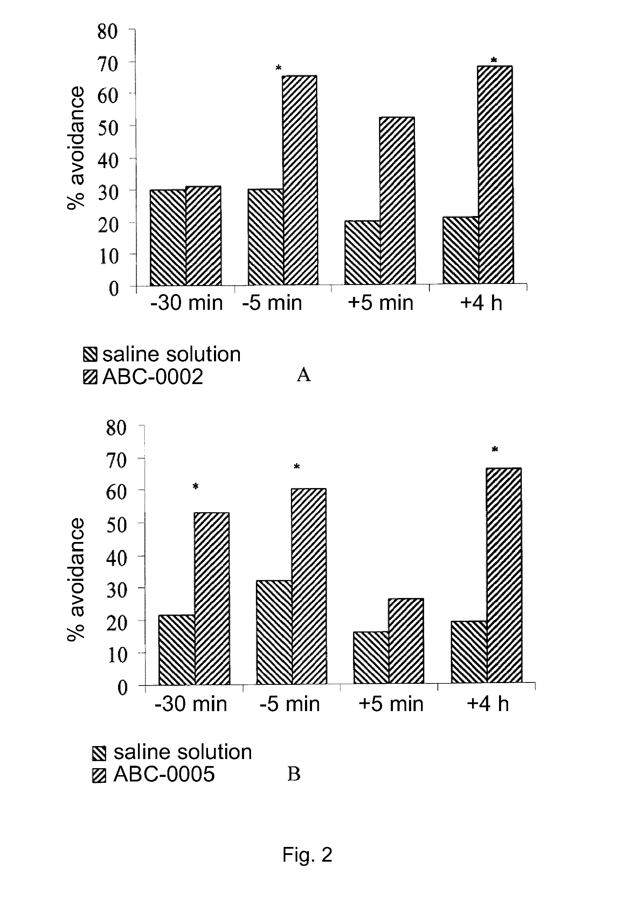

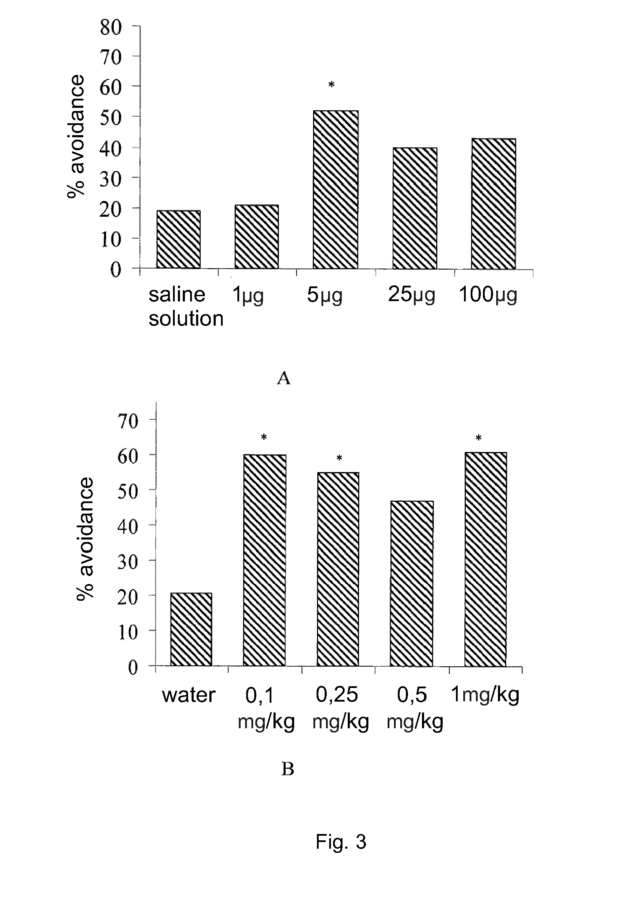

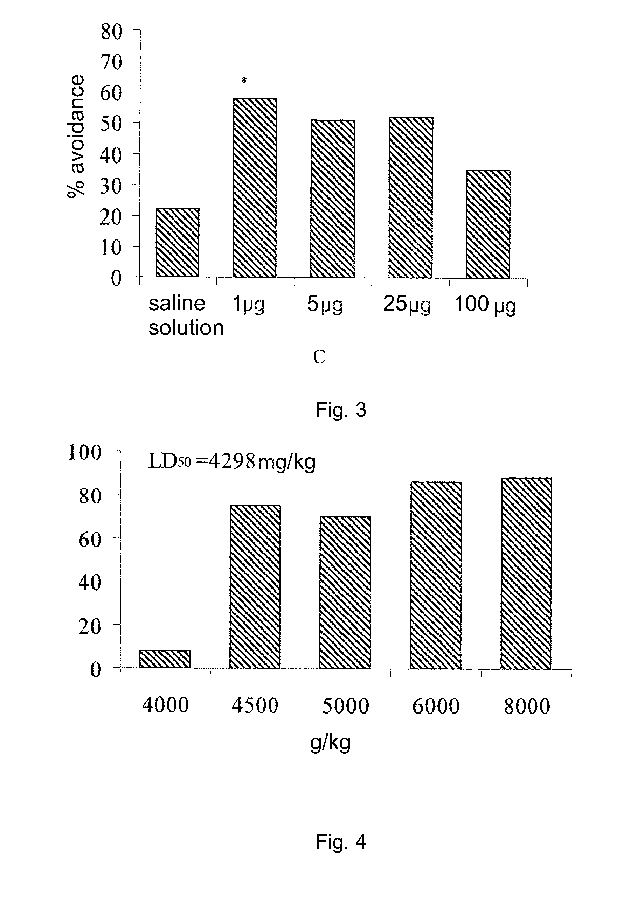

The maximum effect was observed under intracranial injection of ACB-0005 for the dosage of 5 mg 10-30 min before training or for the dosage of 0,1 mg/kg (i.e. 4 mg per animal) 10 min training (Fig.2). Moreover, stimulating effect on memory was observed under oral administration of ACB-0005 30 min before weak treatment at the dosage of 0,1 to 1,0 mg/kg (fig.3).

### Toxicity

In the next series of experiments the acute toxicity of ACB-0002 (P-2) was evaluated (fig.4). The studies were performed on the line C57B6 mice (male) of 22-25 g weight. The tested compound (0,1 ml/10g) were dissolved in distilled water and injected intraperitoneally to 5 groups of 8-12 animals. The following concentrations were investigated: 4000, 4500, 5000, 6000 and 8000 mg/kg. The animals were monitored for two weeks after the beginning of the experiment. The results show the extremely low toxicity of the tested compound:

### Conclusions

It was found that ACB-0002 and ACB-0005 may enhance memory when administrated either immediately before training, or 4 hours after completion of the training which indicates the possibility of potentiation of the processes occurring in late stages of memory formation (for example, the synthesis of effector proteins that provide a modification of neural connections). Consequently, the described compounds may be used to form more stable memory while gaining a new experice and to inforce memory about previous experience.

Thus, the claimed group of compounds can be used to restore memory of patients of all ages in health and none.

## Claims

1. (Azaheterocyclyl)alkyl derivatives of 2-(het)arylglycine amides of the general formula I 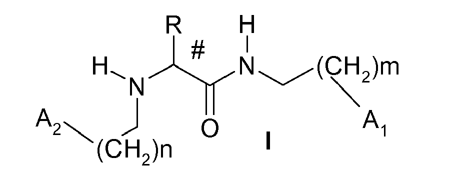 in which:
**n and m** can have the values 0, 1, 2 and 3;
the symbol (#) here and hereinafter signifies the possible presence of a chiral centre;
**R** represents optionally substituted C₅-C₁₀aryl or a 5-7-membered hetaryl containing 1-3 heteroatoms selected from nitrogen, oxygen and sulphur and optionally condensed with a benzene ring;
**A1** and **A2** independently represent an optionally substituted, 3-7-membered, saturated, partially saturated or aromatic azaheterocycle which contains from 1 to 3 atoms of nitrogen in the cycle and is optionally condensed with a benzene ring;
or their pharmaceutically acceptable salts or alkyl esters in the form of individual optical isomers, or combinations thereof.

2. (Azaheterocyclyl)alkyl derivatives of 2-(het)arylglycine amides according to the claim 1, representing (azaheterocyclyl)alkyl derivatives of arylglycine amides of the general formula I.1 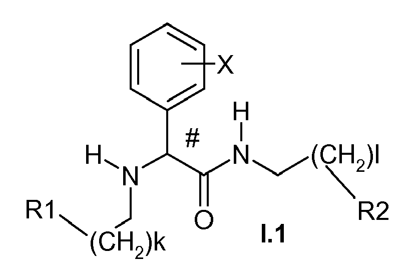 in which:
k and I can have the values 0 and 1;
X represents H, optionally substituted C₁-C₈alkyl, optionally substituted C₁-C₈alkylalkoxy, halogen, OH, CF₃, CN, CF₃O, optionally substituted amino, C₁-C₆acyl, optionally substituted C₆aryl or 5-6-membered hetaryl containing 1-3 heteroatoms, C₂-C₄alkenyl, C₂₋C₄alkynyl, C₆arC₁-C₆alkyl, C₁-C₈alkoxycarbonyl, 5-6-membered hetaryl which contains 1-2 heteroatoms selected from nitrogen, oxygen and sulfur, C₆arylsulfonyl or C₁-C₈ alkylsulfonyl, optionally N-substituted aminosulfonyl;
or **X** represents benzene ring annelated with phenyl or 5-7-membered heterocyclyl containing 1-3 heteroatoms selected from nitrogen, oxygen, sulfur;
**R1** and **R2** independently represent optionally substituted 6-membered azaheterocycle such as pyridin, piperidin, pyrimidine, triazine, quinoline, isoquinoline, quinazoline, quinoxaline, or a pharmaceutically acceptable salt thereof.

3. Compounds according to claim 2 as a mixture of R-(-) - and S-(+)-isomers or individual isomers, respectively, represented by the general formulae **I.1.1** and **I.1.2**: 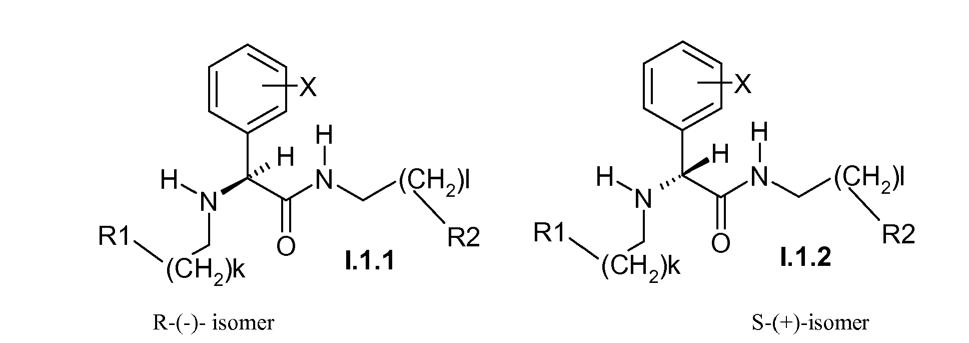 in which:
**k** and I can have the values 0 and 1;
**X** represents H, optionally substituted C₁-C₈alkyl, optionally substituted C₁-C₈alkylalkoxy, halogen, OH, CF₃, CN, CF₃O, optionally substituted amino, C₁-C₆acyl, optionally substituted C₆aryl or 5-6-membered hetaryl containing 1-3 heteroatoms, C₂-C₄alkenyl, C₂₋C₄alkynyl, C₆arC₁-C₆alkyl, C₁-C₈alkoxycarbonyl, 5-6-membered hetaryl which contains 1-2 heteroatoms selected from nitrogen, oxygen and sulfur, C₆arylsulfonyl or C₁-C₈ alkylsulfonyl, optionally N- substituted aminosulfonyl;
or **X** represents benzene ring annelated with phenyl or 5-7-membered heterocyclyl containing 1-3 heteroatoms selected from nitrogen, oxygen, sulfur;
**R1** and **R2** independently represent optionally substituted 6- membered azaheterocycle such as pyridin, piperidin, pyrimidine, triazine, quinoline, isoquinoline, quinazoline, quinoxaline, or a pharmaceutically acceptable salt thereof.

4. Compounds according to the claim 1, selected from the group:
2-(2,4-dichlorophenyl)-N-(2-pyridin-4-ylethyl)-2-[(2-pyridin-4-ylethyl)amino]acetamide;
2-(2,4-dichlorophenyl)-N-(pyridin-4-ylmethyl)-2-[(pyridin-4-ylmethyl)-amino]acetamide;
2-(2,4-dichlorophenyl)-2-[(2-piperidin-4-ylethyl)amino]-N-(2-pyridin-4-ylethyl)acetamide;
2-(2,4-dichlorophenyl)-2-[(2-piperidin-4-ylethyl)-amino]-N-(pyridin-4-ylmethyl)acetamide;
2-(2,5-dimethylphenyl)-N-(2-pyridin-4-ylethyl)-2-[(2-pyridin-3-ylethyl)amino]acetamide;
2-(2,4-dichlorophenyl)-N-(pyridin-4-ylmethyl)-2-[(pyridin-3-ylmethyl)amino]acetamide;
2-(2,4-dichlorophenyl)-N-(pyridin-3-ylmethyl)-2-[(pyridin-4-ylmethyl)amino]acetamide;
2-(2,4-dichlorophenyl)-N-(2-pyridin-3-ylethyl)-2-[(2-pyridin-3-ylethyl)amino]acetamide;
2-(2,4-dichlorophenyl)-N-(2-pyridin-4-ylethyl)-2-[(2-pyridin-3-ylethyl)amino]acetamide;
2-(2,4-dichlorophenyl)-N-(2-pyridin-3-ylethyl)-2-[(2-pyridin-4-ylethyl)-amino]acetamide;
2-(2,5-dichlorophenyl)-N-(2-pyridin-4-ylethyl)-2-[(2-pyridin-4-ylethyl)amino]acetamide;
2-(2,5-dichlorophenyl)-N-(pyridin-4-ylmethyl)-2-[(pyridin-4-ylmethyl)amino]acetamide;
2-(2,5-dichlorophenyl)-2-[(2-piperidin-4-ylethyl)-amino]-N-(2-pyridin-4-ylethyl)acetamide;
2-(2,5-dichlorophenyl)-2-[(2-piperidin-4-ylethyl)amino]-N-(pyridin-4-ylmethyl)acetamide;
2-(2,5-dichlorophenyl)-N-(pyridin-3-ylmethyl)-2-[(pyridin-3-ylmethyl)amino]acetamide;
2-(2,5-dichlorophenyl)-N-(pyridin-4-ylmethyl)-2-[(pyridin-3-ylmethyl)amino]acetamide;
2-(2,5-dichlorophenyl)-N-(pyridin-3-ylmethyl)-2-[(pyridin-4-ylmethyl)amino]acetamide;
2-(2,5-dichlorophenyl)-N-(2-pyridin-3-ylethyl)-2-[(2-pyridin-3-ylethyl)amino]acetamide;
2-(2,5-dichlorophenyl)-N-(2-pyridin-4-ylethyl)-2-[(2-pyridin-3-ylethyl)-amino]acetamide;
2-(2,5-dichlorophenyl)-N-(2-pyridin-3-ylethyl)-2-[(2-pyridin-4-ylethyl)amino]acetamide;
2-(2,4-dimethylphenyl)-N-(2-pyridin-4-ylethyl)-2-[(2-pyridin-4-ylethyl)amino]acetamide;
2-(2,4-dimethylphenyl)-N-(pyridin-4-ylmethyl)-2-[(pyridin-4-ylmethyl)amino]acetamide;
2-(2,4-dimethylphenyl)-2-[(2-piperidin-4-ylethyl)amino]-N-(2-pyridin-4-ylethyl)acetamide;
2-(2,4-dimethylphenyl)-2-[(2-piperidin-4-ylethyl)amino]-N-(pyridin-4-ylmethyl)acetamide;
2-(2,4-dimethylphenyl)-N-(pyridin-3-ylmethyl)-2-[(pyridin-3-ylmethyl)amino]acetamide;
2-(2,4-dimethylphenyl)-N-(pyridin-4-ylmethyl)-2-[(pyridin-3-ylmethyl)amino]acetamide;
2-(2,4-dimethylphenyl)-N-(pyridin -3-ylmethyl)-2-[(pyridin-4-ylmethyl)amino]acetamide;
2-(2,4-dimethylphenyl)-N-(2-pyridin-3-ylethyl)-2-[(2-pyridin-3-ylethyl)amino]acetamide;
2-(2,4-dimethylphenyl)-N-(2-pyridin-4-ylethyl)-2-[(2-pyridin-3-ylethyl)amino]acetamide;
2-(2,4-dimethylphenyl)-N-(2-pyridin-3-ylethyl)-2-[(2-pyridin-4-ylethyl)amino]acetamide;
2-(2,5-dimethylphenyl)-N-(2-pyridin-4-ylethyl)-2-[(2-pyridi-4-ylethyl)amino]acetamide;
2-(2,5-dimethylphenyl)-N-(pyridin-4 ylmethyl)-2-[(pyriclin-4-ylmethyl) amino]acetamide;
2-(2,5-dimethylphenyl)-2-[(2-piperidin-4-ylethyl)amino]-N-(2-pyridin-4-ylethyl)acetamide;
2-(2,5-dimethylphenyl)-2-[(2-piperidin-4-ylethyl)amino]-N-(pyridin-4-ylmethyl)acetamide;
2-(2,5-dimethylphenyl)-N-(pyridin-3-ylmethyl)-2-[(pyridin-3-ylmethyl)amino]acetamide;
2-(2,5-dimethylphenyl)-N-(pyridin-4-ylmethyl)-2-[(pyridin-3-ylmethyl)amino]acetamide;
2-(2,5-dimethylphenyl)-N-(pyridin-3-ylmethyl)-2-[(pyridin-4-ylmethyl)amino]acetamide;
2-(2,5-dimethylphenyl)-N-(2-pyridin-3-ylethyl)-2-[(2-pyridin-3-ylethyl)amino]acetamide;
2-(2,5-dimethylphenyl)-N-(2-pyridin-4-ylethyl)-2-[(2-pyridin-3-ylethyl)amino]acetamide;
2-(2,5-dimethylphenyl)-N-(2-pyridin-3-ylethyl)-2-[(2-pyridin-4-ylethyl)amino]acetamide;
2-(2-ethoxyphenyl)-N-(2-pyridin-4-ylethyl)-2-[(2-pyridin-4-ylethyl)amino]-acetamide;
2-(2-ethoxyphenyl)-N-(pyridin-4-ylmethyl)-2-[(pyridin-4-ylmethyl)-amino]acetamide;
2-(2-ethoxyphenyl)-2-[(2 piperidin-4-ylethyl)amino]-N-(2-pyridin-4-ylethyl)acetamide;
2-(2-ethoxyphenyl)-2-[(2-piperidin-4-ylethyl)amino]-N-(pyridin -4-ylmethyl)acetamide;
2-(2-ethoxyphenyl)-N-(pyridin-3-ylmethyl)-2-[(pyridin-3-ylmethyl)amino]acetamide;
2-(2-ethoxyphenyl)-N-(pyridin-4-ylmethyl)-2-[(-(pyridin-3-ylmethyl)amino]acetamide;
2-(2-ethoxyphenyl)-N-(pyridin-3-ylmethyl)-2-[(pyridin-4-ylmethyl)amino]acetamide;
2-(2-ethoxyphenyl)-N-(2 pyridin-3-ylethyl)-2-[(2-pyridin-3-ylethyl)amino]acetamide;
2-(2-ethoxyphenyl)-N-(2-pyridin-4-ylethyl)-2-[(2-pyridin-3-ylethyl)amino]acetamide;
2-(2-ethoxyphenyl)-N-(2-pyridin-3-ylethyl)-2-[(2 pyridin-4 ylethyl)amino]acetamide;
2-(4-ethoxyphenyl)-N-(2-pyridin-4-ylethyl)-2-[(2 pyridin-4-ylethyl)amino]acetamide;
2-(4-ethoxyphenyl)-N-(pyridin-4-ylmethyl)-2-[(pyridin-4-ylmethyl)amino]acetamide;
2-(4-ethoxyphenyl)-2-[(2-piperidin-4-ylethyl)amino]-N-(2-pyridin-4-ylethyl)acetamide;
2-(4-ethoxyphenyl)-2-[(2-piperidin-4-ylethyl)amino]-N-(pyridin-4-ylmethyl)acetamide;
2-(4-ethoxyphenyl)-N-(pyridin-3-ylmethyl)-2-[(pyridin-3-ylmethyl)amino]acetamide;
2-(4-ethoxyphenyl)-N-(pyridin-4-ylmethyl)-2-[(pyridin-3-ylmethyl)amino]acetamide;
2-(4-ethoxyphenyl)-N-(pyridin-3-ylmethyl)-2-[pyridin-4-ylmethyl) amino]acetamide;
2-(4-ethoxyphenyl)-N-(2-pyridin-3-ylethyl)-2-[(2-pyridin-3-ylethyl) amino]acetamide;
2-(4-ethoxyphenyl)-N-(2-pyridin-4-ylethyl)-2-[(2-pyridin-3-ylethyl) amino]acetamide;
2-(4-ethoxyphenyl)-N-(2-pyridin-3-ylethyl)-2-[(2-pyridin-4-ylethyl)amino]acetamide;
2-(3-ethoxyphenyl)-N-(2-pyridin-4-ylethyl)-2-[(2-pyridin-4-ylethyl)amino]acetamide;
2-(3-ethoxyphenyl)-N-(pyridin-4-ylmethyl)-2-[(pyridin-4-ylmethyl)amino]acetamide;
2-(3-ethoxyphenyl)-2-[(2-piperidin-4-ylethyl)amino]-N-(2-pyridin-4-ylethyl)acetamide;
2-(3-ethoxyphenyl)-2-[(2-piperidin-4-ylethyl)amino]-N-(pyridin-4-ylmethyl)acetamide;
2-(3-ethoxyphenyl)-N-(pyridin-3-ylmethyl)-2-[(pyridin-3-ylmethyl)amino]-acetamide;
2-(3-ethoxyphenyl)-N-(pyridin-4-ylmethyl)-2-[(pyridin-3-ylmethyl)amino]acetamide;
2-(3-ethoxyphenyl)-N-(pyridin-3-ylmethyl)-2-[(pyridin-4-ylmethyl)amino]acetamide;
2-(3-ethoxyphenyl)-N-(2-pyridin-4-ylethyl)-2-[(2-pyridin-3-ylethyl)amino]acetamide;
2-(3-ethoxyphenyl)-N-(2-pyridin-3-ylethyl)-2-[(2-pyridin-4-ylethyl)amino]acetamide;
N-(2-pyridin-4-ylethyl)-2-[(2-pyridin-4-ylethyl)amino]-2-[2-(trifluoromethyl)phenyl]acetamide;
N-(pyridin-4-ylmethyl)-2-[(pyridin-4-ylmethyl) amino]-2-[2-(trifluoromethyl)phenyl]acetamide;
2-[(2-piperidin-4-ylethyl)amino]-N-(2-pyridin-4-ylethyl)-2-[2-(trifluoromethyl)phenyl]acetamide;
2-[(2-piperidin-4-ylethyl)amino]-N-(pyridin-4-ylmethyl)-2-[2-(trifluoromethyl)phenyl]acetamide;
N-(pyridin-3-ylmethyl)-2-[(pyridin-3-ylmethyl) amino]-2-[2-(trifluoromethyl)phenyl]acetamide;
N-(pyridin-4-ylmethyl)-2-[(pyridin-3-ylmethyl) amino]-2-[2-(trifluoromethyl)phenyl]acetamide;
N-(pyridin-3-ylmethyl)-2-[(pyridin-4-ylmethyl) amino]-2-[2-(trifluoromethyl)phenyl]acetamide;
N-(2-pyridin-3-ylethyl)-2-[(2-pyridin-3-ylethyl) amino]-2-[2-(trifluoromethyl)phenyl]acetamide;
N-(2-pyridin-4-ylethyl)-2-[(2-pyridin-3-ylethyl)amino]-2-[2-(trifluoromethyl)phenyl]acetamide;
N-(2-pyridin-3-ylethyl)-2-[(2-pyridin-4-ylethyl)amino]-2-[2-(trifluoromethyl)phenyl]acetamide;
N-(2-pyridin-4-ylethyl)-2-[(2-pyridin-4-ylethyl)amino]-2-[3-(trifluoromethyl)phenyl]acetamide;
N-(pyridin-4-ylmethyl)-2-[(pyridin-4-ylmethyl)amino]-2-[3-(trifluoromethyl) henyl]acetamide;
2-[(2-piperidin-4-ylethyl)amino]-N-(2-piperidin-4-ylethyl)-2-[3(trifluoromethyl)phenyl]acetamide;
2-[(2-piperidin-4-ylethyl)amino]-N-(pyridin-4-ylmethyl)-2-3-(trifluoromethyl)phenyl]acetamide;
N-(pyridin-3-ylmethyl)-2-[(pyridin-3-ylmethyl)amino-2-[3-(trifluoromethyl)phenyl]acetamide;
N-(pyridin-4-ylmethyl)-2-[(pyridin-3-ylmethyl)amino]-2-[3-(trifluoromethyl) phenyl] acetamide;
N-(pyridin-3-ylmethyl)-2-[(pyridin-4-ylmethyl)amino]-2-[3-trifluoromethyl) phenyl] acetamide;
N-(2-pyridin-3-ylethyl)-2-[(2-pyridin-3-ylethyl)amino]-2-[3-trifluoromethyl)phenyl]acetamide;
N-(2-pyridin-4-ylethyl)-2-[(2-pyridin-3-ylethyl)amino]-2-[3-(trifluoromethyl)phenyl]acetamide;
N-(2-pyridin-3-ylethyl)-2-[(2-pyridin-4-ylethyl)amino]-2-[3-(trifluoromethyl)phenyl]acetamide;
2-[4-(methylthio)phenyl]-N-(2-pyridin-4-ylethyl)-2-[(2-pyridin-4-ylethyl)amino]acetamide;
2-[4-(methylthio) phenyl]-N-(pyridin-4-ylmethyl)-2-[(pyridin-4-ylmethyl)amino]acetamide;
2-[4-(methylthio)phenyl]-2-[(2-piperidin-4-ylethyl)amino]-N-(2-pyridin-4-ylethyl)acetamide;
2-[4-(methylthio) phenyl]-2-[(2-piperidin-4-ylethyl)amino]-N-(pyridin-4-ylmethyl)acetamide;
2-[4-(methylthio)phenyl]-N-(pyridin-3-ylmethyl)-2-[(pyridin-3-ylmethyl)amino]-acetamide;
2-[4-(methylthio)phenyl]-N-(pyridin-4-ylmethyl)-2-[(pyridin-3-ylmethyl)-amino]acetamide;
2-[4-(methylthio)phenyl]-N-(pyridin-3-ylmethyl)-2-[(pyridin-4-ylmethyl)amino]acetamide;
2-[4-(methylthio)phenyl]-N-(2-pyridin-3-ylethyl)-2-[(2-pyridin-3-ylethyl)amino]acetamide;
2-[4-(methylthio) phenyl]-N-(2-pyridin-4-ylethyl)-2-[(2-pyridin-3-ylethyl)amino]acetamide;
2-[4-(methylthio)phenyl]-N-(2-pyridin-3-ylethyl)-2-[(2-pyridin-4-ylethyl)amino]acetamide;
N-(2-pyridin-4-ylethyl)-2-[(2-pyridin-4-ylethyl)amino]-2-[4-(trifluoromethyl) phenyl]acetamide;
N-(pyridin-4-ylmethyl)-2-[(pyridin-4-ylmethyl)amino]-2-[4-(trifluoromethyl)phenyl]acetamide;
2-[(2-piperidin-4-ylethyl)amino]-N-(2-pyridin-4-ylethyl)-2-[4-(rifluoromethyl)phenyl]acetamide;
2-[(2-piperidin-4-ylethyl)amino]-N-(pyridin-4-ylmethyl)-2-[4-(trifluoromethyl)phenyl]acetamide;
N-(pyridin-3-ylmethyl)-2-[(pyridin-3-ylmethyl)amino]-2-[4-(trifluoromethyl)phenyl]acetamide;
N-(pyridin-4-ylmethyl)-2-[(pyridin-3-ylmethyl)amino]-2-[4-(trifluoromethyl)phenyl]acetamide;
N-(pyridin-3-ylmethyl)-2-[(pyridin-4-ylmethyl)amino]-2-[4-(trifluoromethyl)phenyl]acetamide;
N-(2-pyridin-3-ylethyl)-2-[(2-pyridin-3-ylethyl)amino]-2-[4-(trifluoromethyl)phenyl]acetamide;
N-(2-pyridin-4-ylethyl)-2-[(2-pyridin-3-ylethyl)amino]-2-[4-(trifluoromethyl)phenyl]acetamide;
N-(2-pyridin-3-ylethyl)-2-[(2-pyridin-4-ylethyl)amino]-2-[4-(trifluoromethyl)phenyl]acetamide;
2-(4-methoxyphenyl)-N-(2-pyridin-4-ylethyl)-2-[(2-pyridin-4-ylethyl)amino]acetamide;
2-(4-methoxyphenyl)-N-(pyriclin-4-ylmethyl)-2-[(pyridin-4-ylmethyl)amino]acetamide;
2-(4-methoxyphenyl)-2-[(2-piperidin-4-ylethyl)amino]-N-(2-pyridin-4-ylethyl)acetamide;
2-(4-methoxyphenyl)-2-[(2-piperidin-4-ylethyl)amino]-N-(pyridin-4-ylmethyl)acetamide;
2-(4-methoxyphenyl)-N-(pyridin-3-ylmethyl)-2-[(pyridin-3-ylmethyl)amino]acetamide;
2-(4-methoxyphenyl)-N-(pyridin-4-ylmethyl)-2-[(pyridin-3-ylmethyl)amino]acetamide;
2-(4-methoxyphenyl)-N-(pyridin-3-ylmethyl)-2-[(pyridin-4-ylmethyl)amino]acetamide;
2-(4-methoxyphenyl)-N-(2-pyridin-3-ylethyl)-2-[(2-pyridin-3-ylethyl)amino]acetamide;
2-(4-methoxyphenyl)-N-(2-pyridin-4-ylethyl)-2-[(2-pyridin-3-ylethyl)amino]acetamide;
2-(4-methoxyphenyl)-N-(2-pyridin-3-ylethyl)-2-[(2-pyridin-4-ylethyl)amino]acetamide;
2-(3-methoxyphenyl)-N-(2-pyridin-4-ylethyl)-2-[(2-pyridin-4-ylethyl)amino]acetamide;
2-(3-methoxyphenyl)-N-(pyridin-4-ylmethyl)-2-[(pyridin-4-ylmethyl)amino]acetamide;
2-(3-methoxyphenyl)-2-[(2-piperidin-4-ylethyl)amino]-N-(2-pyridin-4-ylethyl)acetamide;
2-(3-methoxyphenyl)-2-[(2-piperidin-4-ylethyl)amino]-N-(pyridin-4-ylmethyl)acetamide;
2-(3-methoxyphenyl)-N-(pyridin-3-ylmethyl)-2-[(pyridin-3-ylmethyl)amino]acetamide;
2-(3-methoxyphenyl)-N-(pyridin-4-ylmethyl)-2-[(pyridin-3-ylmethyl)amino]acetamide;
2-(3-methoxyphenyl)-N-(pyridin-3-ylmethyl)-2-[(pyridin-4-ylmethyl)amino]acetamide;
2-(3-methoxyphenyl)-N-(2-pyridin-3-ylethyl)-2-[(2-pyridin-3-ylethyl)ammo]acetamide;
2-(3-methoxyphenyl)-N-(2-pyridin-4-ylethyl)-2-[(2-pyridin-3-ylethyl)amino]acetamide;
2-(3-methoxyphenyl)-N-(2-pyridin-3-ylethyl)-2-[(2-pyridin-4-ylethyl)amino]acetamide;
2-(2-methoxyphenyl)-N-(2-pyridin-4-ylethyl)-2-[(2-pyridin-4-ylethyl)amino]acetamide;
2-(2-methoxyphenyl)-N-(pyridin-4-ylmethyl)-2-[(pyridin-4-ylmethyl)amino]acetamide;
2-(2-methoxyphenyl)-2-[(2-piperidin-4-ylethyl)amino]-N-(2-piperidin-4-ylethyl)amino)acetamide;
2-(2-methoxyphenyl)-2-[(2-piperidin-4-ylethyl)amino]-N-(pyridin-4-ylmethyl)acetamide;
2-(2-methoxyphenyl)-N-(pyridin-3-ylmethyl)-2-[(pyridin-3-ylmethyl)amino]acetamide;
2-(2-methoxyphenyl)-N-(pyridin-4-ylmethyl)-2-[(pyridin-3-ylmethyl)amino]acetamide;
2-(2-methoxyphenyl)-N-(pyridin-3-ylmethyl)-2-[(pyridin-4-ylmethyl)amino]acetamide;
2-(2-methoxyphenyl)-N-(2-pyridin-3-ylethyl)-2-[(2-pyridin-3-ylethyl)amino]acetamide;
2-(2-methoxyphenyl)-N-(2-pyridin-4-ylethyl)-2-[(2-pyridin-3-ylethyl)amino]acetamide;
2-(2-methoxyphenyl)-N-(2-pyridin-3-ylethyl)-2-[(2-pyridin-4-ylethyl)amino]acetamide;
2-(3-chlorophenyl)-N-(2-pyridin-4-ylethyl)-2-[(2-pyridin-4-ylethyl)amino]acetamide;
2-(3-chlorophenyl)-N-(pyridin-4-ylethyl)-2-[(pyridin-4-ylethyl)amino]acetamide;
2-(3-chlorophenyl)-2-[(2-piperidin-4-ylethyl)amino]-N-(2-pyridin-4-ylethyl)acetamide;
2-(3-chlorophenyl)-2-[(2-piperidin-4-ylethyl)amino]-N-(pyridin-4-ylethyl)acetamide;
2-(3-chlorophenyl)-N-(pyridin-3-ylmethyl)-2-[(pyridin-3-ylmethyl)amino]acetamide;
2-(3-chlorophenyl)-N-(pyridin-4-ylmethyl)-2-[(pyridin-3-ylmethyl)amino]acetamide;
2-(3-chlorophenyl)-N-(pyridin-3-ylmethyl)-2-[(pyridin-4-ylmethyl)amino]acetamide;
2-(3-chlorophenyl)-N-(2-pyridin-3-ylethyl)-2-[(2-pyridin-3-ylethyl)amino]acetamide;
2-(3-chlorophenyl)-N-(2-pyridin-4-ylethyl)-2-[(2-pyridin-3-ylethyl amino]acetamide;
2-(3-chlorophenyl)-N-(2-pyridin-3-ylethyl)-2-[(2-pyridin-4-ylethyl)amino]acetamide;
2-(2-bromophenyl)-N-(2-pyridin-4-ylethyl)-2-[(2-pyridin-4-ylethyl)amino]acetamide;
2-(2-bromophenyl)-N-(pyridin-4-ylmethyl)-2-[(pyridin-4-ylmethyl)amino]acetamide;
2-(2-bromophenyl)-2-[(2-piperidin-4-ylethyl)amino]-N-(2-pyridin-4-ylethyl)acetamide;
2-(2-bromophenyl)-2-[(2-piperidin-4-ylethyl)amino]-N-(pyridin-4-ylmethyl)acetamide;
2-(2-bromophenyl)-N-(pyridin-3-ylmethyl)-2-[(pyridin-3-ylmethyl)amino]acetamide;
2-(2-bromophenyl)-N-(pyridin-4-ylmethyl)-2-[(pyridin-3-ylmethyl)amino]acetamide;
2-(2-bromophenyl)-N-(pyridin-3-ylmethyl)-2-[(pyridin-4-ylmethyl)amino]acetamide;
2-(2-bromophenyl)-N-(2-pyridin-3-ylethyl)-2-[(2-pyridin-3-ylethyl)amino]acetamide;
2-(2-bromophenyl)-N-(2-pyridin-4-ylethyl)-2-[(2-pyridin-3-ylethyl)amino]acetamide;
2-(2-bromophenyl)-N-(2-pyridin-3-ylethyl)-2-[(2-pyridin-4-ylethyl)amino]acetamide;
2-(3-bromophenyl)-N-(2-pyridin-4-ylethyl)-2-[(2-pyridin-4-ylethyl)amino]acetamide;
2-(3-bromophenyl)-N-(pyridin-4-ylmethyl)-2-[(pyridin-4-ylmethyl)amino]acetamide;
2-(3-bromophenyl)-2-[(2-piperidin-4-ylethyl)amino]-N-(2-pyridin-4-ylethyl)acetamide;
2-(3-bromophenyl)-2-[(2-piperidin-4-ylethyl)amino]-N-(pyridin-4-ylmethyl)acetamide;
2-(3-bromophenyl)-N-(pyridin-3-ylmethyl)-2-[(pyridin-3-ylmethyl)amino]acetamide;
2-(3-bromophenyl)-N-(pyridin-4-ylmethyl)-2-[(pyridin-3-ylmethyl)amino]acetamide;
2-(3-bromophenyl)-N-(pyridin-3-ylmethyl)-2-[(pyridin-4-ylmethyl)amino]acetamide;
2-(3-bromophenyl)-N-(2-pyridin-3-ylethyl)-2-[(2-pyridin-3-ylethyl)amino]acetamide;
2-(3-bromophenyl)-N-(2-pyridin-4-ylethyl)-2-[(2-pyridin-3-ylethyl)amino]acetamide;
2-(3-bromophenyl)-N-(2-pyridin-3-ylethyl)-2-[(2-pyridin-4-ylethyl)amino]acetamide;
2-(4-bromophenyl)-N-(2-pyridin-4-ylethyl)-2-[(2-pyridin-4-ylethyl)amino]acetamide;
2-(4-bromophenyl)-N-(pyridin-4-ylmethyl)-2-[(pyridin-4-ylmethyl)amino]acetamide;
2-(4-bromophenyl)-2-[(2-piperidin-4-ylethyl)amino]-N-(2-pyridin-4-ylethyl)acetamide;
2-(4-bromophenyl)-2-[(2-piperidin-4-ylethyl)amino]-N-(pyridin-4-ylmethyl)acetamide;
2-(4-bromophenyl)-N-(pyridin-3-ylmethyl)-2-[(pyridin-3-ylmethyl)amino]acetamide;
2-(4-bromophenyl)-N-(pyridin-4-ylmethyl)-2-[(pyridin-3-ylmethyl)amino]acetamide;
2-(4-bromophenyl)-N-(pyridin-3-ylmethyl)-2-[(pyridin-4-ylmethyl)amino]acetamide;
2-(4-bromophenyl)-N-(2-pyridin-3-ylethyl)-2-[(2-pyridin-3-ylethyl)amino]acetamide;
2-(4-bromophenyl)-N-(2-pyridin-4-ylethyl)-2-[(2-pyridin-3-ylethyl)amino]acetamide;
2-(4-bromophenyl)-N-(2-pyridin-3-ylethyl)-2-[(2-pyridin-4-ylethyl)amino]acetamide;
2-(4-chlorophenyl)-N-(2-pyridin-4-ylethyl)-2-[(2-pyridin-4-ylethyl)amino]acetamide;
2-(4-chlorophenyl)-N-(pyridin-4-ylmethyl)-2-[(pyridin-4-ylmethyl)amino]acetamide;
2-(4-chlorophenyl)-2-[(2-piperidin-4-ylethyl)amino]-N-(2-pyridin-4-ylethyl)acetamide;
2-(4-chlorophenyl)-2-[(2-piperidin-4-ylethyl)amino]-N-(pyridin-4-ylmethyl)acetamide;
2-(4-chlorophenyl)-N-(pyridin-3-ylmethyl)-2-[(pyridin-3-ylmethyl amino]acetamide;
2-(4-chlorophenyl)-N-(pyridin-4-ylmethyl)-2-[(pyridin-3-ylmethyl)amino]acetamide;
2-(4-chlorophenyl)-N-(pyridin-3-ylmethyl)-2-[(pyridin-4-ylmethyl)amino]acetamide;
2-(4-chlorophenyl)-N-(2-pyridin-3-ylethyl)-2-[(2-pyridin-3-ylethyl)amino]acetamide;
2-(4-chlorophenyl)-N-(2-pyridin-4-ylethyl)-2-[(2-pyridin-3-ylethyl)amino]acetamide;
2-(4-chlorophenyl)-N-(2-pyridin-3-ylethyl)-2-[(2-pyridin-4-ylethyl)amino]acetamide;
2-(2-chlorophenyl)-N-(2-pyridin-4-ylethyl)-2-[(2-pyridin-4-ylethyl)amino]acetamide;
2-(2-chlorophenyl)-N-(pyridin-4-ylmethyl)-2-[(pyridin-4-ylmethyl)amino]acetamide;
2-(2-chlorophenyl)-2-[(2-piperidin-4-ylethyl)amino]-N-(2-pyridin-4-ylethyl)acetamide;
2-(2-chlorophenyl)-2-[(2-piperidin-4-ylethyl)amino]-N-(pyridin-4-ylmethyl)acetamide;
2-(2-chlorophenyl)-N-(pyridin-3-ylmethyl)-2-[(pyridin-3-ylmethyl)amino] acetamide;
2-(2-chlorophenyl)-N-(pyridin-4-ylmethyl)-2-[(pyridin-3-ylmethyl)amino]acetamide;
2-(2-chlorophenyl)-N-(pyridin-3-ylmethyl)-2-[(pyridin-4-ylmethyl)amino]acetamide;
2-(2-chlorophenyl)-N-(2-pyridin-3-ylethyl)-2-[(2-pyridin-3-ylethyl)amino]acetamide;
2-(2-chlorophenyl)-N-(2-pyridin-4-ylethyl)-2-[(2-pyridin-3-ylethyl)amino]acetamide;
2-(2-chlorophenyl)-N-(2 pyridin-3-ylethyl)-2-[(2-pyridin-4-ylethyl)amino]acetamide;
2-(2-fluorophenyl)-N-(2-pyridin-4-ylethyl)-2-[(2-pyridin-4-ylethyl)amino]acetamide;
2-(2-fluorophenyl)-N-(pyridin-4-ylmethyl)-2-[(pyridin-4-ylmethyl)amino]acetamide;
2-(2-fluorophenyl)-2-[(2-piperidin-4-ylethyl)amino]-N-(2-pyridin-4-ylethyl)acetamide;
2-(2-fluorophenyl)-2-[(2-piperidin-4-ylethyl)amino]-N-(pyridin-4-ylmethyl)acetamide;
2-(2-fluorophenyl)-N-(pyridin-3-ylmethyl)-2-[(pyridin-3-ylmethyl)amino]acetamide;
2-(2-fluorophenyl)-N-(pyridin-4-ylmethyl)-2-[(pyridin-3-ylmethyl)amino]acetamide;
2-(2-fluorophenyl)-N-(pyridin-3-ylmethyl)-2-[(pyridin-4-ylmethyl)amino]acetamide;
2-(2-fluorophenyl)-N-(2-pyridin-3-ylethyl)-2-[(2-pyridin-3-ylethyl)amino]acetamide;
2-(2-fluorophenyl)-N-(2-pyridin-4-ylethyl)-2-[(2-pyridin-3-ylethyl)amino]acetamide;
2-(2-fluorophenyl)-N-(2-pyridin-3-ylethyl)-2-[(2-pyridin-4-ylethyl)amino]acetamide;
2-(2,4-dimethoxyphenyl)-N-(2-pyridin-4-ylethyl)-2-[(2-pyridin-4-ylethyl)amino]-acetamide;
2-(2,4-dimethoxyphenyl)-N-(pyridin-4-ylmethyl)-2-[(pyridin-4-ylmethyl)amino]acetamide;
2-(2,4-dimethoxyphenyl)-2-[(2-piperidin-4-ylethyl)amino]-N-(2-pyridin-4-ylethyl)acetamide;
2-(2,4-dimethoxyphenyl)-2-[(2-piperidin-4-ylethyl)amino]-N-(pyridin-4-ylmethyl)acetamide;
2-(2,4-dimethoxyphenyl)-N-(pyridin-3-ylmethyl)-2-[(pyridin-3-ylmethyl)amino]acetamide;
2-(2,4-dimethoxyphenyl)-N-(pyridin-4-ylmethyl)-2-[(pyridin-3-ylmethyl) amino]acetamide;
2-(2,4-dimethoxyphenyl)-N-(pyridin-3-ylmethyl)-2-[(pyridin-4-ylmethyl)amino]acetamide;
2-(2,4-dimethoxyphenyl)-N-(2-pyridin-3-ylethyl)-2-[(2-pyridin-3-ylethyl)amino]acetamide;
2-(2,4-dimethoxyphenyl)-N-(2-pyridin-4-ylethyl)-2-[(2-pyridin-3-ylethyl)amino]acetamide;
2-(2,4-dimethoxyphenyl)-N-(2-pyriclin-3-ylethyl)-2-[(2-pyridin-4-ylethyl)amino]acetamide;
2-(2,5-dimethoxyphenyl)-N-(2-pyridin-4-ylethyl)-2-[(2-pyridin-4-ylethyl)amino]acetamide;
2-(2,5-dimethoxyphenyl)-N-(pyridin-4-ylmethyl)-2-[(pyridin-4-ylmethyl)amino]acetamide;
2-(2,5-dimethoxyphenyl)-2-[(2-piperidin-4-ylethyl)amino]-N-(2-pyridin-4-ylethyl)acetamide;
2-(2,5-dimethoxyphenyl)-2-[(2-piperidin-4-ylethyl)amino]-N-(pyridin-4-ylmethyl)acetamide;
2-(2,5-dimethoxyphenyl)-N-(pyridin-4-ylmethyl)-2-[(pyridin-4-ylmethyl)amino]acetamide;
2-(2,5-dimethoxyphenyl)-N-(pyridin-4-ylmethyl)-2-[(pyridin-3-ylmethyl)amino]acetamide;
2-(2,5-dimethoxyphenyl)-N-(pyridin-3-ylmethyl)-2-[(pyridin-4-ylmethyl)amino]acetamide;
2-(2,5-dimethoxyphenyl)-N-(2-pyridin-3-ylmethyl)-2-[(2-pyridin-3-ylmethyl)amino]acetamide;
2-(2,5-dimethoxyphenyl)-N-(2-pyridin-4-ylethyl)-2-[(2-pyridin-3-ylethyl)amino]acetamide;
2-(2,5-dimethoxyphenyl)-N-(2-pyridin-3-ylethyl)-2-[(2-pyridin-4-ylethyl)amino]-acetamide;
2-(3,4-dimethoxyphenyl)-N-(2-pyridin-4-ylethyl)-2-[(2-pyridin-4-ylethyl)amino]acetamide;
2-(3,4-dimethoxyphenyl)-N-(pyridin-4-ylmethyl)-2-[(pyridin-4-ylmethyl)amino]acetamide;
2-(3,4-dimethoxyphenyl)-2-[(2-piperidin-4-ylethyl)amino]-N-(2-pyridin-4-ylethyl)acetamide;
2-(3,4-dimethoxyphenyl)-2-[(2-piperidin-4-ylethyl)amino]-N-(pyridin-4-ylmethyl)acetamide;
2-(3,4-dimethoxyphenyl)-N-(pyridin-3-ylmethyl)-2-[(pyridin-3-ylmethyl)amino]acetamide;
2-(3,4-dimethoxyphenyl)-N-(pyridin-4-ylmethyl)-2-[(pyridin-3-ylmethyl)amino]acetamide;
2-(3,4-dimethoxyphenyl)-N-(pyridin-3-ylmethyl)-2-[(pyridin-4-ylmethyl)amino]acetamide;
2-(3,4-dimethoxyphenyl)-N-(2-pyridin-3-ylethyl)-2-[(2-pyridin-3-ylethyl)amino]acetamide;
2-(3,4-dimethoxyphenyl)-N-(2-pyridin-4-ylethyl)-2-[(2-pyridin-3-ylethyl)amino]acetamide;
2-(3,4-dimethoxyphenyl)-N-(2-pyridin-3-ylethyl)-2-[(2-pyridin-4-ylethyl)amino]acetamide;
2-(3-fluorophenyl)-N-(2-pyridin-4-ylethyl)-2-[(2-pyridin-4-ylethyl)amino]acetamide;
2-(3-fluorophenyl)-N-(pyridin-4-ylmethyl)-2-[(pyridin-4-ylmethyl)amino]acetamide;
2-(3-fluorophenyl)-2-[(2-piperidin-4-ylethyl)amino]-N-(2-pyridin-4-ylethyl)acetamide;
2-(3-fluorophenyl)-2-[(2-piperidin-4-ylethyl)amino]-N-(pyridin-4-ylmethyl)acetamide;
2-(3-fluorophenyl)-N-(pyridin-3-ylmethyl)-2-[(pyridin-3-ylmethyl)amino]acetamide;
2-(3-fluorophenyl)-N-(pyridin-4-ylmethyl)-2-[(pyridin-3-ylmethyl)amino] acetamide;
2-(3-fluorophenyl)-N-(pyridin-3-ylmethyl)-2-[(pyridin-4-ylmethyl)amino]acetamide;
2-(3-fluorophenyl)-N-(2-pyridin-3-ylethyl)-2-[(2-pyridin-3-ylethyl)amino]acetamide;
2-(3-fluorophenyl)-N-(2-pyridin-3-ylethyl)-2-[(2-pyridin-3-ylethyl)amino]acetamide;
2-(3-fluorophenyl)-N-(2-pyridin-3-ylethyl)-2-[(2-pyridin-4-ylethyl)amino]acetamide;
2-(4-fluorophenyl)-N-(2-pyridin-4-ylethyl)-2-[(2-pyridin-4-ylethyl)amino]acetamide;
2-(4-fluorophenyl)-N-(pyridin-4-ylmethyl)-2-[(pyridin-4-ylmethyl)amino]acetamide;
2-(4-fluorophenyl)-2-[(2-piperidin-4-ylethyl)amino]-N-(2-pyridin-4-ylethyl)acetamide;
2-(4-fluorophenyl)-2-[(2-piperidin-4-ylethyl)amino]-N-(pyridin-4-ylmethyl)acetamide;
2-(4-fluorophenyl)-N-(pyridin-3-ylmethyl)-2-[(pyridin-3-ylmethyl)amino]acetamide;
2-(4-fluorophenyl)-N-(pyridin-4-ylmethyl)-2-[(pyridin-3-ylmethyl)amino]acetamide;
2-(4-fluorophenyl)-N-(pyridin-3-ylmethyl)-2-[(pyridin-4-ylmethyl)amino]acetamide;
2-(4-fluorophenyl)-N-(2-pyridin-3-ylethyl)-2-[(2-pyridin-3-ylethyl)amino]acetamide;
2-(4-fluorophenyl)-N-(2-pyridin-4-ylethyl)-2-[(2-pyridin-3-ylethyl)amino]acetamide;
2-(4-fluorophenyl)-N-(2-pyridin-3-ylethyl)-2-[(2-pyridin-4-ylethyl)amino]acetamide;
2-(1-naphthyl)-N-(2-pyridin-4-ylethyl)-2-[(2-pyridin-4-ylethyl)amino]acetamide;
2-(1-naphthyl)-N-(pyridin-4-ylmethyl)-2-[(pyridin-4-ylmethyl)amino] acetamide;
2-(1-naphthyl)-2-[(2-piperidin-4-ylethyl)amino]-N-(2-pyridin-4-ylethyl)acetamide;
2-(1-naphthyl)-2-[(2-piperidin-4-ylethyl)amino]-N-(pyridin-4-ylmethyl) acetamide;
2-(1-naphthyl)-N-(pyridin-3-ylmethyl)-2-[(pyridin-3-ylmethyl)amino]acetamide;
2-(1-naphthyl)-N-(pyridin-4-ylmethyl)-2-[(pyridin-3-ylmethyl)amino]acetamide;
2-(1-naphthyl)-N-(pyridin-3-ylmethyl)-2-[(pyridin-4-ylmethyl)amino]acetamide;
2-(1-naphthyl)-N-(2-pyridin-3-ylethyl)-2-[(2-pyridin-3-ylethyl)amino]acetamide;
2-(1-naphthyl)-N-(2-pyridin-4-ylethyl)-2-[(2-pyridin-3-ylethyl)amino]acetamide;
2-(1-naphthyl)-N-(2-pyridin-3-ylethyl)-2-[(2-pyridin-4-ylethyl)amino]acetamide;
2-(1,3-benzodioxole-5-yl)-N-(2-pyridin-4-ylethyl)-2-[(2-pyridin-4-ylethyl)amino]acetamide;
2-(1,3-benzodioxole-5-yl)-N-(pyridin-4-ylmethyl)-2-[(pyridin-4-ylmethyl)amino]acetamide;
2-(1,3-benzodioxole-5-yl)-2-[(2-piperidin-4-ylethyl)amino]-N-(2-pyridin-4-ylethyl)acetamide;
2-(1,3-benzodioxole-5-yl)-2-[(2-piperidin-4-ylethyl)amino]-N-(pyridin-4-ylmethyl)acetamide;
2-(1,3-benzodioxole-5-yl)-N-(pyridin-3-ylmethyl)-2-[(pyridin-3-ylmethyl)amino]acetamide;
2-(1,3-benzodioxole-5-yl)-N-(pyridin-4-ylmethyl)-2-[(pyridin-3-ylmethyl)amino]acetamide;
2-(1,3-benzodioxole-5-yl)-N-(pyridin-3-ylmethyl)-2-[(pyridin-4-ylmethyl)amino]acetamide;
2-(1,3-benzodioxole-5-yl)-N-(2-pyridin-3-ylethyl)-2-[(2-pyridin-3-ylethyl)amino]acetamide;
2-(1,3-benzodioxole-5-yl)-N-(2-pyridin-4-ylethyl)-2-[(2-pyridin-3-ylethyl)amino]acetamide;
2-(1,3-benzodioxole-5-yl)-N-(2-pyridin-3-ylethyl)-2-[(2-pyridin-4-ylethyl)amino]acetamide;
2-(2-methylphenyl)-N-(2-pyridin-4-ylethyl)-2-[(2-pyridin-4-ylethyl)amino]acetamide;
2-(2-methylphenyl)-N-(pyridin-4-ylmethyl)-2-[(pyridin-4-ylmethyl)amino] acetamide;
2-(2-methylphenyl)-2-[(2-piperidin-4-ylethyl)amino]-N-(2-pyridin-4-ylethyl)acetamide;
2-(2-methylphenyl)-2-[(2-piperidin-4-ylethyl)amino]-N-(pyridin-4-ylmethyl)acetamide;
2-(2-methylphenyl)-N-(pyridin-3-ylmethyl)-2-[(pyridin-3-ylmethyl)amino]acetamide;
2-(2-methylphenyl)-N-(pyridin-4-ylmethyl)-2-[(pyridin-3-ylmethyl)amino] acetamide;
2-(2-methylphenyl)-N-(pyridin-3-ylmethyl)-2-[(pyridin-4-ylmethyl)amino]acetamide;
2-(2-methylphenyl)-N-(2-pyridin-3-ylethyl)-2-[(2-pyridin-3-ylethyl)amino]acetamide;
2-(2-methylphenyl)-N-(2-pyridin-4-ylethyl)-2-[(2-pyridin-3-ylethyl)amino]acetamide;
2-(2-methylphenyl)-N-(2-pyridin-3-ylethyl)-2-[(2-pyridin-4-ylethyl)-amino]acetamide;
2-(3-methylphenyl)-N-(2-pyridin-4-ylethyl)-2-[(2-pyridin-4-ylethyl)amino]acetamide;
2-(3-methylphenyl)-N-(pyridin-4-ylmethyl)-2-[(pyridin-4-ylmethyl) amino]acetamide;
2-(3-methylphenyl)-2-[(2-piperidin-4-ylethyl)amino]-N-(2-pyridin-4-ylethyl)acetamide;
2-(3-methylphenyl)-2-[(2-piperidin-4-ylethyl)-amino]-N-(pyridin-4-ylmethyl)acetamide;
2-(3-methylphenyl)-N-(pyridin-3-ylmethyl)-2-[(pyridm-3-ylmethyl)amino]acetamide;
2-(3-methylphenyl)-N-(pyridin-4-ylmethyl)-2-[(pyridin-3-ylmethyl)amino]acetamide;
2-(3-methylphenyl)-N-(pyridin-3-ylmethyl)-2-[(pyridin-4-ylmethyl)amino]acetamide;
2-(3-methylphenyl)-N-(2-pyridin-3-ylethyl)-2-[(2-pyridin-3-ylethyl)amino]acetamide;
2-(3-methylphenyl)-N-(2-pyridin-4-ylethyl)-2-[(2-pyridin-3-ylethyl)amino]acetamide;
2-(3-methylphenyl)-N-(2-pyridin-3-ylethyl)-2-[(2-pyridin-4-ylethyl)amino]acetamide;
2-(4-methylphenyl)-N-(2-pyridin-4-ylethyl)-2-[(2-pyridin-4-ylethyl)amino]acetamide;
2-(4-methylphenyl)-N-(pyridin-4-ylmethyl)-2-[(pyridin-4-ylmethyl)amino]acetamide;
2-(4-methylphenyl)-2-[(2-piperidin-4-ylethyl)amino]-N-(2-pyridin-4-ylethyl)-acetamide;
2-(4-methylphenyl)-2-[(2-piperidin-4-ylethyl)amino]-N-(pyridin-4-ylmethyl)acetamide;
2-(4-methylphenyl)-N-(pyridin-3-ylmethyl)-2-[(pyridin-3-ylmethyl)amino]acetamide;
2-(4-methylphenyl)-N-(pyridin-4-ylmethyl)-2-[(pyridin-3-ylmethyl)amino]acetamide;
2-(4-methylphenyl)-N-(pyridin-3-ylmethyl)-2-[(pyridin-4-ylmethyl)amino]acetamide;
2-(4-methylphenyl)-N-(2-pyriclin-3-ylethyl)-2-[(2-pyridin-3-ylethyl)amino]acetamide;
2-(4-methylphenyl)-N-(2-pyridin-4-ylethyl)-2-[(2-pyridin-3-ylethyl)amino]acetamide;
2-(4-methylphenyl)-N-(2-pyridin-3-ylethyl)-2-[(2-pyridin-4-ylethyl)amino]acetamide;
2-(4-ethylphenyl)-N-(2-pyridin-4-ylethyl)-2-[(2-pyridin-4-ylethyl)amino]acetamide;
2-(4-ethylphenyl)-N-(pyridin-4-ylmethyl)-2-[(pyridin-4-ylmethyl)amino]acetamide;
2-(4-ethylphenyl)-2-[(2-piperidin-4-ylethyl)amino]-N-(2-pyridin-4-ylethyl)acetamide;
2-(4-ethylphenyl)-2-[(2-piperidin-4-ylethyl)amino]-N-(pyridin-4-ylmethyl)acetamide;
2-(4-ethylphenyl)-N-(pyridin-3-ylmethyl)-2-[(pyridin-3-ylmethyl)amino]acetamide;
2-(4-ethylphenyl)-N-(pyridin-4-ylmethyl)-2-[(pyridin-3-ylmethyl)amino]acetamide;
2-(4-ethylphenyl)-N-(pyridin-3-ylmethyl)-2-[(pyridin-4-ylmethyl)amino]acetamide;
2-(4-ethylphenyl)-N-(2-pyridin-3-ylethyl)-2-[(2-pyridin-3-ylethyl)amino]acetamide;
2-(4-ethylphenyl)-N-(2-pyridin-4-ylethyl)-2-[(2-pyridin-3-ylethyl)amino]acetamide;
2-(4-ethylphenyl)-N-(2-pyridin-3-ylethyl)-2-[(2-pyridin-4-ylethyl)amino]acetamide;
2-(4-isopropylphenyl)-N-(2-pyridin-4-ylethyl)-2-[(2-pyridin-4-ylethyl)amino]acetamide;
2-(4-isopropylphenyl)-N-(pyridin-4-ylmethyl)-2-[(pyridin-4-ylmethyl)amino]acetamide;
2-(4-isopropylphenyl)-2-[(2-piperidin-4-ylethyl)amino]-N-(2-pyridin-4-ylethyl)acetamide;
2-(4-isopropylphenyl)-2-[(2-piperidin-4-ylethyl)amino]-N-(pyridin-4-ylmethyl) acetamide;
2-(4-isopropylphenyl)-N-(pyridin-3-ylmethyl)-2-[(pyridin-3-ylmethyl)amino]acetamide;
2-(4-isopropylphenyl)-N-(pyridin-4-ylmethyl)-2-[(pyridin-3-ylmethyl)amino]acetamide;
2-(4-isopropylphenyl)-N-(pyridin-3-ylmethyl)-2-[(pyridin-4-ylmethyl)amino]acetamide;
2-(4-isopropylphenyl)-N-(2-pyridin-3-ylethyl)-2-[(2-pyridin-3-ylethyl)amino]acetamide;
2-(4-isopropylphenyl)-N-(2-pyridin-4-ylethyl)-2-[(2-pyridin-3-ylethyl)amino]acetamide;
2-(4-isopropylphenyl)-N-(2-pyridin-3-ylethyl)-2-[(2-pyridin-4-ylethyl)amino]acetamide;
2-(3,5-dimethylphenyl)-N-(2-pyridin-4-ylethyl)-2-[(2-pyridin-4-ylethyl)amino]acetamide;
2-(3,5-dimethylphenyl)-N-(pyridin-4-ylmethyl)-2-[(pyridin-4-ylmethyl)amino]acetamide;
2-(3,5-dimethylphenyl)-2-[(2-piperidin-4-ylethyl)amino]-N-(2-pyridin-4-ylethyl)acetamide;
2-(3,5-dimethylphenyl)-2-[(2-piperidin-4-ylethyl)amino]-N-(pyridin-4-ylmethyl)acetamide;
2-(3,5-dimethylphenyl)-N-(pyridin-3-ylmethyl)-2-[(pyridin-3-ylmethyl)amino]acetamide;
2-(3,5-dimethylphenyl)-N-(pyridin-4-ylmethyl)-2-[(pyridin-3-ylmethyl)amino]acetamide;
2-(3,5-dimethylphenyl)-N-(pyridin-3-ylmethyl)-2-[(pyridin-4-ylmethyl)amino]acetamide;
2-(3,5-dimethylphenyl)-N-(2-pyridin-3-ylethyl)-2-[(2-pyridin-3-ylethyl)amino]acetamide;
2-(3,5-dimethylphenyl)-N-(2-pyridin-4-ylethyl)-2-[(2-pyridin-3-ylethyl)amino]acetamide;
2-(3,5-dimethylphenyl)-N-(2-pyridin-3-ylethyl)-2-[(2-pyridin-4-ylethyl)amino]acetamide;
2-(2-naphthyl)-N-(2-pyridin-4-ylethyl)-2-[(2-pyridin-4-ylethyl)amino]acetamide;
2-(2-naphthyl)-N-(pyridin-4-ylmethyl)-2-[(pyridin-4-ylmethyl)amino]acetamide;
2-(2-naphthyl)-2-[(2-piperidin-4-ylethyl)amino]-N-(2-pyridin-4-ylethyl)acetamide;
2-(2-naphthyl)-2-[(2-piperidin-4-ylethyl)amino]-N-(pyridin-4-ylmethyl)acetamide;
2-(2-naphthyl)-N-(pyridin-3-ylmethyl)-2-[(pyridin-3-ylmethyl)amino]acetamide;
2-(2-naphthyl)-N-(pyridin-4-ylmethyl)-2-[(pyridin-3-ylmethyl)amino]acetamide;
2-(2-naphthyl)-N-(pyridin-3-ylmethyl)-2-[(pyridin-4-ylmethyl)amino]acetamide;
2-(2-naphthyl)-N-(2-pyridin-3-ylethyl)-2-[(2-pyridin-3-ylethyl)amino]acetamide;
2-(2-naphthyl)-N-(2-pyridin-4-ylethyl)-2-[(2-pyridin-3-ylethyl)amino]acetamide;
2-(2-naphthyl)-N-(2-pyridin-3-ylethyl)-2-[(2-pyridin-4-ylethyl)amino]acetamide;
N-(2-pyridin-4-ylethyl)-2-[(2-pyridin-4-ylethyl)amino]-2-(2,4,5-trimethylphenyl)acetamide;
N-(pyridin-4-ylmethyl)-2-[(pyridin-4-ylmethyl)amino]-2-(2,4,5-trimethylphenyl)acetamide;
2-[(2-piperidin-4-ylethyl)-amino]-N-(2-pyridin-4-ylethyl)-2-(2,4,5-trimethylphenyl)acetamide;
2-[(2-piperidin-4-ylethyl)amino]-N-(pyridin-4-ylmethyl)-2-(2,4,5-trimethylphenyl)acetamide;
N-(pyridin-3-ylmethyl)-2-[(pyridin-3-ylmethyl)amino]-2-(2,4,5-trimethylphenyl)acetamide;
N-(pyridin-4-ylmethyl)-2-[(pyridin-3-ylmethyl)amino]-2-(2,4,5-trimethylphenyl)acetamide;
N-(pyridin-3-ylmethyl)-2-[(pyridin-4-ylmethyl)amino]-2-(2,4,5-trimethylphenyl)acetamide;
N-(2-pyridin-3-ylethyl)-2-[(2-pyridin-3-ylethyl)amino]-2-(2,4,5-trimethylphenyl)acetamide;
N-(2-pyridin-4-ylethyl)-2-[(2-pyridin-3-ylethyl)amino]-2-(2,4,5-trimethylphenyl)acetamide;
N-(2-pyridin-3-ylethyl)-2-[(2-pyridin-4-ylethyl)amino]-2-(2,4,5-trimethylphenyl)acetamide;
2-(4-tert-butylphenyl)-N-(2-pyridin-4-ylethyl)-2-[(2-pyridin-4-ylethyl)amino]acetamide;
2-(4-tert-butylphenyl)-N-(pyridin-4-ylmethyl)-2-[(pyridin-4-ylmethyl)amino]acetamide;
2-(4-tert-butylphenyl)-2-[(2-piperidin-4-ylethyl)amino]-N-(2-pyridin-4-ylethyl)acetamide;
2-(4-tert-butylphenyl)-2-[(2-piperidin-4-ylethyl)amino]-N-(pyridin-4-ylmethyl)acetamide;
2-(4-tert-butylphenyl)-N-(pyridin-3-ylmethyl)-2-[(pyridin-3-ylmethyl)amino]acetamide;
2-(4-tert-butylphenyl)-N-(pyridin-4-ylmethyl)-2-[(pyridin-3-ylmethyl)amino]acetamide;
2-(4-tert-butylphenyl)-N-(pyridin-3-ylmethyl)-2-[(pyridin-4-ylmethyl)amino]acetamide;
2-(4-tert-butylphenyl)-N-(2-pyridin-3-ylethyl)-2-[(2-pyridin-3-ylethyl)amino]acetamide;
2-(4-tert-butylphenyl)-N-(2-pyridin-4-ylethyl)-2-[(2-pyridin-3-ylethyl)amino]acetamide;
2-(4-tert-butylphenyl)-N-(2-pyridin-3-ylethyl)-2-[(2-pyridin-4-ylethyl)amino]acetamide;
2-[4-(dimethylamino)phenyl]-N-(2-pyridin-4-ylethyl)-2-[(2-pyridin-4-ylethyl)amino]acetamide;
2-[4-(dimethylamino)phenyl]-N-(pyridin-4-ylmethyl)-2-[(pyridin-4-ylmethyl)amino]acetamide;
2-[4-(dimethylamino)phenyl]-2-[(2-piperidin-4-ylethyl)amino]-N-(2-pyridin-4-ylethyl)acetamide;
2-[4-(dimethylamino)phenyl]-2-[(2-piperidin-4-ylethyl)amino]-N-(pyridin-4-ylmethyl)acetamide;
2-[4-(dimethylamino)phenyl]-N-(pyridin-3-ylmethyl)-2-[(pyridin-3-ylmethyl)amino]acetamide;
2-[4-(dimethylamino)phenyl]-N-(pyridin-4-ylmethyl)-2-[(pyridin-3-ylmethyl)amino]acetamide;
2-[4-(dimethylamino)phenyl]-N-(pyridin-3-ylmethyl)-2-[(pyridin-4-ylmethyl)amino]acetamide;
2-[4-(dimethylamino)phenyl]-N-(2-pyridin-3-ylethyl)-2-[(2-pyridin-3-ylethyl)amino]acetamide;
2-[4-(dimethylamino)phenyl]-N-(2-pyridin-4-ylethyl)-2-[(2-pyridin-3-ylethyl)amino]acetamide;
2-[4-(dimethylamino)phenyl]-N-(2-pyridin-3-ylethyl)-2-[(2-pyridin-4-ylethyl)amino]acetamide;
2-(4-propoxyphenyl)-N-(2-pyridin-4-ylethyl)-2-[(2-pyridin-4-ylethyl)amino]acetamide;
2-(4-propoxyphenyl)-N-(pyridin-4-ylmethyl)-2-[(pyridin-4-ylmethyl)amino]acetamide;
2-[(2-piperidin-4-ylethyl)amino]-2-(4-propoxyphenyl)-N-(2-pyridin-4-ylethyl)acetamide;
2-[(2-piperidin-4-ylethyl)amino]-2-(4-propoxyphenyl)-N-(pyridin-4-ylmethyl)acetamide;
2-(4-propoxyphenyl)-N-(pyridin-3-ylmethyl)-2-[(pyridin-3-ylmethyl)amino]acetamide;
2-(4-propoxyphenyl)-N-(pyridin-4-ylmethyl)-2-[(pyridin-3-ylmethyl)amino]acetamide;
2-(4-propoxyphenyl)-N-(pyridin-3-ylmethyl)-2-[(pyridin-4-ylmethyl)amino]acetamide;
2-(4-propoxyphenyl)-N-(2-pyridin-3-ylethyl)-2-[(2-pyridin-3-ylethyl)amino]acetamide;
2-(4-propoxyphenyl)-N-(2-pyridin-4-ylethyl)-2-[(2-pyridin-3-ylethyl)amino]acetamide;
2-(4-propoxyphenyl)-N-(2-pyridin-3-ylethyl)-2-[(2-pyridin-4-ylethyl)amino]acetamide;
2-(4-butoxyphenyl)-N-(2-pyridin-4-ylethyl)-2-[(2-pyridin-4-ylethyl)amino]acetamide;
2-(4-butoxyphenyl)-N-(pyridin-4-ylmethyl)-2-[(pyridin-4-ylmethyl)amino]acetamide;
2-(4-butoxyphenyl)-2-[(2-piperidin-4-ylethyl)amino]-N-(2-pyridin-4-ylethyl)acetamide;
2-(4-butoxyphenyl)-2-[(2-piperidin-4-ylethyl)amino]-N-(pyridin-4-ylmethyl)acetamide;
2-(4-butoxyphenyl)-N-(pyridin-3-ylmethyl)-2-[(pyridin-3-ylmethyl)amino]acetamide;
2-(4-butoxyphenyl)-N-(pyridin-4-ylmethyl)-2-[(pyridin-3-ylmethyl)amino]acetamide;
2-(4-butoxyphenyl)-N-(pyridin-3-ylmethyl)-2-[(pyridin-4-ylmethyl)amino]acetamide;
2-(4-butoxyphenyl)-N-(2-pyridin-3-ylethyl)-2-[(2-pyridin-3-ylethyl)amino]acetamide;
2-(4-butoxyphenyl)-N-(2-pyridin-4-ylethyl)-2-[(2-pyridin-3-ylethyl)amino]acetamide;
2-(4-butoxyphenyl)-N-(2-pyriclin-3-ylethyl)-2-[(2-pyridin-4-ylethyl)amino]acetamide;
2-(2,6-dichlorophenyl)-N-(2-pyridin-4-ylethyl)-2-[(2-pyridin-4-ylethyl)amino]acetamide;
2-(2,6-dichlorophenyl)-N-(pyridin-4-ylmethyl)-2-[(pyridin-4-ylmethyl)amino]acetamide;
2-(2,6-dichlorophenyl)-2-[(2-piperidin-4-ylethyl)amino]-N-(2-pyridin-4-ylethyl)acetamide;
2-(2,6-dichlorophenyl)-2-[(2-piperidin-4-ylethyl)amino]-N-(pyridin-4-ylmethyl)acetamide;
2-(2,6-dichlorophenyl)-N-(pyridin-3-ylmethyl)-2-[(pyridin-3-ylmethyl)amino]acetamide;
2-(2,6-dichlorophenyl)-N-(pyridin-4-ylmethyl)-2-[(pyridin-3-ylmethyl)amino]acetamide;
2-(2,6-dichlorophenyl)-N-(pyridin-3-ylmethyl)-2-[(pyridin-4-ylmethyl)amino]acetamide;
2-(2,6-dichlorophenyl)-N-(2-pyridin-3-ylethyl)-2-[(2-pyridin-3-ylethyl)amino]acetamide;
2-(2,6-dichlorophenyl)-N-(2-pyridin-4-ylethyl)-2-[(2-pyridin-3-ylethyl)amino]acetamide;
2-(2,6-dichlorophenyl)-N-(2-pyridin-3-ylethyl)-2-[(2-pyridin-4-ylethyl)amino]acetamide;
2-(2-chloro-6-fluorophenyl)-N-(2-pyridin-4-ylethyl)-2-[(2-pyridin-4-ylethyl)amino]acetamide;
2-(2-chloro-6-fluorophenyl)-N-(pyridin-4-ylmethyl)-2-[(pyridin-4-ylmethyl)amino]acetamide;
2-(2-chloro-6-fluorophenyl)-2-[(2-piperidin-4-ylethyl)amino]-N-(2-pyridin-4-ylethyl)acetamide;
2-(2-chloro-6-fluorophenyl)-2-[(2-piperidin-4-ylethyl)amino]-N-(pyridin-4-ylmethyl)acetamide;
2-(2-chloro-6-fluorophenyl)-N-(pyridin-3-ylmethyl)-2-[(pyridin-3-ylmethyl)amino]acetamide;
2-(2-chloro-6-fluorophenyl)-N-(pyridin-4-ylmethyl)-2-[(pyridin-3-ylmethyl)amino]acetamide;
2-(2-chloro-6-fluorophenyl)-N-(pyridin-3-ylmethyl)-2-[(pyridin-4-ylmethyl)amino]acetamide;
2-(2-chloro-6-fluorophenyl)-N-(2-pyridin-3-ylethyl)-2-[(2-pyridin-3-ylethyl)amino]acetamide;
2-(2-chloro-6-fluorophenyl)-N-(2-pyridin-4-ylethyl)-2-[(2-pyridin-3-ylethyl)amino]acetamide;
2-(2-chloro-6-fluorophenyl)-N-(2-pyridin-3-ylethyl)-2-[(2-pyridin-4-ylethyl)amino]acetamide;
2-(2,3-dimethylphenyl)-N-(2-pyridin-4-ylethyl)-2-[(2-pyridin-4-ylethyl)amino]acetamide;
2-(2,3-dimethylphenyl)-N-(pyridin-4-ylmethyl)-2-[(pyridin-4-ylmethyl)amino]acetamide;
2-(2,3-dimethylphenyl)-2-[(2-piperidin-4-ylethyl)amino]-N-(2-pyridin-4-ylethyl)acetamide;
2-(2,3-dimethylphenyl)-2-[(2-piperidin-4-ylethyl)amino]-N-(pyridin-4-ylmethyl)acetamide;
2-(2,3-dimethylphenyl)-N-(pyridin-3-ylmethyl)-2-[(pyridin-3-ylmethyl)amino]acetamide;
2-(2,3-dimethylphenyl)-N-(pyridin-4-ylmethyl)-2-[(pyridin-3-ylmethyl)amino]acetamide;
2-(2,3-dimethylphenyl)-N-(pyridin-3-ylmethyl)-2-[(pyridin-4-ylmethyl)amino]acetamide;
2-(2,3-dimethylphenyl)-N-(2-pyridin-3-ylethyl)-2-[(2-pyridin-3-ylethyl)amino]acetamide;
2-(2,3-dimethylphenyl)-N-(2-pyridin-4-ylethyl)-2-[(2-pyridin-3-ylethyl)-amino]acetamide;
2-(2,3-dimethylphenyl)-N-(2-pyridin-3-ylethyl)-2-[(2-pyridin-4-ylethyl)amino]acetamide;
2-(4-hydroxy-3-methoxyphenyl)-N-(2-pyridin-4-ylethyl)-2-[(2-pyridin-4-ylethyl)amino]acetamide;
2-(4-hydroxy-3-methoxyphenyl)-N-(pyridin-4-ylmethyl)-2-[(pyridin-4-ylmethyl)amino]acetamide;
2-(4-hydroxy-3-methoxyphenyl)-2-[(2-piperidin-4-ylethyl)amino]-N-(2-pyridin-4ylmethyl) acetamide;
2-(4-hydroxy-3-methoxyphenyl)-2-[(2-piperidin-4-ylethyl)amino]-N-(pyridin-4-ylmethyl) acetamide;
2-(4-hydroxy-3-methoxyphenyl)-N-(pyridin-3-ylmethyl)-2-[(pyridin-3-ylmethyl)amino]acetamide;
2-(4-hydroxy-3-methoxyphenyl)-N-(pyridin-4-ylmethyl)-2-[(pyridin-3-ylmethyl)amino]acetamide;
2-(4-hydroxy-3-methoxyphenyl)-N-(pyridin-3-ylmethyl)-2-[(pyridin-4-ylmethyl)amino]acetamide;
2-(4-hydroxy-3-methoxyphenyl)-N-(2-pyridin-3-ylethyl)-2-[(2-pyridin-3-ylethyl)amino]acetamide;
2-(4-hydroxy-3-methoxyphenyl)-N-(2-pyridin-4-ylethyl)-2-[(2-pyridin-3-ylethyl)amino]acetamide;
2-(4-hydroxy-3-methoxyphenyl)-N-(2-pyridin-3-ylethyl)-2-[(2-pyridin-4-ylethyl)amino]acetamide;
N-(2-pyridin-4-ylethyl)-2-[(2-pyridin-4-ylethyl)amino]-2-(2-thienyl)acetamide;
N-(pyridin-4-ylmethyl)-2-[(pyridin-4-ylmethyl)amino]-2-(2-thienyl)acetamide;
2-[(2-piperidin-4-ylethyl)amino]-N-(2-pyridin-4-ylethyl)-2-(2-thienyl)acetamide;
2-[(2-piperidin-4-ylethyl)amino]-N-(pyridin-4-ylmethyl)-2-(2-hienyl)acetamide;
N-(pyridin-3-ylmethyl)-2-[(pyridin-3-ylmethyl)amino]-2-(2-thienyl)acetamide;
N-(pyridin-4-ylmethyl)-2-[(pyridin-3-ylmethyl)amino]-2-(2-thienyl)acetamide;
N-(pyridin-3-ylmethyl)-2-[(pyridin-4-ylmethyl)amino]-2-(2-thienyl)acetamide;
N-(2-pyridin-3-ylethyl)-2-[(2-pyridin-3-ylethyl)amino]-2-(2-thienyl acetamide;
N-(2-pyridin-4-ylethyl)-2-[(2-pyridin-3-ylethyl)amino]-2-(2-thienyl)acetamide;
N-(2-pyridin-3-ylethyl)-2-[(2-pyridin-4-ylethyl)amino]-2-(2-thienyl)acetamide;
2-(5-methyl-2-thienyl)-N-(2-pyridin-4-ylethyl)-2-[(2-pyridin-4-ylethyl)amino]acetamide;
2-(5-methyl-2-thienyl)-N-(pyridin-4-ylmethyl)-2-[(pyridin-4-ylmethyl)amino]acetamide;
2-(5-methyl-2- thienyl)-2-[(2-piperidin-4-ylethyl)amino]-N-(2-pyridin-4-ylethyl)acetamide;
2-(5-methyl-2-thienyl)-2-[(2-piperidin-4-ylethyl)amino]-N-(pyridin-4-ylmethyl)acetamide;
2-(5-methyl-2-thienyl)-N-(pyridin-3-ylmethyl)-2-[(pyridin-3-ylmethyl)amino]acetamide;
2-(5-methyl-2-thienyl)-N-(pyridin-4-ylmethyl)-2-[(pyridin-3-ylmethyl)amino]acetamide;
2-(5-methyl-2-thienyl)-N-(pyridin-3-ylmethyl)-2-[(pyridin-4-ylmethyl)amino]acetamide;
2-(5-methyl-2-thienyl)-N-(2-pyridin-3-ylethyl)-2-[(2-pyridin-3-ylethyl)amino]acetamide;
2-(5-methyl-2- thienyl)-N-(2-pyridin-4-ylethyl)-2-[(2-pyridin-3-ylethyl)amino]acetamide;
2-(5-methyl-2-thienyl)-N-(2-pyridin-3-ylethyl)-2-[(2-pyridin-4-ylethyl)amino]acetamide;
2-(3-methyl-2-thienyl)-N-(2-pyridin-4-ylethyl)-2-[(2-pyridin-4-ylethyl)amino]acetamide;
2-(3-methyl-2-thienyl)-N-(pyridin-4-ylmethyl)-2-[(pyridin-4-ylmethyl)amino]acetamide;
2-(3-methyl-2- thienyl)-2-[(2-piperidin-4-ylethyl)amino]-N-(2-pyridin-4-ylethyl)acetamide;
2-(3-methyl-2-thienyl)-2-[(2-piperidin-4-ylethyl)amino]-N-(pyridin-4-ylmethyl)acetamide;
2-(3-methyl-2-thienyl)-N-(pyridin-3-ylmethyl)-2-[(pyridin-3-ylmethyl)amino]acetamide;
2-(3-methyl-2-thienyl)-N-(pyridin-4-ylmethyl)-2-[(pyridin-3-ylmethyl)amino]acetamide;
2-(3-methyl-2-thienyl)-N-(pyridin-3-ylmethyl)-2-[(pyridin-4-ylmethyl)amino]acetamide;
2-(3-methyl-2-thienyl)-N-(2-pyridin-3-ylethyl)-2-[(2-pyridin-3-ylethyl)amino]acetamide;
2-(3-methyl-2-thienyl)-N-(2-pyridin-4-ylethyl)-2-[(2-pyridin-3-ylethyl)amino]acetamide;
2-(3-methyl-2-thienyl)-N-(2-pyridin-3-ylethyl)-2-[(2-pyridin-4-ylethyl)amino]acetamide;
N-(2-pyridin-4-ylethyl)-2-[(2-pyridin-4-ylethyl)amino]-2-(3-hienyl)acetamide;
N-(pyridin-4-ylmethyl)-2-[(pyridin-4-ylmethyl)amino]-2-(3-thienyl)acetamide;
2-[(2-piperidin-4-ylethyl)amino]-N-(2-pyridin-4-ylethyl)-2-(3-thienyl)acetamide;
2-[(2piperidin-4-ylethyl)amino]-N-(pyridin-4-ylmethyl)-2-(3-thienyl)acetamide;
N-(pyridin-3-ylmethyl)-2-[(pyridin-3-ylmethyl)amino]-2-(3-thienyl)acetamide;
N-(pyridin-4-ylmethyl)-2-[(pyridin-3-ylmethyl)amino]-2-(3-thienyl)acetamide;
N-(pyridin-3-ylmethyl)-2-[(pyridin-4-ylmethyl)amino]-2-(3-thienyl)acetamide;
N-(2-pyridin-3-ylethyl)-2-[(2-pyridin-3-ylethyl)amino]-2-(3-thienyl)acetamide;
N-(2-pyridin-4-ylethyl)-2-[(2-pyridin-3-ylethyl)amino]-2-(3-thienyl)acetamide;
N-(2-pyridin-3-ylethyl)-2-[(2-pyridin-4-ylethyl)amino]-2-(3-thienyl)acetamide;
2-pyridin -4-yl-N-(2-pyridin-4-ylethyl)-2-[(2-pyridin-4-ylethyl) amino]acetamide;
2-pyridin-4-yl-N-(pyridin-4-ylmethyl)-2-[(pyridin-4-ylmethyl) amino]acetamide;
2-[(2-piperidin-4-ylethyl)amino]-2-pyridin-4-yl-N-(2-pyridin-4-ylethyl)acetamide;
2-[(2-piperidin-4-ylethyl)amino]-2-pyridin-4-yl-N-(pyridin-4-ylmethyl)acetamide;
2-pyridin -4-yl-N-(pyridin-3-ylmethyl)-2-[(pyridin-3-ylmethyl) amino]acetamide;
2-pyridin -4-yl-N-(pyridin-4-ylmethyl)-2-[(pyridin-3-ylmethyl) amino]acetamide;
2-pyridin -4-yl-N-(pyridin-3-ylmethyl)-2-[(pyridin-4-ylmethyl) amino]acetamide;
2-pyridin -4-yl-N-(2-pyridin-3-ylethyl)-2-[(2-pyridin-3-ylethyl) amino]acetamide;
2-pyridin -4-yl-N-(2-pyridin-4-ylethyl)-2-[(2-pyridin-3-ylethyl) amino]acetamide;
2-pyridin -4-yl -N-(2-pyridin-3-ylethyl)-2-[(2-pyridin-4-ylethyl) amino]acetamide;
2-pyridin-2-yl -N-(2-pyridin-4-ylethyl)-2-[(2-pyridin-4-ylethyl) amino] acetamide;
2-pyridin-2-yl -N-(pyridin-4-ylmethyl)-2-[(pyridin-4-ylmethyl) amino]acetamide;
2-[(2-piperidin-4-ylethyl)amino]-2-pyridin-2-yl-N-(2-pyridin-4-ylethyl)acetamide;
2-[(2-piperidin-4-ylethyl)amino]-2-pyridin-2-yl-N-(pyridin-4-ylmethyl)acetamide;
2-pyridin-2-yl -N-(pyridin-3-ylmethyl)-2-[(pyridin-3-ylmethyl) amino]acetamide;
2-pyridin-2-yl -N-(pyridin-4-ylmethyl)-2-[(pyridin-3-ylmethyl) amino]acetamide;
2-pyridin-2-yl -N-(pyridin-3-ylmethyl)-2-[(pyridin-4-ylmethyl) amino]acetamide;
2-pyridin-2-yl -N-(2-pyridin-3-ylethyl)-2-[(2-pyridin-3-ylethyl) amino]acetamide;
2-pyridin-2-yl -N-(2-pyridin-4-ylethyl)-2-[(2-pyridin-3-ylethyl) amino]acetamide;
2-pyridin-2-yl -N-(2-pyridin-3-ylethyl)-2-[(2-pyridin-4-ylethyl) amino]acetamide;
N-(2-pyridin-4-ylethyl)-2-[(2-pyridin-4-ylethyl)amino]-2-quinoline-2-ylacetamide;
N-(pyridin-4-ylmethyl)-2-[(pyridin-4-ylmethyl) amino]-2-quinoline-2-ylacetamide;
2-[(2-piperidin-4-ylethyl)amino]-N-(2-pyridin-4-ylethyl)-2-quinoline-2-ylacetamide;
2-[(2-piperidin-4-ylethyl)amino]-N-(pyridin-4-ylmethyl)-2-quinoline-2-ylacetamide;
N-(pyridin-3-ylmethyl)-2-[(pyridin-3-ylmethyl) amino]-2-quinoline-2-ylacetamide;
N-(pyridin-4-ylmethyl)-2-[(pyridin-3-ylmethyl) amino]-2-quinoline-2-ylacetamide;
N-(pyridin-3-ylmethyl)-2-[(pyridin-4-ylmethyl) amino]-2-quinoline-2-ylacetamide;
N-(2-pyridin-3-ylethyl)-2-[(2-pyridin-3-ylethyl) amino]-2-quinoline-2-ylacetamide;
N-(2-pyridin-4-ylethyl)-2-[(2-pyridin-3-ylethyl) amino]-2-quinoline-2-ylacetamide;
N-(2-pyridin-3-ylethyl)-2-[(2-pyridin-4-ylethyl)amino]-2-quinoline-2-ylacetamide;
2-(2,3-dimethoxyphenyl)-N-(2-pyridin-4-ylethyl)-2-[(2-pyridin-4-ylethyl) amino]acetamide;
2-(2,3-dimethoxyphenyl)-N-(pyridin-4-ylmethyl)-2-[(pyridin-4-ylmethyl) amino]acetamide;
2-(2,3-dimethoxyphenyl)-2-[(2-piperidin-4-ylethyl)amino]-N-(2-pyridin-4-ylethyl)acetamide;
2-(2,3-dimethoxyphenyl)-2-[(2-piperidin-4-ylethyl)amino]-N-(pyridin-4-ylmethyl)acetamide;
2-(2,3-dimethoxyphenyl)-N-(pyridin-3-ylmethyl)-2-[(pyridin-3-ylmethyl)amino]acetamide;
2-(2,3-dimethoxyphenyl)-N-(pyridin-4-ylmethyl)-2-[(pyridin-3-ylmethyl)amino]acetamide;
2-(2,3-dimethoxyphenyl)-N-(pyridin-3-ylmethyl)-2-[(pyridin-4-ylmethyl)amino]acetamide;
2-(2,3-dimethoxyphenyl)-N-(2-pyridin-3-ylethyl)-2-[(2-pyridin-3-ylethyl)amino]acetamide;
2-(2,3-dimethoxyphenyl)-N-(2-pyridin-4-ylethyl)-2-[(2-pyridin-3-ylethyl)amino]acetamide;
2-(2,3- dimethoxyphenyl)-N-(2-pyridin-3-ylethyl)-2-[(2-pyridin-4-ylethyl)amino]acetamide;
2-(4-nitrophenyl)-N-(2-pyridin-4-ylethyl)-2-[(2-pyridin-4-ylethyl) amino]acetamide;
2-(4- nitrophenyl)-N-(pyridin-4-ylmethyl)-2-[(pyridin-4-ylmethyl) amino] acetamide;
2-(4-nitrophenyl)-2-[(2-piperidin-4-ylethyl)amino]-N-(2-pyridin-4-ylethyl)acetamide;
2-(4- nitrophenyl)-2-[(2-piperidin-4-ylethyl)amino]-N-(pyridin-4-ylmethyl)acetamide;
2-(4-nitrophenyl)-N-(pyridin-3-ylmethyl)-2-[(pyridin-3-ylmethyl)amino]acetamide;
2-(4-nitrophenyl)-N-(pyridin-4-ylmethyl)-2-[(pyridin-3-ylmethyl)amino]acetamide;
2-(4-nitrophenyl)-N-(pyridin-3-ylmethyl)-2-[(pyridin-4-ylmethyl)amino]acetamide;
2-(4-nitrophenyl)-N-(2-pyridin-3-ylethyl)-2-[(2-pyridin-3-ylethyl)amino]acetamide;
2-(4-nitrophenyl)-N-(2-pyridin-4-ylethyl)-2-[(2-pyridin-3-ylethyl)amino]acetamide;
2-(4-nitrophenyl)-N-(2-pyridin-3-ylethyl)-2-[(2-pyridin-4-ylethyl)amino]acetamide;
2-(2- nitrophenyl)-N-(2-pyridin-4-ylethyl)-2-[(2-pyridin-4-ylethyl)amino]acetamide;
2-(2-nitrophenyl)-N-(pyridin-4-ylmethyl)-2-[(pyridin-4-ylmethyl) amino]acetamide;
2-(2-nitrophenyl)-2-[(2-piperidin-4-ylethyl)amino]-N-(2-pyridin-4-ylethyl) acetamide;
2-(2-nitrophenyl)-2-[(2-piperidin-4-ylethyl)amino]-N-(pyridin-4-ylmethyl) acetamide;
2-(2-nitrophenyl)-N-(pyridin-3-ylmethyl)-2-[(pyridin-3-ylmethyl)amino]acetamide;
2-(2-nitrophenyl)-N-(pyridin-4-ylmethyl)-2-[(pyridin-3-ylmethyl)amino]acetamide;
2-(2-nitrophenyl)-N-(pyriclin-3-ylmethyl)-2-[(pyriclin-4-ylmethyl)amino]acetamide;
2-(2-nitrophenyl)-N-(2-pyridin-3-ylethyl)-2-[(2-pyridin-3-ylethyl)amino]acetamide;
2-(2-nitrophenyl)-N-(2-pyridin-4-ylethyl)-2-[(2-pyridin-3-ylethyl)amino]acetamide;
2-(2-nitrophenyl)-N-(2-pyridin-3-ylethyl)-2-[(2-pyridin-4-ylethyl)amino]acetamide;
2-(3-nitrophenyl)-N-(2-pyridin-4-ylethyl)-2-[(2-pyridin-4-ylethyl)amino]acetamide;
2-(3-nitrophenyl)-N-(pyridin-4-ylmethyl)-2-[(pyridin-4-ylmethyl)amino]acetamide;
2-(3-nitrophenyl)-2-[(2-piperidin-4-ylethyl)amino]-N-(2-pyridin-4-ylethyl)acetamide;
2-(3-nitrophenyl)-2-[(2-piperidin-4-ylethyl)amino]-N-(pyridin-4-ylmethyl) acetamide;
2-(3-nitrophenyl)-N-(pyridin-3-ylmethyl)-2-[(pyridin-3-ylmethyl)amino]acetamide;
2-(3-nitrophenyl)-N-(pyridin-4-ylmethyl)-2-[(pyridin-3-ylmethyl)amino]acetamide;
2-(3-nitrophenyl)-N-(pyridin-3-ylmethyl)-2-[(pyridin-4-ylmethyl)amino]acetamide;
2-(3-nitrophenyl)-N-(2-pyridin-3-ylethyl)-2-[(2-pyridin-3-ylethyl)amino]acetamide;
2-(3-nitrophenyl)-N-(2-pyridin-4-ylethyl)-2-[(2-pyridin-3-ylethyl)amino]acetamide;
2-(3-nitrophenyl)-N-(2-pyridin-3-ylethyl)-2-[(2-pyridin-4-ylethyl)amino]acetamide;
2-pyridin-3-yl-N-(2-pyridin-4-ylethyl)-2-[(2-pyridin-4-ylethyl)amino]acetamide;
2-pyridin-3-ylN-(pyridin-4-ylmethyl)-2-[(pyridin-4-ylmethyl)amino]acetamide;
2-[(2-piperidin-4-ylethyl)amino]-2-pyridin-3-yl-N-(2-pyridin-4-ylethyl)acetamide;
2-[(2-piperidin-4-ylethyl)amino]-2-pyridin-3-yl -N-(pyridin-4-ylmethyl)acetamide;
2-pyridin-3-yl-N-(pyridin-3-ylmethyl)-2-[(pyridin-3-ylmethyl)amino] acetamide;
2-pyridin-3-yl-N-(pyridin-4-ylmethyl)-2-[(pyridin-3-ylmethyl)amino]acetamide;
2-pyridin-3-yl-N-(pyridin-3-ylmethyl)-2-[(pyridin-4-ylmethyl)amino]acetamide;
2-pyridin-3-yl-N-(2-pyridin-3-ylethyl)-2-[(2-pyridin-3-ylethyl)amino]acetamide;
2-pyridin-3-yl-N-(2-pyridin-4-ylethyl)-2-[(2-pyridin-3-ylethyl)amino]acetamide;
2-pyridin-3-yl-N-(2-pyridin-3-ylethyl)-2-[(2-pyridin-4-ylethyl)amino]acetamide;
2-(2,3-dichlorophenyl)-N-(2-pyridin-4-ylethyl)-2-[(2-pyridin-4-ylethyl)amino]acetamide;
2-(2,3-dichlorophenyl)-N-(pyridin-4-ylmethyl)-2-[(pyridin-4-ylmethyl)amino]acetamide;
2-(2,3-dichlorophenyl)-2-[(2-piperidin-4-ylethyl)amino]-N-(2-pyridin-4-ylethyl)acetamide;
2-(2,3-dichlorophenyl)-2-[(2-piperidin-4-ylethyl)amino]-N-(pyridin-4-ylmethyl)acetamide;
2-(2,3-dichlorophenyl)-N-(pyridin-3-ylmethyl)-2-[(pyridin-3-ylmethyl)amino]acetamide;
2-(2,3-dichlorophenyl)-N-(pyridin-4-ylmethyl)-2-[(pyridin-3-ylmethyl)amino]acetamide;
2-(2,3-dichlorophenyl)-N-(pyridin-3-ylmethyl)-2-[(pyridin-4-ylmethyl)amino]acetamide;
2-(2,3-dichlorophenyl)-N-(2-pyridin-3-ylethyl)-2-[(2-pyridin-3-ylethyl)amino]acetamide;
2-(2,3-dichlorophenyl)-N-(2-pyridin-4-ylethyl)-2-[(2-pyridin-3-ylethyl)amino]acetamide;
2-(2,3-dichlorophenyl)-N-(2-pyridin-3-ylethyl)-2-[(2-pyridin-4-ylethyl)amino]acetamide;
2-phenyl-N-(2-pyridin-4-ylethyl)-2-[(2-pyridin-4-ylethyl) amino]acetamide;
2-phenyl-N-(pyridin-4-ylmethyl)-2-[(pyridin-4-ylmethyl) amino]acetamide;
2-phenyl-2-[(2-piperidin-4-ylethyl)amino]-N-(2-pyridin-4-ylethyl)acetamide;
2-phenyl-2-[(2-piperidin-4-ylethyl)amino]-N-(pyridin-4-ylmethyl)acetamide;
2-phenyl-N-(pyridin-3-ylethyl)-2-[(pyridin-3-ylethyl)amino]acetamide;
2-phenyl-N-(pyridin-4-ylmethyl)-2-[(pyridin-3-ylmethyl)amino]acetamide;
2-phenyl-N-(pyridin-3-ylmethyl)-2-[(pyridin-4-ylmethyl)amino]acetamide;
2-phenyl-N-(2-pyridin-3-ylethyl)-2-[(2-pyridin-3-ylethyl)amino]acetamide;
2-phenyl-N-(2-pyridin-4-ylethyl)-2-[(2-pyridin-3-ylethyl)amino]acetamide;
2-phenyl-N-(2-pyridin-3-ylethyl)-2-[(2-pyridin-4-ylethyl)amino]acetamide;
2-(4-hydroxyphenyl)-N-(2-pyridin-4-ylethyl)-2-[(2-pyridin-4-ylethyl)amino]acetamide;
2-(4-hydroxyphenyl)-N-(pyridin-4-ylmethyl)-2-[(pyridin-4-ylmethyl) amino]acetamide;
2-(4-hydroxyphenyl)-2-[(2-piperidin-4-ylethyl)amino]-N-(2-pyridin-4-ylethyl)acetamide;
2-(4-hydroxyphenyl)-2-[(2-piperidin-4-ylethyl)amino]-N-(pyridin-4-ylmethyl)acetamide;
2-(4-hydroxyphenyl)-2-[(2-piperidin-4-ylethyl)amino]-N-(pyridin-4-ylmethyl)acetamide;
2-(4-hydroxyphenyl)-N-(pyridin-4-ylmethyl)-2-[(pyridin-3-ylmethyl)amino]acetamide;
2-(4-hydroxyphenyl)-N-(pyridin-3-ylmethyl)-2-[(pyridin-4-ylmethyl)amino]acetamide;
2-(4-hydroxyphenyl)-N-(2-pyridin-3-ylethyl)-2-[(2-pyridin-3-ylethyl)amino]acetamide;
2-(4-hydroxyphenyl)-N-(2-pyridin-4-ylethyl)-2-[(2-pyridin-3-ylethyl)amino]acetamide;
2-(4-hydroxyphenyl)-N-(2-pyridin-3-ylethyl)-2-[(2-pyridin-4-ylethyl)amino]acetamide;
2-(3-hydroxyphenyl)-N-(2-pyridin-4-ylethyl)-2-[(2-pyridin-4-ylethyl)amino]acetamide;
2-(3-hydroxyphenyl)-N-(pyridin-4-ylmethyl)-2-[(pyridin-4-ylmethyl)amino]acetamide;
2-(3-hydroxyphenyl)-2-[(2-piperidin-4-ylethyl)amino]-N-(2-pyridin-4-ylethyl)acetamide;
2-(3-hydroxyphenyl)-2-[(2-piperidin-4-ylethyl)amino]-N-(pyridin-4-ylmethyl)acetamide;
2-(3-hydroxyphenyl)-N-(pyridin-3-ylmethyl)-2-[(pyridin-3-ylmethyl)amino]acetamide;
2-(3-hydroxyphenyl)-N-(pyridin-4-ylmethyl)-2-[(pyridin-3-ylmethyl)amino]acetamide;
2-(3-hydroxyphenyl)-N-(pyridin-3-ylmethyl)-2-[(pyridin-4-ylmethyl)amino]acetamide;
2-(3-hydroxyphenyl)-N-(2-pyridin-3-ylethyl)-2-[(2-pyridin-3-ylethyl)amino]acetamide;
2-(3-hydroxyphenyl)-N-(2-pyridin-4-ylethyl)-2-[(2-pyridin-3-ylethyl)amino]acetamide;
2-(3-hydroxyphenyl)-N-(2-pyridin-3-ylethyl)-2-[(2-pyridin-4-ylethyl)amino]acetamide;
2-(2-hydroxyphenyl)-N-(2-pyridin-4-ylethyl)-2-[(2-pyridin-4-ylethyl)amino]acetamide;
2-(2-hydroxyphenyl)-N-(pyridin-4-ylmethyl)-2-[(pyridin-4-ylmethyl)amino]acetamide;
2-(2-hydroxyphenyl)-2-[(2-piperidin-4-ylethyl)amino]-N-(2-pyridin-4-ylethyl)acetamide;
2-(2-hydroxyphenyl)-2-[(2-piperidin-4-ylethyl)amino]-N-(pyridin-4-ylmethyl)acetamide;
2-(2-hydroxyphenyl)-N-(pyridin-3-ylmethyl)-2-[(pyridin-3-ylmethyl)amino]acetamide;
2-(2-hydroxyphenyl)-N-(pyridin-4-ylmethyl)-2-[(pyridin-3-ylmethyl)amino]acetamide;
2-(2-hydroxyphenyl)-N-(pyridin-3-ylmethyl)-2-[(pyridin-4-ylmethyl)amino]acetamide;
2-(2-hydroxyphenyl)-N-(2-pyridin-3-ylethyl)-2-[(2-pyridin-3-ylethyl)amino]acetamide;
2-(2-hydroxyphenyl)-N-(2-pyridin-4-ylethyl)-2-[(2-pyridin-3-ylethyl)amino]acetamide;
2-(2-hydroxyphenyl)-N-(2-pyridin-3-ylethyl)-2-[(2-pyridin-4-ylethyl)amino]acetamide;
2-(2,3-dihydro-1,4-benzodioxin-6-yl)-N-(2-pyridin-4-ylethyl)-2-[(2-pyridin-4-ylethyl)amino] acetamide;
2-(2,3-dihydro-1,4-benzodioxin-6-yl)-N-(pyridin-4-ylmethyl)-2-[(pyridin-4-ylmethyl)amino] acetamide;
2-(2,3-dihydro-1,4-benzodioxin-6-yl)-2-[(2-piperidin-4-ylethyl)amino]-N-(2-pyridin-4ylethyl) acetamide;
2-(2,3-dihydro-1,4-benzodioxin-6-yl)-2-[(2-piperidin-4-ylethyl)amino]-N-(pyridin-4-ylmethyl) acetamide;
2-(2,3-dihydro-1,4-benzodioxin-6-yl)-N-(pyridin-3-ylmethyl)-2-[(pyridin-3-ylmethyl)-amino] acetamide;
2-(2,3-dihydro-1,4-benzodioxin-6-yl)-N-(pyridin-4-ylmethyl)-2-[(pyridin-3-ylmethyl)amino] acetamide;
2-(2,3-dihydro-1,4-benzodioxin-6-yl)-N-(pyridin-3-ylmethyl)-2-[(pyridin-4-ylmethyl)amino] acetamide;
2-(2,3-dihydro-1,4-benzodioxin-6-yl)-N-(2-pyridin-3-ylethyl)-2-[(2-pyridin-3-ylethyl)amino] acetamide;
2-(2,3-dihydro-1,4-benzodioxin-6-yl)-N-(2-pyridin-4-ylethyl)-2-[(2-pyridin-3-ylethyl)amino] acetamide;
2-(2,3-dihydro-1,4-benzodioxin-6-yl)-N-(2-pyridin-3-ylethyl)-2-[(2-pyridin-4-ylethyl)amino] acetamide;
N-(2-pyridin-4-ylethyl)-2-[(2-pyridin-4-ylethyl)amino]-2-(3,4,5-trimethoxyphenyl)acetamide;
N-(pyridin-4-ylmethyl)-2-[(pyridin-4-ylmethyl) amino]-2-(3,4,5-trimethoxyphenyl)acetamide;
2-[(2-piperidin-4-ylethyl)amino]-N-(2-pyridin-4-ylethyl)-2-(3,4,5-trimethoxyphenyl)acetamide;
2-[(2-piperidin-4-ylethyl)amino]-N-(pyridin-4-ylmethyl)-2-(3,4,5-trimethoxyphenyl)acetamide;
N-(pyridin-3-ylmethyl)-2-[(pyridin-3-ylmethyl)amino]-2-(3,4,5-trimethoxyphenyl)acetamide;
N-(pyridin-4-ylmethyl)-2-[(pyridin-3-ylmethyl)amino]-2-(3,4,5-trimethoxyphenyl)acetamide;
N-(pyridin-3-ylmethyl)-2-[(pyridin-4-ylmethyl)amino]-2-(3,4,5-trimethoxyphenyl)acetamide;
N-(2-pyridin-3-ylethyl)-2-[(2-pyridin-3-ylethyl)amino]-2-(3,4,5-trimethoxyphenyl)acetamide;
N-(2-pyridin-4-ylmethyl)-2-[(2-pyridin-3-ylethyl) amino]-2-(3,4,5-trimethoxyphenyl)acetamide;
N-(2-pyridin-3-ylethyl)-2-[(2-pyridin-4-ylethyl)amino]-2-(3,4,5-trimethoxyphenyl)acetamide;
3-{2-oxo-1,2-bis[(2-pyridin-4-ylethyl)amino]ethyl}benzoic acid;
3-{2-oxo-1,2-bis[(pyridin-4-ylmethyl)amino]ethyl}benzoic acid;
3-{2-oxo-1,2-bis[(pyridin-4-ylmethyl)amino]ethyl}benzoic acid;
3-{2-oxo-1-[(2-piperidin-4-ylethyl)amino]-2-[(pyridin-4-ylmethyl)amino] ethyl}benzoic acid;
3-{2-oxo-1,2-bis[(pyridin-3-ylmethyl)amino]ethyl}benzoic acid;
3-{2-oxo-1-[(pyridin-3-ylmethyl)amino]-2-[(pyridin-4-ylmethyl)amino] ethyl}benzoic acid;
3-{2-oxo-2-[(pyridin-3-ylmethyl)amino]-1-[(pyridin-4-ylmethyl)amino]ethyl}benzoic acid;
3-{2-oxo-1,2-bis[(2-pyridin-3-ylethyl)amino]ethyl}benzoic acid;
3-{2-oxo-1-[(2-pyridin-3-ylethyl)amino]-2-[(2-pyridin-4-ylethyl)amino]ethyl}benzoic acid;
3-{2-oxo-2-[(2-pyridin-3-ylethyl)amino]-1-[(2-pyridin-4-ylethyl)amino]ethyl}benzoic acid;
4-{2-oxo-1,2-bis[(2-pyridin-4-ylethyl)amino]ethyl}benzoic acid;
4-{2-oxo-1,2-bis[(pyridin-4-ylmethyl)amino]ethyl}benzoic acid;
4-{2-oxo-1-[(2-piperidin-4-ylethyl)amino]-2-[(2-pyridin-4-ylethyl)-amino]ethyl}benzoic acid;
4-{2-oxo-1-[(2-piperidin-4-ylethyl)amino]-2-[(pyridin-4-ylmethyl)-amino]ethyl}benzoic acid;
4-{2-oxo-1,2-bis[(pyriclin-3-ylmethyl)amino]ethyl}benzoic acid;
4-{2-oxo-1-[(pyridin-3-ylmethyl)amino]-2-[(pyridin-4-ylmethyl)amino]ethyl}benzoic acid;
4-{2-oxo-2-[(pyridin-3-ylmethyl)amino]-1-[(pyridin-4-ylmethyl)amino]ethyl}benzoic acid;
4-{2-oxo-1,2-bis[(2-pyridin-3-ylethyl)amino]ethyl}benzoic acid;
4-{2-oxo-1-[(2-pyridin-3-ylethyl)amino]-2-[(2-pyridin-4-ylethyl)amino]ethyl}benzoic acid;
4-{2-oxo-2-[(2-pyridin-3-ylethyl)amino]-1-[(2-pyridin-4-ylethyl)amino]ethyl}benzoic acid;
methyl 3-{2-oxo-1,2-bis[(2-pyridin-4-ylethyl)amino]ethyl}benzoate;
methyl 3-{2-oxo-1,2-bis[(pyridin-4-ylmethyl)amino]ethyl}benzoate;
methyl 3-{2-oxo-1-[(2-piperidin-4-ylethyl)amino]-2-[(2-pyridin-4-ylethyl)amino]ethyl} benzoate;
methyl 3-{2-oxo-1-[(2-piperidin-4-ylethyl)amino]-2-[(pyridin-4-ylmethyl)amino]ethyl}benzoate;
methyl 3-{2-oxo-1,2-bis[(pyridin-3-ylmethyl)amino]ethyl}benzoate;
methyl 3-{2-oxo-1,2-bis[(pyridin-3-ylmethyl)amino]ethyl}benzoate;
methyl 3-{2-oxo-2-[(pyridin-3-ylmethyl)amino]-1-[(pyridin-4-ylmethyl)amino]ethyl}benzoate;
methyl 3-{2-oxo-1,2-bis[(2-pyridin-3-ylethyl)amino]ethyl}benzoate;
methyl 3-{2-oxo-1-[(2-pyridin-3-ylethyl)amino]-2-[(2-pyridin-4-ylethyl)-amino]ethyl}benzoate;
methyl 3-{2-oxo-2-[(2-pyridin-3-ylethyl)amino]-1-[(2-pyridin-4-ylethyl)amino]ethyl}benzoate;
methyl 4-{2-oxo-1,2-bis[(2-pyridin-4-ylethyl)amino]ethyl}benzoate;
methyl 4-{2-oxo-1,2-bis[(pyridin-4-ylmethyl)amino]ethyl}benzoate;
methyl 4-{2-oxo-1-[(2-piperidin-4-ylethyl)amino]-2-[(2-pyridin-4-ylethyl)amino]ethyl}benzoate;
methyl 4-{2-oxo-1-[(2-piperidin-4-ylethyl)amino]-2-[(pyridin-4-ylmethyl)amino]ethyl}benzoate;
methyl 4-{2-oxo-1,2-bis[(pyridin-3-ylmethyl)amino]ethyl}benzoate;
methyl 4-{2-oxo-1-[(pyridin-3-ylmethyl)amino]-2-[(pyridin-4-ylmethyl)amino]ethyl}benzoate;
methyl 4-{2-oxo-2-[(pyridin-3-ylmethyl)amino]-1-[(pyridin-4-ylmethyl)-amino]ethyl}benzoate;
methyl 4-{2-oxo-1,2-bis[(2-pyridin-3-ylethyl)amino]ethyl}benzoate;
methyl 4-{2-oxo-1-[(2-pyridin-3-ylethyl)amino]-2-[(2-pyridin-4-ylethyl)amino]ethyl}benzoate;
methyl 4-{2-oxo-2-[(2-pyridin-3-ylethyl)amino]-1-[(2-pyridin-4-ylethyl)amino]ethyl}benzoate;
ethyl 4-{2-oxo-1,2-bis[(2-pyridin-4-ylethyl)amino]ethylbenzoate;
ethyl 4-{2-oxo-1,2-bis[(pyridin-4-ylmethyl)amino]ethyl}benzoate;
ethyl [4-{2-oxo-1-[(2-piperidin-4-ylethyl)amino]-2-[(2-pyridin-4-ylethyl)amino]ethyl}benzoate;
ethyl [4-{2-oxo-1-[(2-piperidin-4-ylethyl)amino]-2-[(pyridin-4-ylmethyl)amino]ethyl}-benzoate;
ethyl 4-{2-oxo-1,2-bis[(pyridin-3-ylmethyl)amino]ethyl}benzoate;
ethyl 4-{2-oxo-1-[(pyridin-3-ylmethyl)amino]-2-[(pyridin-4-ylmethyl)amino]ethyl}benzoate;
ethyl 4-{2-oxo-2-[(pyridin-3-ylmethyl)amino]-1-[(pyridin-4-ylmethyl)amino]ethyl}benzoate;
ethyl 4-{2-oxo-1,2-bis[(2-pyridin-3-ylethyl)amino]ethyl}benzoate;
ethyl 4-{2-oxo-1-[(2-pyridin-3-ylethyl)amino]-2-[(2-pyridin-4-ylethyl)amino]ethyl}benzoate;
ethyl 4-{2-oxo-2-[(2-pyridin-3-ylethyl)amino]-1-[(2-pyridin-4-ylethyl)amino]ethyl}benzoate;
ethyl 3-{2-oxo-1,2-bis[(2-pyridin-4-ylethyl)amino]ethyl}benzoate;
ethyl 3-{2-oxo-1,2-bis[(pyridin-4-ylmethyl)amino]ethyl}benzoate;
ethyl 3-{2-oxo-1-[(2-piperidin-4-ylethyl)amino]-2-[(2-pyridin-4-ylethyl)amino]ethyl}benzoate;
ethyl 3-{2-oxo-1-[(2-piperidin-4-ylethyl)amino]-2-[(pyridin-4-ylmethyl)amino]ethyl}benzoate;
ethyl 3-{2-oxo-1,2-bis[(pyridin-3-ylmethyl)amino]ethyl}benzoate;
ethyl 3-{2-oxo-1-[(pyridin-3-ylmethyl)amino]-2-[(pyridin-4-ylmethyl)amino]ethyl}benzoate;
ethyl 3-{2-oxo-2-[(pyridin-3-ylmethyl)amino]-1-[(pyridin-4-ylmethyl)amino]ethyl}benzoate;
ethyl 3-{2-oxo-1,2-bis[(2-pyridin-3-ylethyl)amino]ethylbenzoate;
ethyl 3-{2-oxo-1-[(2-pyridin-3-ylethyl)amino]-2-[(2-pyridin-4-ylethyl)amino]ethyl}benzoate;
ethyl 3-{2-oxo-2-[(2-pyridin-3-ylethyl)amino]-1-[(2-pyridin-4-ylethyl)amino]ethyl}benzoate;
R-(-)-2-(4-methoxyphenyl)-2-[(2-piperidin-4-ylethyl)amino]-N-(2-pyridin-4-ylethyl)acetamide;
S-(+)-2-(4-methoxyphenyl)-2-[(2-piperidin-4-ylethyl)amino]-N-(2-pyridin-4-ylethyl)acetamide;
R-(-)-2-(3-chlorophenyl)-2-[(2-piperidin-4-ylethyl)amino]-N-(2-pyridin-4-ylethyl)acetamide;
S-(+)-2-(3-chlorophenyl)-2-[(2-piperidin-4-ylethyl)amino]-N-(2-pyridin-4-ylethyl)acetamide;
R-(-)-2-(2-fluorophenyl)-2-[(2-piperidin-4-ylethyl)amino]-N-(2-pyridin-4-ylethyl)acetamide;
S-(+)-2-(2-fluorophenyl)-2-[(2-piperidin-4-ylethyl)amino]-N-(2-pyridin-4-ylethyl)acetamide;
R-(-)-2-(1,3-benzodioxole-5-yl)-N-(pyridin-4-ylmethyl)-2-[(pyridin-4-ylmethyl)amino]acetamide;
S-(+)-2-(1,3-benzodioxole-5-yl)-N-(pyridin-4-ylmethyl)-2-[(pyridin-4-ylmethyl)amino]acetamide;
R-(-)-methyl 4-{2-oxo-1,2-bis[(2-pyridin-4-ylethyl)amino]ethyl}benzoate;
S-(+)-methyl 4-{2-oxo-1,2-bis[(2-pyridin-4-ylethyl)amino]ethyl}benzoate;
R-(-)-methyl 3-{2-oxo-1,2-bis [(2-pyridin-4-ylethyl)amino]ethyl}benzoate;
S-(+)- methyl 3-{2-oxo-1,2-bis[(2-pyridin-4-ylethyl)amino]ethyl}benzoate;
R-(-)-2-(3-fluorophenyl)-N-(2-pyridin-4-ylethyl)-2-[(2-pyridin-3-ylethyl)amino]acetamide;
S-(+)-2-(3-fluorophenyl)-N-(2-pyridin-4-ylethyl)-2-[(2-pyridin-3-ylethyl)amino]acetamide;
R-(-)-2-(2-ethoxyphenyl)-N-(pyridin-4-ylmethyl)-2-[(pyridin-4-ylmethyl)amino]acetamide;
S-(+)-2-(2-ethoxyphenyl)-N-(pyridin-4-ylmethyl)-2-[(pyridin-4-ylmethyl)amino]acetamide;
R-(-)-2-(2-bromophenyl)-N-(2-pyridin-3-ylethyl)-2-[(2-pyridin-4-ylethyl)amino]acetamide;
S-(+)-2-(2- bromophenyl)-N-(2-pyridin-3-ylethyl)-2-[(2-pyridin-4-ylethyl)amino]acetamide;
R-(-)-2-(2,4-dichlorophenyl)-N-(2-pyridin-4-ylethyl)-2-[(2-pyridin-4-ylethyl)amino]acetamide;
S-(+)-2-(2,4-dichlorophenyl)-N-(2-pyridin-4-ylethyl)-2-[(2-pyridin-4-ylethyl)amino]acetamide;
R-(-)-2-(2,4-dimethylphenyl)-N-(2-pyridin-4-ylethyl)-2-[(2-pyridin-4-ylethyl)amino]acetamide;
S-(+)-2-(2,4-dimethylphenyl)-N-(2-pyridin-4-ylethyl)-2-[(2-pyridin-4-ylethyl)amino]acetamide;
R-(-)-2-[(2-piperidin-4-ylethyl)amino]-N-(2-pyridin-4-ylethyl)-2-[3-(trifluoromethyl) phenyl] acetamide;
S-(+)-2-[(2-piperidin-4-ylethyl)amino]-N-(2-pyridin-4-ylethyl)-2-[3-(trifluoromethyl) phenyl] acetamide;
R-(-)-N-(2-pyridin-4-ylethyl)-2-[(2-pyridin-4-ylethyl)amino]-2-(2-thienyl)acetamide;
S-(+)-N-(2-pyridin-4-ylethyl)-2-[(2-pyridin-4-ylethyl)amino]-2-(2- thienyl)acetamide;
R-(-)-2-phenyl-N-(pyridin-4-ylmethyl)-2-[(pyridin-4-ylmethyl)amino]acetamide;
S-(+)-2-phenyl-N-(pyridin-4-ylmethyl)-2-[(pyridin-4-ylmethyl)amino]acetamide;
R-(-)-N-(pyridin-4-ylmethyl)-2-[(pyridin-3-ylmethyl)amino]-2-(2,4,5-trimethylphenyl)acetamide;
S-(+)-N-(pyridin-4-ylmethyl)-2-[(pyridin-3-ylmethyl)amino]-2-(2,4,5-trimethylphenyl)acetamide;
or pharmaceutically acceptable salts thereof, unless it is specifically indicated.

5. Compounds according to claim 4 selected from methyl 4-{2-oxo-1,2-bis[(2-pyridin-4-ylethyl) amino] ethyl} benzoate and 2-(1,3-benzodioxol-5-yl)-N-(2-pyridin-4-ylethyl)-2-[(2-pyridin-4-ylethyl)- amino]acetamide.

6. Compounds according to any one of claims 1-4 of the general formulae **I, I.1, I.1.1** or **I.1.2** or pharmaceutically acceptable salts thereof or alkyl esters thereof in the form of individual optical isomers or its mixtures, of any one of the claims 1-5 having sAPP-mimetics properties as an active component for producing a medecine or a pharmaceutical composition.

7. Physiologically active agent comprising a compound of the general formulae **I, I.1, I.1.1** or **I.1.2** or pharmaceutically acceptable salts thereof or alkyl esters in the form of individual optical isomers or its mixtures, of any one of the claims 1-5 that are able to effectively influence the processes for memory formation, storage and recall.

8. Physiologically active agent according to the claim 7 for potentiation of early stages of memory formation to form more stable long-term memory.

9. Physiologically active agent according to the claim 7 for potentiation of late stages of memory formation to enhance memory relating to previously gained experience.

10. Compound of the general formulae **I, I.1, I.1.1** and **I.1.2** or pharmaceutically acceptable salts thereof or alkyl esters thereof in the form of individual optical isomers or its mixtures, of any one of the claims 1-6 having the ability for effective influence the processes for memory formation, storage and recall as an active component for producing a medecine or a pharmaceutical composition.

11. Pharmaceutical composition having effective influence the memory formation, storage and recall comprising an active component according to claim 10.

12. Medecine in the form of tablets, capsules or injections placed in packing suitable for pharmaceuticals for treatment and prophylaxis of a disorder associated with impairment of memory formation, storage and recall comprising an active component according to claim 10 or pharmaceutical composition according to claim 11.

13. Compounds of the general formulae **I, I.1, I.1.1** and **I.1.2** according to any one of claims 1-6 intending for studing (*in vitro* and in *v*ivo) the biochemical signaling, neurotrophic and neuroprotective processes associated with secretory protein sAPP and for studying the mechanisms of the memory formation, storage and regeneration.

14. Method of producing the compounds of the formula **I** by multi-component reaction of aldehyde, amine, izonitriles and carboxylic acid followed by removal of N-acyl group according with the scheme 1: 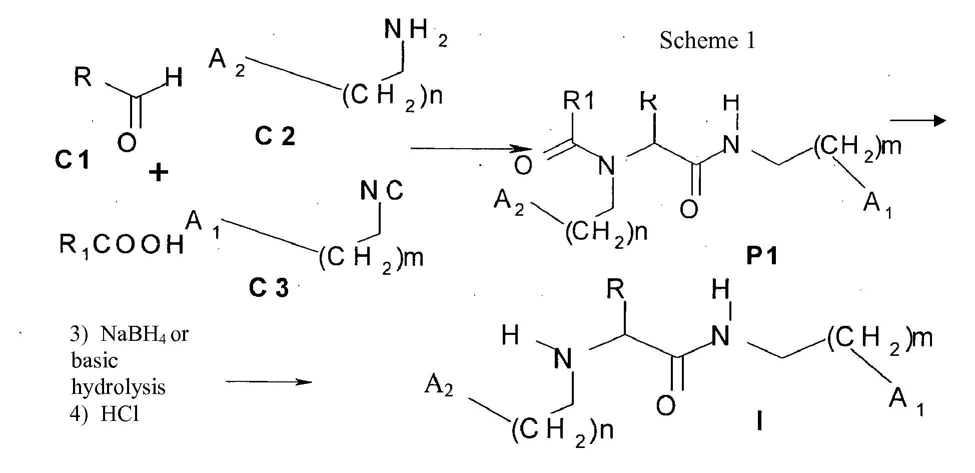 where R, A₁ and A₂ are as defined in claim 1.
